(19) Europäisches Patentamt
European Patent Office
Office européen des brevets

(11) EP 4 567 028 A1

(12) **EUROPEAN PATENT APPLICATION**

(43) Date of publication:
**11.06.2025 Bulletin 2025/24**

(21) Application number: **25157062.8**

(22) Date of filing: **28.04.2022**

(51) International Patent Classification (IPC):
***C07D 237/34*** *(2006.01)* ***C07D 403/04*** *(2006.01)*
***C07D 403/12*** *(2006.01)* ***C07D 403/14*** *(2006.01)*
***C07D 471/04*** *(2006.01)* ***C07D 487/04*** *(2006.01)*
***A61P 25/28*** *(2006.01)* ***A61P 29/00*** *(2006.01)*
***A61P 35/00*** *(2006.01)* ***A61P 37/00*** *(2006.01)*
***A61K 31/502*** *(2006.01)*

(52) Cooperative Patent Classification (CPC):
**C07D 471/04; A61P 25/28; A61P 29/00; A61P 35/00; A61P 37/00; C07D 237/34; C07D 403/04; C07D 403/12; C07D 403/14; C07D 487/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.04.2021 EP 21171231**

(62) Document number(s) of the earlier application(s) in accordance with Art. 76 EPC:
**22727001.4 / 4 330 235**

(71) Applicant: **JANSSEN Pharmaceutica NV**
**2340 Beerse (BE)**

(72) Inventors:
- **OEHLRICH, Daniel**
  **2340 Beerse (BE)**
- **VAN OPDENBOSCH, Nina**
  **2340 Beerse (BE)**
- **LAMKANFI, Mohamed**
  **2340 Beerse (BE)**
- **CAÑELLAS ROMAN, Santiago**
  **28042 Madrid (ES)**
- **VAN GOOL, Michiel, Luc, Maria**
  **28042 Madrid (ES)**
- **DIÉGUEZ VÁZQUEZ, Alejandro**
  **28042 Madrid (ES)**

(74) Representative: **Carpmaels & Ransford LLP**
**One Southampton Row**
**London WC1B 5HA (GB)**

Remarks:
•This application was filed on 11-02-2025 as a divisional application to the application mentioned under INID code 62.
•Claims filed after the date of filing of the application / after the date of receipt of the divisional application (Rule 68(4) EPC).

(54) **PHTHALAZINONE DERIVATIVES AS NLRP3 INFLAMMASOME INHIBITORS**

(57) Provided are novel compounds for use as inhibitors of NLRP3 inflammasome production, wherein such compounds are as defined by compounds of formula (I)

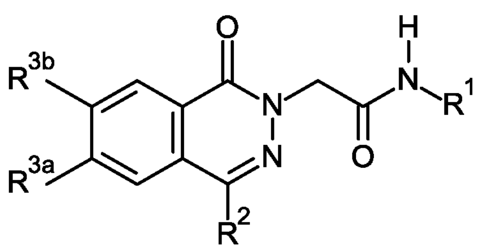

(I)

wherein $R^1$, $R^2$ and $R^{3a}$ and $R^{3b}$ are as defined in the description, and where the compounds may be useful as medicaments, for instance for use in the treatment of a disease or disorder that is associated with NLRP3 inflammasome activity.

EP 4 567 028 A1

**Description**

## FIELD OF THE INVENTION

The present invention relates to novel phthalazinone derivatives that are useful as inhibitors of the NOD-like receptor protein 3 (NLRP3) inflammasome pathway. The invention also relates to processes for the preparation of said compounds, pharmaceutical compositions comprising said compounds, the use of said compounds in the treatment of various diseases and disorders mediated by NLRP3.

## BACKGROUND OF THE INVENTION

Inflammasomes, considered as central signalling hubs of the innate immune system, are multi-protein complexes that are assembled upon activation of a specific set of intracellular pattern recognition receptors (PRRs) by a wide variety of pathogen- or danger- associated molecular patterns (PAMPs or DAMPs). To date, it was shown that inflammasomes can be formed by nucleotide-binding oligomerization domain (NOD)-like receptors (NLRs) and Pyrin- and HIN200-domain-containing proteins (Van Opdenbosch N and Lamkanfi M. Immunity, 2019 Jun 18;50(6):1352-1364). The NLRP3 inflammasome is assembled upon detection of environmental crystals, pollutants, host-derived DAMPs and protein aggregates (Tartey S and Kanneganti TD. Immunology, 2019 Apr;156(4):329-338). Clinically relevant DAMPs that engage NLRP3 include uric acid and cholesterol crystals that cause gout and atherosclerosis, amyloid-β fibrils that are neurotoxic in Alzheimer's disease and asbestos particles that cause mesothelioma (Kelley et al., Int J Mol Sci, 2019 Jul 6;20(13)). Additionally, NLRP3 is activated by infectious agents such as *Vibrio cholerae*; fungal pathogens such as *Aspergillus fumigatus* and *Candida albicans*; adenoviruses, influenza A virus and SARS-CoV-2 (Tartey and Kanneganti, 2019 (see above); Fung et al. Emerg Microbes Infect, 2020 Mar 14;9(1):558-570).

Although the precise NLRP3 activation mechanism remains unclear, for human monocytes, it has been suggested that a one-step activation is sufficient while in mice a two-step mechanism is in place. Given the multitude in triggers, the NLRP3 inflammasome requires add-on regulation at both transcriptional and post-transcriptional level (Yang Y et al., Cell Death Dis, 2019 Feb 12;10(2):128).

The NLRP3 protein consists of an N-terminal pyrin domain, followed by a nucleotide-binding site domain (NBD) and a leucine-rich repeat (LRR) motif on C-terminal end (Sharif et al., Nature, 2019 Jun; 570(7761):338-343). Upon recognition of PAMP or DAMP, NLRP3 aggregates with the adaptor protein, apoptosis-associated speck-like protein (ASC), and with the protease caspase-1 to form a functional inflammasome. Upon activation, procaspase-1 undergoes autoproteolysis and consequently cleaves gasdermin D (Gsdmd) to produce the N-terminal Gsdmd molecule that will ultimately lead to pore-formation in the plasma membrane and a lytic form of cell death called pyroptosis. Alternatively, caspase-1 cleaves the pro-inflammatory cytokines pro-IL-1β and pro-IL-18 to allow release of its biological active form by pyroptosis (Kelley et al., 2019).

Dysregulation of the NLRP3 inflammasome or its downstream mediators are associated with numerous pathologies ranging from immune/inflammatory diseases, auto-immune/auto-inflammatory diseases (Cryopyrin-associated Periodic Syndrome (Miyamae T. Paediatr Drugs, 2012 Apr 1;14(2):109-17); sickle cell disease; systemic lupus erythematosus (SLE)) to hepatic disorders (e.g. non-alcoholic steatohepatitis (NASH), chronic liver disease, viral hepatitis, alcoholic steatohepatitis, and alcoholic liver disease) (Szabo G and Petrasek J. Nat Rev Gastroenterol Hepatol, 2015 Jul;12(7):387-400) and inflammatory bowel diseases (e.g. Crohn's disease, ulcerative colitis) (Zhen Y and Zhang H. Front Immunol, 2019 Feb 28;10:276). Also, inflammatory joint disorders (e.g. gout, pseudogout (chondrocalcinosis), arthropathy, osteoarthritis, and rheumatoid arthritis (Vande Walle L et al., Nature, 2014 Aug 7;512(7512):69-73) were linked to NLRP3. Additionally, kidney related diseases (hyperoxaluria (Knauf et al., Kidney Int, 2013 Nov;84(5):895-901), lupus nephritis, hypertensive nephropathy (Krishnan et al., Br J Pharmacol, 2016 Feb;173(4):752-65), hemodialysis related inflammation and diabetic nephropathy which is a kidney-related complication of diabetes (Type 1, Type 2 and mellitus diabetes), also called diabetic kidney disease (Shahzad et al., Kidney Int, 2015 Jan;87(1):74-84) are associated to NLRP3 inflammasome activation. Reports link onset and progression of neuroinflammation-related disorders (e.g. brain infection, acute injury, multiple sclerosis, Alzheimer's disease) and neurodegenerative diseases (Parkinson's disease) to NLRP3 inflammasome activation (Sarkar et al., NPJ Parkinson's Dis, 2017 Oct 17;3:30). In addition, cardiovascular or metabolic disorders (e.g. cardiovascular risk reduction (CvRR), atherosclerosis, type I and type II diabetes and related complications (e.g. nephropathy, retinopathy), peripheral artery disease (PAD), acute heart failure and hypertension (Ridker et al., CANTOS Trial Group. N Engl J Med, 2017 Sep 21;377(12):1119-1131; and Toldo S and Abbate A. Nat Rev Cardiol, 2018 Apr;15(4):203-214) have recently been associated to NLRP3. Also, skin associated diseases were described (e.g. wound healing and scar formation; inflammatory skin diseases, e.g. acne, hidradenitis suppurativa (Kelly et al., Br J Dermatol, 2015 Dec;173(6)). In addition, respiratory conditions have been associated with NLRP3 inflammasome activity (e.g. asthma, sarcoidosis, *Severe Acute Respiratory Syndrome* (SARS) (Nieto-Torres et al., Virology, 2015 Nov;485:330-9)) but also age-related macular degeneration (Doyle et al., Nat Med, 2012 May;18(5):791-8). Several

cancer related diseases/disorders were described linked to NLRP3 (e.g. myeloproliferative neoplasms, leukemias, myelodysplastic syndromes (MOS), myelofibrosis, lung cancer, colon cancer (Ridker et al., Lancet, 2017 Oct 21; 390(10105):1833-1842; Derangere et al., Cell Death Differ. 2014 Dec;21(12):1914-24; Basiorka et al., Lancet Haematol, 2018 Sep;5(9): e393-e402, Zhang et al., Hum Immunol, 2018 Jan;79(1):57-62).

Several patent applications describe NLRP3 inhibitors, with recent ones including for instance international patent application WO 2020/018975, WO 2020/037116, WO 2020/021447, WO 2020/010143, WO 2019/079119, WO 2019/0166621 and WO 2019/121691, which disclose a range of specific compounds.

There is a need for new inhibitors of the NLRP3 inflammasome pathway to provide alternative treatments for the diseases/disorders mentioned herein.

## SUMMARY OF THE INVENTION

The invention provides compounds which inhibit the NLRP3 inflammasome pathway. Thus, in an aspect of the invention, there is now provided a compound of formula (I),

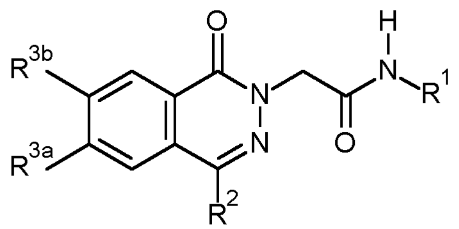

(I)

or a pharmaceutically acceptable salt thereof, wherein:

$R^1$ represents:

(i) $C_{3-6}$ cycloalkyl optionally substituted with one or more substituents independently selected from -OH and $-C_{1-3}$ alkyl;
(ii) aryl or heteroaryl, each of which is optionally substituted with 1 to 3 substituents independently selected from halo, -OH, $-O-C_{1-3}$ alkyl, $-C_{1-3}$ alkyl, halo$C_{1-3}$alkyl, hydroxy$C_{1-3}$ alkyl, $C_{1-3}$ alkoxy, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents $-N(R^{2a})R^{2b}$;

$R^{2a}$ and $R^{2b}$ each represent hydrogen or $C_{1-4}$ alkyl optionally substituted with one or more substituents selected from halo, $-OC_{1-3}$ alkyl and $-N(C_{1-3}\ alkyl)_2$, or $R^{2a}$ and $R^{2b}$ may be linked together to form a 3- to 4-membered ring optionally substituted by one or more fluoro atoms;

one of $R^{3a}$ and $R^{3b}$ represents hydrogen, and the other represents $R^3$;

$R^3$ represents:

(i) hydrogen;
(ii) halo;
(iii) $C_{1-4}$ alkyl optionally substituted with one or more substituents independently selected from halo, -OH and $-OC_{1-3}$ alkyl;
(iv) $C_{2-4}$ alkenyl optionally substituted with $-OC_{1-3}$ alkyl;
(v) $C_{3-6}$ cycloalkyl; or
(vi) $-OC_{1-3}$ alkyl.

In another aspect, there are provided compounds of formula (I) for use as a medicament. In another aspect, there is provided a pharmaceutical composition comprising a therapeutically effective amount of a compound of formula (I).

In a further aspect, there is provided compounds of formula (I) and/or pharmaceutical compositions comprising such compounds for use: in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject

in need thereof); and/or as an NLRP3 inhibitor. Specific diseases or disorders may be mentioned herein, and may for instance be selected from inflammasome-related diseases or disorders, immune diseases, inflammatory diseases, auto-immune diseases, or auto-inflammatory diseases.

In another aspect, there is provided a use of compounds of formula (I) (and/or pharmaceutical compositions comprising such compounds): in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor.

In another aspect, there is provided use of compounds of formula (I) (and/or pharmaceutical compositions comprising such compounds) in the manufacture of a medicament for: the treatment of a disease or disorder associated with NLRP3 activity (including inflammasome activity); the treatment of a disease or disorder in which the NLRP3 signalling contributes to the pathology, and/or symptoms, and/or progression, of said disease/disorder; and/or inhibiting NLRP3 inflammasome activity (including in a subject in need thereof).

In another aspect, there is provided a method of treating a disease or disorder in which the NLRP3 signalling contributes to the pathology, and/or symptoms, and/or progression, of said disease/disorder, comprising administering a therapeutically effective amount of a compound of formula (I), for instance to a subject (in need thereof). In a further aspect there is provided a method of inhibiting the NLRP3 inflammasome activity in a subject (in need thereof), the method comprising administering to the subject in need thereof a therapeutically effective amount of a compound of formula (I).

## DETAILED DESCRIPTION OF THE INVENTION

The invention provides a compound of formula (I),

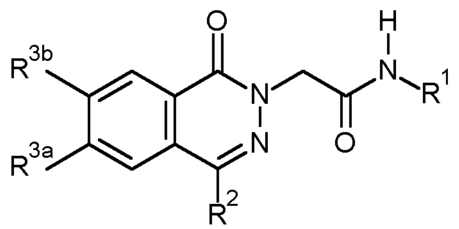

(I)

or a pharmaceutically acceptable salt thereof, wherein:

$R^1$ represents:

(i) $C_{3-6}$ cycloalkyl optionally substituted with one or more substituents independently selected from -OH and -$C_{1-3}$ alkyl;
(ii) aryl or heteroaryl, each of which is optionally substituted with 1 to 3 substituents independently selected from halo, -OH, -O-$C_{1-3}$ alkyl, -$C_{1-3}$ alkyl, halo$C_{1-3}$alkyl, hydroxy$C_{1-3}$ alkyl, $C_{1-3}$ alkoxy, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents -N($R^{2a}$)$R^{2b}$;

$R^{2a}$ and $R^{2b}$ each represent hydrogen or $C_{1-4}$ alkyl optionally substituted with one or more substituents selected from halo, -O$C_{1-3}$ alkyl and -N($C_{1-3}$ alkyl)$_2$, or $R^{2a}$ and $R^{2b}$ may be linked together to form a 3- to 4-membered ring optionally substituted by one or more fluoro atoms;

one of $R^{3a}$ and $R^{3b}$ represents hydrogen, and the other represents $R^3$;

$R^3$ represents:

(i) hydrogen;
(ii) halo;
(iii) $C_{1-4}$ alkyl optionally substituted with one or more substituents independently selected from halo, -OH and -O$C_{1-3}$ alkyl;
(iv) $C_{2-4}$ alkenyl optionally substituted with -O$C_{1-3}$ alkyl;
(v) $C_{3-6}$ cycloalkyl; or

(vi) $-OC_{1-3}$ alkyl.

Pharmaceutically-acceptable salts include acid addition salts and base addition salts. Such salts may be formed by conventional means, for example by reaction of a free acid or a free base form of a compound of formula (I) with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of formula (I) in the form of a salt with another counter-ion, for example using a suitable ion exchange resin.

Pharmaceutically acceptable acid addition salts can be formed with inorganic acids and organic acids.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine, and tromethamine

Compounds of formula (I) may contain double bonds and may thus exist as *E* (e*ntgegen*) and *Z* (*zusammen*) geometric isomers about each individual double bond.

Compounds of formula (I) may also exhibit tautomerism. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerisations. Valence tautomers include interconversions by reorganisation of some of the bonding electrons.

Compounds of formula (I) may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC techniques. Alternatively the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person.

In the structures shown herein, where the stereochemistry of any particular chiral atom is not specified, then all stereoisomers are contemplated and included as the compounds of formula (I). Where stereochemistry is specified by a solid wedge or dashed line representing a particular configuration, then that stereoisomer is so specified and defined.

When an absolute configuration is specified, it is according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer.

The compounds of formula (I) may exist in unsolvated as well as solvated forms with pharmaceutically acceptable solvents such as water, ethanol, and the like.

The compounds of formula (I) may be isotopically labelled. Such compounds are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of formula (I). Exemplary isotopes that can be incorporated into compounds of formula (I) include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$, $^{13}N$, $^{15}O$, $^{17}O$, $^{18}O$, $^{32}P$, $^{33}P$, $^{35}S$, $^{18}F$, $^{36}Cl$, $^{123}I$, and $^{125}I$. Certain isotopically-labelled compounds of formula (I) (e.g., those labelled with $^{3}H$ and $^{14}C$) are

useful in compound distribution assays. Tritiated ($^3H$) and carbon-l4 ($^{14}C$) isotopes are useful for their ease of preparation and detectability. Further, substitution with heavier isotopes such as deuterium (i.e., $^2H$ may afford certain therapeutic advantages resulting from greater metabolic stability (e.g., increased *in vivo* half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies to examine substrate receptor occupancy. Isotopically labelled compounds of formula (I) can generally be prepared by following procedures analogous to those disclosed in the description/Examples hereinbelow, by substituting an isotopically labelled reagent for a non-isotopically labelled reagent.

Unless otherwise specified, $C_{1-q}$ alkyl groups (where q is the upper limit of the range) defined herein may be straight-chain or, when there is a sufficient number (i.e. a minimum of two or three, as appropriate) of carbon atoms, be branched-chain. Such a group is attached to the rest of the molecule by a single bond.

$C_{2-q}$ alkenyl when used herein (again where q is the upper limit of the range) refers to an alkyl group that contains unsaturation, i.e. at least one double bond.

$C_{3-q}$ cycloalkyl (where q is the upper limit of the range) refers to an alkyl group that is cyclic, for instance cycloalkyl groups may be monocyclic or, if there are sufficient atoms, bicyclic. In an embodiment, such cycloalkyl groups are monocyclic.

The term "halo", when used herein, preferably includes fluoro, chloro, bromo and iodo.

$C_{1-q}$ alkoxy groups (where q is the upper limit of the range) refers to the radical of formula $-OR^a$, where $R^a$ is a $C_{1-q}$ alkyl group as defined herein.

Halo$C_{1-q}$ alkyl (where q is the upper limit of the range) groups refer to $C_{1-q}$ alkyl groups, as defined herein, where such group is substituted by one or more halo. Hydroxy$C_{1-q}$ alkyl (where q is the upper limit of the range) refers to $C_{1-q}$ alkyl groups, as defined herein, where such group is substituted by one or more (e.g. one) hydroxy (-OH) groups (or one or more, e.g. one, of the hydrogen atoms is replaced with -OH). Similarly, halo$C_{1-q}$ alkoxy and hydroxy$C_{1-q}$ alkoxy represent corresponding $-OC_{1-q}$ alkyl groups that are substituted by one or more halo, or, substituted by one or more (e.g. one) hydroxy, respectively.

Heterocyclyl groups that may be mentioned include non-aromatic monocyclic and bicyclic heterocyclyl groups in which at least one (e.g. one to four) of the atoms in the ring system is other than carbon (i.e. a heteroatom), and in which the total number of atoms in the ring system is between 3 and 20 (e.g. between three and ten, e.g. between 3 and 8). Such heterocyclyl groups may also be bridged. Such heterocyclyl groups are saturated. $C_{2-q}$ heterocyclyl groups that may be mentioned include 7-azabicyclo[2.2.1]heptanyl, 6-azabicyclo[3.1.1]heptanyl, 6-azabicyclo[3.2.1]-octanyl, 8-azabicyclo-[3.2.1]octanyl, aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, 7-oxabicyclo[2.2.1]heptanyl, 6-oxabicyclo-[3.2.1]octanyl, oxetanyl, oxiranyl, piperazinyl, piperidinyl, non-aromatic pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl (such as 1,2,3,4-tetrahydropyridyl and 1,2,3,6-tetrahydropyridyl), thietanyl, thiiranyl, thiolanyl, thiomorpholinyl, trithianyl (including 1,3,5-trithianyl), tropanyl and the like. Substituents on heterocyclyl groups may, where appropriate, be located on any atom in the ring system including a heteroatom. The point of attachment of heterocyclyl groups may be *via* any atom in the ring system including (where appropriate) a heteroatom (such as a nitrogen atom), or an atom on any fused carbocyclic ring that may be present as part of the ring system. Heterocyclyl groups may also be in the *N*- or *S*- oxidised form. In an embodiment, heterocyclyl groups mentioned herein are monocyclic.

Aryl groups that may be mentioned include $C_{6-20}$, such as $C_{6-12}$ (e.g. $C_{6-10}$) aryl groups. Such groups may be monocyclic, bicyclic or tricyclic and have between 6 and 12 (e.g. 6 and 10) ring carbon atoms, in which at least one ring is aromatic. $C_{6-10}$ aryl groups include phenyl, naphthyl and the like, such as 1,2,3,4-tetrahydronaphthyl. The point of attachment of aryl groups may be *via* any atom of the ring system. For example, when the aryl group is polycyclic the point of attachment may be *via* atom including an atom of a non-aromatic ring. However, when aryl groups are polycyclic (e.g. bicyclic or tricyclic), they are preferably linked to the rest of the molecule *via* an aromatic ring. When aryl groups are polycyclic, in an embodiment, each ring is aromatic. In an embodiment, aryl groups mentioned herein are monocyclic or bicyclic. In a further embodiment, aryl groups mentioned herein are monocyclic.

"Heteroaryl" when used herein refers to an aromatic group containing one or more heteroatom(s) (e.g. one to four heteroatoms) preferably selected from N, O and S. Heteroaryl groups include those which have between 5 and 20 members (e.g. between 5 and 10) and may be monocyclic, bicyclic or tricyclic, provided that at least one of the rings is aromatic (so forming, for example, a mono-, bi-, or tricyclic heteroaromatic group). When the heteroaryl group is polycyclic the point of attachment may be *via* any atom including an atom of a non-aromatic ring. However, when heteroaryl groups are polycyclic (e.g. bicyclic or tricyclic), they are preferably linked to the rest of the molecule *via* an aromatic ring. In an embodiment, when heteroaryl groups are polycyclic, then each ring is aromatic. Heteroaryl groups that may be mentioned include 3,4-dihydro-1*H*-isoquinolinyl, 1,3-dihydroisoindolyl, 1,3-dihydroisoindolyl (e.g. 3,4-dihydro-1*H*-isoquinolin-2-yl, 1,3-dihydroisoindol-2-yl, 1,3-dihydroisoindol-2-yl; i.e. heteroaryl groups that are linked *via* a non-aromatic ring), or, preferably, acridinyl, benzimidazolyl, benzodioxanyl, benzodioxepinyl, benzodioxolyl (including 1,3-benzodioxolyl),

benzofuranyl, benzofurazanyl, benzothiadiazolyl (including 2,1,3-benzothiadiazolyl), benzothiazolyl, benzoxadiazolyl (including 2,1,3-benzoxadiazolyl), benzoxazinyl (including 3,4-dihydro-2*H*-1,4-benzoxazinyl), benzoxazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-*a*]pyridyl, indazolyl, indolinyl, indolyl, isobenzofuranyl, isochromanyl, isoindolinyl, isoindolyl, isoquinolinyl, isothiaziolyl, isothiochromanyl, isoxazolyl, naphthyridinyl (including 1,6-naphthyridinyl or, preferably, 1,5-naphthyridinyl and 1,8-naphthyridinyl), oxadiazolyl (including 1,2,3-oxadiazolyl, 1,2,4-oxadiazolyl and 1,3,4-oxadiazolyl), oxazolyl, phenazinyl, phenothiazinyl, phthalazinyl, pteridinyl, purinyl, pyranyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3,4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1,2,4-thiadiazolyl and 1,3,4-thiadiazolyl), thiazolyl, thiochromanyl, thiophenetyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be *via* any atom in the ring system including (where appropriate) a heteroatom (such as a nitrogen atom), or an atom on any fused carbocyclic ring that may be present as part of the ring system. Heteroaryl groups may also be in the *N*- or *S*- oxidised form. When heteroaryl groups are polycyclic in which there is a non-aromatic ring present, then that non-aromatic ring may be substituted by one or more =O group. In an embodiment, heteroaryl groups mentioned herein may be monocyclic or bicyclic. In a further embodiment, heteroaryl groups mentioned herein are monocyclic.

Heteroatoms that may be mentioned include phosphorus, silicon, boron and, preferably, oxygen, nitrogen and sulfur.

For the avoidance of doubt, where it is stated herein that a group may be substituted by one or more substituents (e.g. selected from $C_{1-6}$ alkyl), then those substituents (e.g. alkyl groups) are independent of one another. That is, such groups may be substituted with the same substituent (e.g. same alkyl substituent) or different (e.g. alkyl) substituents.

All individual features (e.g. preferred features) mentioned herein may be taken in isolation or in combination with any other feature (including preferred feature) mentioned herein (hence, preferred features may be taken in conjunction with other preferred features, or independently of them).

Various embodiments of the invention will now be described, including embodiments of the compounds of formula (I)

In an embodiment, compounds of formula (I) include those in which $R^1$ represents: (i) $C_{3-6}$ cycloalkyl; (ii) aryl or heteroaryl; or (iii) or heterocyclyl, all of which are optionally substituted as herein defined. In a particular embodiment, $R^1$ represents: (i) $C_{3-6}$ cycloalkyl; or (ii) aryl or heteroaryl, all of which are optionally substituted as herein defined.

In an embodiment when $R^1$ represents optionally substituted $C_{3-6}$ cycloalkyl, then it represents $C_{3-6}$ cycloalkyl (or, in an embodiment, $C_{3-4}$ cycloalkyl) optionally substituted by one or two substituents selected from $C_{1-3}$ alkyl (e.g. methyl) and -OH. In a further embodiment, $R^1$ represents cyclopropyl (e.g. unsubstituted) or cyclobutyl.

In a further embodiment, $R^1$ represents cyclohexyl. In yet a further embodiment, $R^1$ represents unsubstituted cyclopropyl or cyclobutyl substituted by -OH and methyl (e.g. at the same carbon atom). In yet a further embodiment, $R^1$ represents cyclohexyl, for instance substituted by -OH (e.g. by one -OH group). In an embodiment therefore, $R^1$ represents:

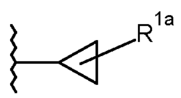 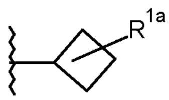 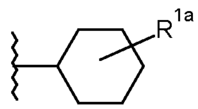

where each $R^{1a}$ represents one or two optional substituents selected from -OH and $C_{1-3}$ alkyl (e.g. methyl). In a particular embodiment of this aspect, $R^1$ represents $C_{3-6}$ cycloalkyl, such as optionally substituted cyclohexyl, optionally substituted cyclobutyl or unsubstituted (or optionally substituted) cyclopropyl, for instance:

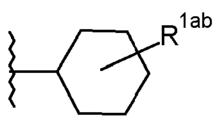 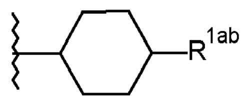

where each $R^{1ab}$ represents one or two optional substituents selected from those defined by $R^{1a}$, and in an embodiment, represents one optional substituent selected from -OH;

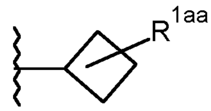 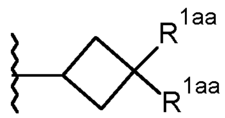

where each $R^{1aa}$ represents one or two optional substituents selected from those defined by $R^{1a}$, and in an embodiment represents two substituents, methyl and -OH; or

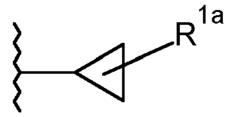 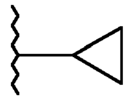

where $R^{1a}$ is as defined above, but where, in a particular embodiment, it is not present.

In an embodiment where $R^1$ represents aryl or heteroaryl, optionally substituted as defined herein, then it may represent: (i) phenyl; (ii) a 5- or 6-membered mono-cyclic heteroaryl group; or (iii) a 9- or 10-membered bicyclic heteroaryl group, all of which are optionally substituted by one to three substituents as defined herein. In an embodiment, the aforementioned aryl and heteroaryl groups are optionally substituted with one or two (e.g. one) substituent(s) selected from halo (e.g. fluoro), -OH, $C_{1-3}$ alkyl and $-OC_{1-3}$ alkyl. In one embodiment, $R^1$ represents phenyl or a mono-cyclic 6-membered heteroaryl group and in another embodiment it may represent a 9- or 10-membered (e.g. 9-membered) bicyclic heteroaryl group. Hence, in an embodiment, $R^1$ may represent:

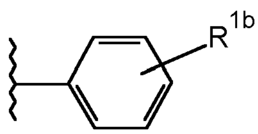 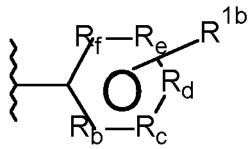

wherein $R^{1b}$ represents one or two optional substituents selected from halo, $-CH_3$, -OH and $-OCH_3$ (and in a further embodiment, such optional substituents are selected from fluoro and methoxy), and at least one of $R_b$, $R_c$, $R_d$, $R_e$ and $R_f$ represents a nitrogen heteroatom (and the others represent CH). In an embodiment, either one or two of $R_b$, $R_c$, $R_d$, $R_e$ and $R_f$ represent(s) a nitrogen heteroatom, for instance, $R_d$ represents nitrogen and, optionally, $R_b$ represents nitrogen, or, $R_c$ represents nitrogen. In an aspect: (i) $R_b$ and $R_d$ represent nitrogen; (ii) $R_d$ represents nitrogen; or (iii) $R_c$ represents nitrogen. Hence, $R^1$ may represent 3-pyridyl or 4-pyrimidinyl, both of which are optionally substituted as herein defined; however, in an embodiment, such groups are unsubstituted.

In another embodiment, $R^1$ may represent:

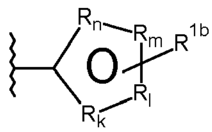 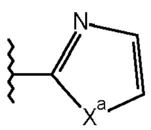 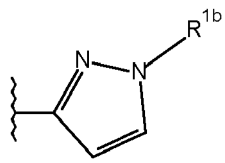

wherein $R^{1b}$ is as defined above (i.e. represents one or two optional substituents), and at least one of $R_k$, $R_l$, $R_m$ and $R_n$ represents a heteroatom, and in an embodiment, at least one of these represents N and the others are independently selected from CH, N, O and S (provided that the rules of valency are adhered to; above, $X^a$ may also represent NH, O or S); for instance, in an embodiment, one of $R_k$ and $R_l$ represents N, the other represents N, O, S or CH, and $R_m$ and $R_n$ each represent CH, and, in a further particular embodiment, $R_k$ and $R_l$ both represent N, and $R_m$ and $R_n$ each represent CH, so forming a 3-pyrazolyl group. As such, in a particular embodiment, $R^1$ represents 3-pyrazolyl. In another particular embodiment, $R^1$ represents a 3-pyrazolyl group (for instance in which $R_k$ and $R_l$ represents N, $R_n$ and $R_m$ represent CH, and $R^{1b}$ represents a $C_{1-4}$ alkyl (e.g. isopropyl) that is on the 1-(N) atom).

In a particular embodiment, $R^1$ represents

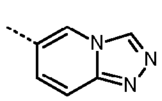 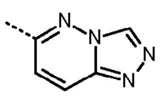 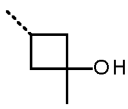

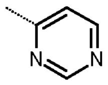 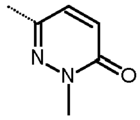 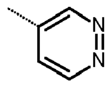 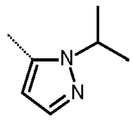

In more particular embodiment, $R^1$ represents

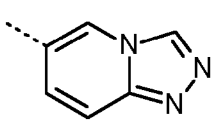 or 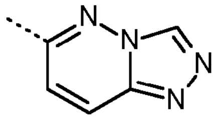

In an embodiment $R^1$ represents:

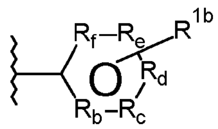 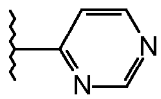

in which $R_b$ and $R_d$ represent a nitrogen atom, and, in an embodiment, there is no $R^{1b}$ substituent present.

In another embodiment $R^1$ represents:

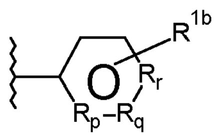 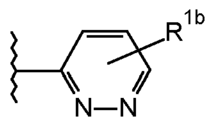 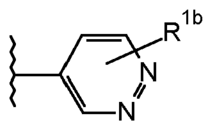 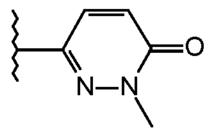

in which $R_p$ and $R_q$ both represent N and $R_r$ represents CH or $R_q$ and $R_r$ both represent N and $R_p$ represents CH, and $R^{1b}$ represents one or more optional substituent selected from -OH (or its tautomeric equivalent), $C_{1-3}$ alkyl (e.g. methyl) and $-OC_{1-2}$ alkyl.

In another embodiment, $R^1$ represents:

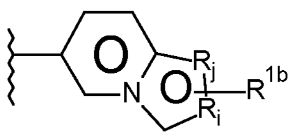 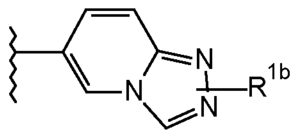

in which one of $R_i$ and $R_j$ represents N and the other represents C, or, both $R_i$ and $R_j$ represent N, and, in an embodiment, there is no $R^{1b}$ substituent present.

In a further embodiment, $R^1$ represents:

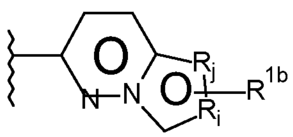 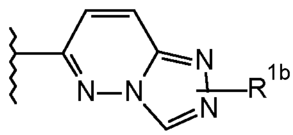

in which $R^i$, $R^j$ and $R^{1b}$ are as hereinbefore defined, for instance, in which any one or two of $R_i$ and $R_j$ represents N and the other, if applicable, represents CH and $R^{1b}$ represents one or more optional substituents as hereinbefore defined.

In an embodiment, $R^{2a}$ and $R^{2b}$ represent $C_{1-3}$ alkyl optionally substituted by one or more substituents selected from $-N(C_{1-2}\ alkyl)_2$ and $-OC_{1-2}$ alkyl, or $R^{2a}$ and $R^{2b}$ are linked together to form a 3- or 4-membered ring optionally substituted by one or more fluoro atoms. In another embodiment, one of $R^{2a}$ and $R^{2b}$ represents $C_{1-3}$ alkyl (e.g. unsubstituted methyl) and the other represents $C_{1-3}$ alkyl optionally substituted by one substituent selected from $-N(CH_3)_2$ and $-OCH_3$, or $R^{2a}$ and $R^{2b}$ are linked together to form a 4-membered ring optionally substituted by one or two fluoro atoms.

In a particular embodiment, $R^2$ represents 3,3-difluoro-1-azetidine, $-N(CH_3)_2$, $-N(CH_3)-CH_2-CH_2-N(CH_3)_2$ or $-N(CH_3)-CH_2-CH_2-OCH_3$.

In an embodiment $R^{3a}$ represents hydrogen and $R^{3b}$ represents $R^3$. In a preferred embodiment, $R^{3b}$ represents hydrogen and $R^{3a}$ represents $R^3$.

In an embodiment $R^3$ does not represent hydrogen. In an embodiment, $R^3$ represents; (i) halo (e.g. bromo or chloro); (ii) $C_{1-4}$ alkyl optionally substituted with one or more substituents independently selected from halo, -OH and $-OC_{1-2}$ alkyl; (iii) $C_{3-6}$ cycloalkyl (e.g. cyclopropyl); or (iv) $-OC_{1-3}$ alkyl. In an embodiment when $R^3$ represents optionally substituted $C_{1-4}$ alkyl, then it represents $C_{1-3}$ alkyl optionally substituted by one or more fluoro atoms. In an embodiment when $R^3$ represents $C_{3-6}$ cycloalkyl, then it represents cyclopropyl. In an embodiment when $R^3$ represents $-OC_{1-3}$ alkyl, then it represents $-OC_{1-2}$ alkyl (e.g. $-OCH_3$).

In a particular embodiment, $R^{3a}$ represents bromo, chloro or $-CF_3$ and $R^{3b}$ represents hydrogen.

The names of the compounds of the present invention were generated according to the nomenclature rules agreed upon by the Chemical Abstracts Service (CAS) using Advanced Chemical Development, Inc., software (ACD/-Name product version 10.01; Build 15494, 1 Dec 2006) or according to the nomenclature rules agreed upon by the International Union of Pure and Applied Chemistry (IUPAC) using Advanced Chemical Development, Inc., software (ACD/Name product version 10.01.0.14105, October 2006). In case of tautomeric forms, the name of the depicted tautomeric form of the structure was generated.

**Preparation of the compounds**

In an aspect of the invention, there is provided a process for the preparation of compounds of formula (I), where reference here is made to compounds of formula (I) as defined herein.

Compounds of formula (I) may be prepared by:

(i) reaction of a compound of formula (II),

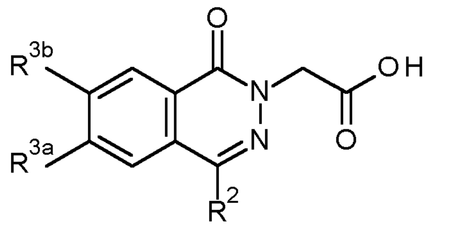

(II)

or

a derivative thereof (e.g. a salt), wherein $R^2$ and $R^{3a}$ and $R^{3b}$ are as hereinbefore defined, with a compound of formula (III),

$H_2N-R^1$ (III)

or a derivative thereof, wherein $R^1$ is as hereinbefore defined, under amide-forming reaction conditions (also referred to as amidation), for example in the presence of a suitable coupling reagent (e.g. propylphosphonic anhydride, 1-[bis(dimethyl-amino)methylene]-1H-1,2,3-triazolo[4,5-b]pyridinium 3-oxide hexafluorophosphate (*O*-(7-azabenzotriazol-1-yl)-*N*,*N*,*N'*,*N'*-tetramethyluronium hexafluorophosphate), 1,1'-carbonyldiimidazole, *N*, *N'*-dicyclohexylcarbodiimide, 1-(3-dimethylaminopropyl)-3-ethylcarbodiimide (or hydrochloride thereof), *N*,*N'*-disuccinimidyl carbonate, benzotriazol-1-yloxytris(dimethylamino)phosphonium hexafluoro-phosphate, 2-(1*H*-benzotriazol-1-yl)-1,1,3,3-tetramethyluronium hexa-fluorophosphate (i.e. *O*-(1H-benzotriazol-1-yl)-*N,N,N',N'*-tetramethyluronium hexafluorophosphate), benzotriazol-1-yloxytris-pyrrolidinophosphonium hexa-fluorophosphate, bromo-tris-pyrrolidinophosphonium hexafluorophosphate, 2-(1*H*-benzotriazol-1-yl)-1,1,3,3-tetramethyluronium tetra-fluorocarbonate, 1-cyclohexylcarbodiimide-3-propyloxymethyl polystyrene, *O*-benzotriazol-1-yl-*N,N,N',N'*-tetramethyluronium tetrafluoroborate), optionally in the presence of a suitable base (e.g. sodium hydride, sodium bicarbonate, potassium carbonate, pyridine, triethylamine, dimethylaminopyridine, diisopropylamine, sodium hydroxide, potassium *tert*-butoxide and/or lithium diisopropylamide (or variants thereof) and an appropriate solvent (e.g. tetrahydrofuran, pyridine, toluene, dichloromethane, chloroform, acetonitrile, dimethylformamide, trifluoromethylbenzene, dioxane or triethylamine). Such reactions may be performed in the presence of a further additive such as 1-hydroxybenzotriazole hydrate. Alternatively, a carboxylic acid group may be converted under standard conditions to the corresponding acyl chloride (e.g. in the presence of $SOCl_2$ or oxalyl

chloride), which acyl chloride is then reacted with a compound of formula (II), for example under similar conditions to those mentioned above;

(ii) reaction of a compound of formula (IV),

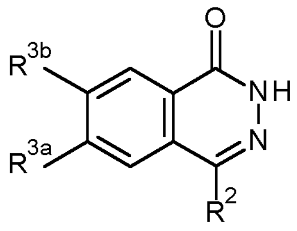

(IV)

wherein $R^2$ and $R^{3a}$ and $R^{3b}$ are as hereinbefore defined, with a compound of formula (V),

$LG^a$-$CH_2$-C(O)-N(H)$R^1$ (V)

wherein $LG^a$ represents a suitable leaving group (e.g. halo, such as chloro) and $R^1$ is as defined herein, under suitable reaction conditions, e.g. in the presence of an appropriate base, e.g. $Cs_2CO_3$, $K_2CO_3$ or LiHMDS, or the like, or alternative alkylation reaction conditions;

(iii) reaction of a compound of formula (IVA),

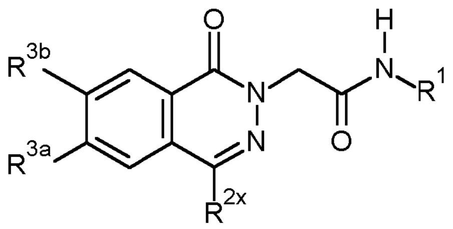

(IVA)

wherein $R^1$ and $R^{3a}$ and $R^{3b}$ are as hereinbefore defined, and $R^{2x}$ represents halo (e.g. iodo or preferably bromo), with a compound of formula (IVB),

HN($R^{2a}$)$R^{2b}$ (IVB)

wherein $R^{2a}$ and $R^{2b}$ are as hereinbefore defined, under appropriate reaction conditions such as in an amination reaction under appropriate conditions, e.g. using microwave reaction conditions and an excess of amine of formula (IVB), or, under standard amine coupling conditions, in the presence of a catalyst, *e.g.* CuI, a ligand, *e.g.* D/L-proline and a base, *e.g.* $K_2CO_3$;

(iv) by transformation (such transformation steps may also take place on intermediates) of a certain compound of formula (I) into another, for example:

- for compounds of formula (I) containing an alkene, reduction to a corresponding compound of formula (I) containing an alkane, under reduction conditions, e.g. with hydrogen in the presence of a suitable catalyst such as, for example, palladium on carbon, in a suitable reaction-inert solvent, such as, for example, ethanol or methanol;

- coupling to convert a halo or triflate group to e.g. an alkyl, alkenyl or cycloalkyl group, for example in the presence of a suitable coupling reagent, e.g. where the reagent comprises the appropriate alkyl, alkenyl or aryl/heteroaryl group attached to a suitable group such as $-B(OH)_2$, $-B(OR^{wx})_2$, zincates (e.g. including $-Zn(R^{wx})_2$, $-ZnBrR^{wx}$) or $-Sn(R^{wx})_3$, in which each $R^{wx}$ independently represents a $C_{1-6}$ alkyl group, or, in the case of $-B(OR^{wx})_2$, the respective $R^{wx}$ groups may be linked together to form a 4- to 6-membered cyclic group (such as a 4,4,5,5-tetramethyl-1,3,2-dioxaborolan-2-yl group), thereby forming e.g. a pinacolato boronate ester group. The reaction may be performed in the presence of a suitable catalyst system, e.g. a metal (or a salt or complex thereof) such as Pd, CuI, Pd/C, $PdCl_2$, $Pd(OAc)_2$, $Pd(Ph_3P)_2Cl_2$, $Pd(Ph_3P)_4$ (i.e. palladium tetrakistriphenylphosphine), $Pd_2$

(dba)$_3$ and/or $NiCl_2$ (preferred catalysts include RuPhos Pd G3, XPhos Pd and bis(tri-*tert*-butylphosphine)-palladium(0)) and optionally a ligand such as $PdCl_2$(dppf).DCM, *t*-$Bu_3P$, $(C_6H_{11})_3P$, $Ph_3P$, $AsPh_3$, P(*o*-Tol)$_3$, 1,2-bis(diphenyl-phosphino)ethane, 2,2'-bis(di-*tert*-butylphosphino)-1,1'-biphenyl, 2,2'-bis(diphenylphosphino)-1,1'-bi-naphthyl, 1,1'-bis(diphenyl-phosphino-ferrocene), 1,3-bis(diphenylphosphino)propane, XantPhos, or a mixture thereof, together with a suitable base, such as $Na_2CO_3$, $K_3PO_4$, $Cs_2CO_3$, NaOH, KOH, $K_2CO_3$, CsF, $Et_3N$, (*i*-Pr)$_2$NEt, *t*-BuONa or *t*-BuOK (or mixtures thereof; preferred bases include $Na_2CO_3$ and $K_2CO_3$) in a suitable solvent such as dioxane, toluene, ethanol, dimethylformamide, dimethoxyethane, ethylene glycol dimethyl ether, water, dimethylsulfoxide, acetonitrile, dimethylacetamide, *N*-methylpyrrolidinone, tetrahydrofuran or mixtures thereof (preferred solvents include dimethylformamide and dimethoxy ethane);

- reduction of a ketone to an alcohol, in the presence of suitable reducing conditions, e.g. $NaBH_4$ or the like;
- conversion of a -C(O)alkyl moiety to a -C(OH)(alkyl)(alkyl) moiety by reaction of an appropriate Grignard reagent, e.g. alkylMgBr;
- transformation of a alkene $=CH_2$ moiety to a carbonyl =O moiety, for instance, in the presence of AD-mix-Alpha and methane-sulfonamide, for instance a $-CH=CH_2$ moiety may be converted to a -C(O)H moiety (e.g. by reaction with osmium tetraoxide), which in turn may be converted to a $-CHF_2$ group by reaction with DAST;
- transformation of a ketone to an alcohol -OH moiety;
- alkylation of a -OH moiety (to -O-alkyl), under appropriate reaction conditions.

The compound of formula (II) may be prepared by hydrolysis of the corresponding carboxylic acid ester (for example under standard hydrolysis conditions, e.g. base hydrolysis in the presence of an alkali metal hydroxide (such as lithium hydroxide)), which in turn is prepared by reaction of a compound of formula (IV),

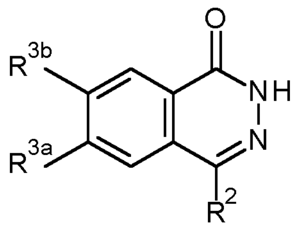

(IV)

wherein $R^2$ and $R^{3a}$ and $R^{3b}$ are as hereinbefore defined, with a compound of formula (VI),

$LG-CH_2-C(O)O-R^{aa}$ (VI)

wherein $R^{aa}$ represents $C_{1-6}$ alkyl (e.g. ethyl) and LG represents a suitable leaving group, such as halo (e.g. chloro), for instance under reaction conditions and using reagent such as those described herein.

Compound of formula (II) may be prepared by reaction of a compound of formula (VII)

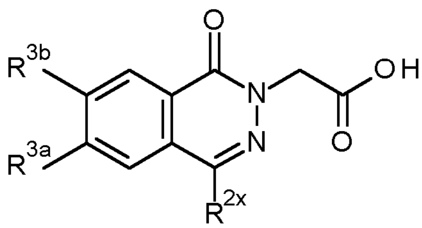

(VII)

or a derivative thereof (e.g. an ester), in which $R^{3a}$ and $R^{3b}$ and $R^{2x}$ are as hereinbefore defined, under reaction conditions such as those mentioned above, e.g. amine coupling from a corresponding compound in which $R^{2x}$ is bromo, under conditions such as those described herein (e.g. in the description below or in respect of preparation of compounds of formula (I) process step (iii)).

Compounds of formula (VII) may be prepared by reaction of a compound of formula (VIII),

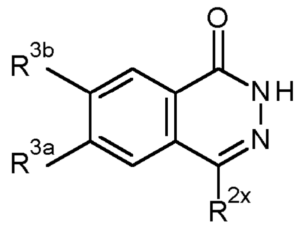

(VIII)

wherein $R^{2x}$ and $R^{3a}$ and $R^{3b}$ are as hereinbefore defined, with a compound of formula (VI) as defined above (followed by, if needed, conversion of the ester to the carboxylic acid), under reaction conditions described herein.

Compounds of formula (VII) may be made in accordance with the following Scheme 1 and using reaction conditions such as those described herein:

**Scheme 1**

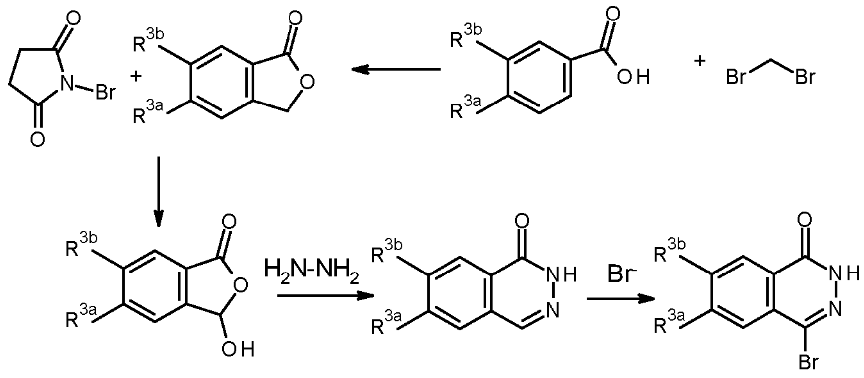


(VIII)

In general the compounds of formula (I) can therefore be made with reference to the procedures above. However, in the interests of versatility, further schemes are provided below in order to provide intermediate and final compounds of formula (I). Further details are provided in the scheme below (as well as in the specific details of the experimental described hereinafter).

Scheme 2 below provides other routes to compounds of formula (IV), also referred to above as compounds (M6). For instance, as per the scheme, compounds of formula (M6A) may undergo bromination to provide a compound of formula (IV) but in which $R^2$ represents bromo (M6B). Thereafter further variations of $R^2$ groups in downstream products may be obtained. For instance, from (M6B), a Buchwald coupling may provide further compounds such as those of formula (IV) in which $R^2$ represents an amino (e.g. -N($R^{2a}$)($R^{2b}$) group, or an another amine group which may be converted to such a group), for instance by reaction in the presence of an amine (e.g. HN($R^{2a}$)$R^{2b}$) and an appropriate catalyst (e.g. Pd-based catalyst or another as described herein), optionally with a suitable base and ligand (for example one as described herein, in respect of preparations of compounds of formula (I)).

**Scheme 2**

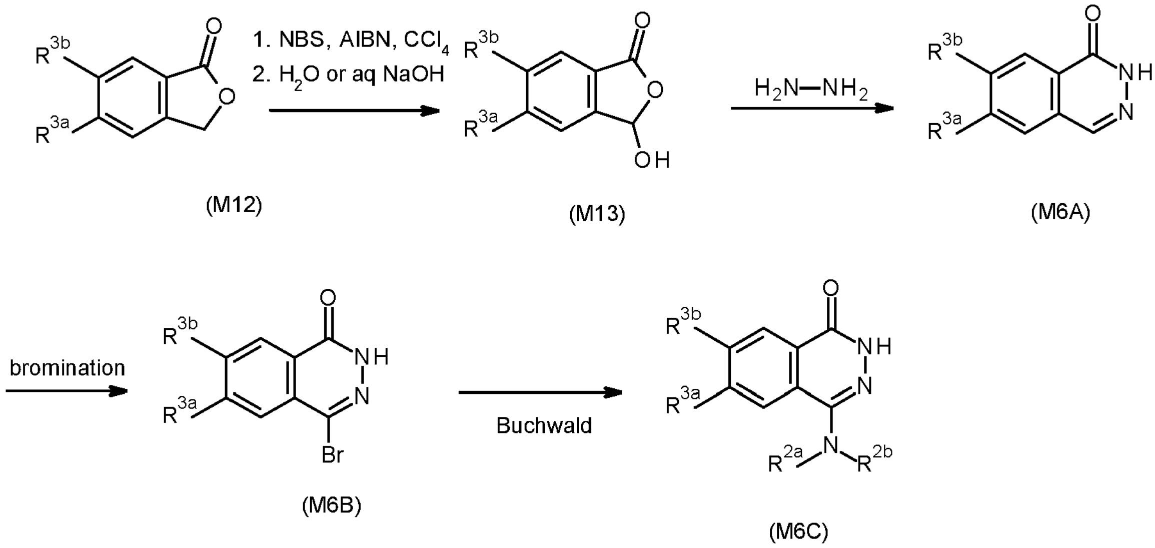


(M12) (M13) (M6A)

(M6B) (M6C)

Certain intermediate compounds may be commercially available, may be known in the literature, or may be obtained either by analogy with the processes described herein, or by conventional synthetic procedures, in accordance with standard techniques, from available starting materials using appropriate reagents and reaction conditions.

Certain substituents on/in final compounds of formula (I) or relevant intermediates may be modified one or more times, after or during the processes described above by way of methods that are well known to those skilled in the art. Examples of such methods include substitutions, reductions, oxidations, alkylations, acylations, hydrolyses, esterifications, etherifications, halogenations, nitrations or couplings.

Compounds of formula (I) may be isolated from their reaction mixtures using conventional techniques (e.g. recrystallisations, where possible under standard conditions).

It will be appreciated by those skilled in the art that, in the processes described above and hereinafter, the functional groups of intermediate compounds may need to be protected by protecting groups.

The need for such protection will vary depending on the nature of the remote functionality and the conditions of the preparation methods (and the need can be readily determined by one skilled in the art). Suitable amino-protecting groups include acetyl, trifluoroacetyl, t-butoxycarbonyl (BOC), benzyloxycarbonyl (CBz), 9-fluorenyl-methyleneoxycarbonyl (Fmoc) and 2,4,4-trimethylpentan-2-yl (which may be deprotected by reaction in the presence of an acid, e.g. HCl in water/alcohol (e.g. MeOH)) or the like. The need for such protection is readily determined by one skilled in the art. For example the a -C(O)O-*tert*-butyl ester moiety may serve as a protecting group for a -C(O)OH moiety, and hence the former may be converted to the latter for instance by reaction in the presence of a mild acid (e.g. TFA, or the like).

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described herein may be converted chemically to unprotected compounds using standard deprotection techniques.

The type of chemistry involved will dictate the need, and type, of protecting groups as well as the sequence for accomplishing the synthesis.

The use of protecting groups is fully described in "Protective Groups in Organic Synthesis", 3rd edition, T.W. Greene & P.G.M. Wuts, Wiley-Interscience (1999).

The compounds of formula (I) as prepared in the hereinabove described processes may be synthesized in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting

materials.

**PHARMACOLOGY**

There is evidence for a role of NLRP3-induced IL-1 and IL-18 in the inflammatory responses occurring in connection with, or as a result of, a multitude of different disorders (Menu et al., Clinical and Experimental Immunology, 2011, 166, 1-15; Strowig et al., Nature, 2012, 481, 278-286). NLRP3 mutations have been found to be responsible for a set of rare autoinflammatory diseases known as CAPS (Ozaki et al., J. Inflammation Research, 2015, 8, 15-27; Schroder et al., Cell, 2010, 140: 821-832; Menu et al., Clinical and Experimental Immunology, 2011, 166, 1-15). CAPS are heritable diseases characterized by recurrent fever and inflammation and are comprised of three autoinflammatory disorders that form a clinical continuum. These diseases, in order of increasing severity, are familial cold autoinflammatory syndrome (FCAS), Muckle-Wells syndrome (MWS), and chronic infantile cutaneous neurological articular syndrome (CINCA; also called neonatal-onset multisystem inflammatory disease, NOMID), and all have been shown to result from gain-of-function mutations in the NLRP3 gene, which leads to increased secretion of IL-1 beta. NLRP3 has also been implicated in a number of autoinflammatory diseases, including pyogenic arthritis, pyoderma gangrenosum and acne (PAPA), Sweet's syndrome, chronic nonbacterial osteomyelitis (CNO), and acne vulgaris (Cook et al., Eur. J. Immunol., 2010, 40, 595-653).

A number of autoimmune diseases have been shown to involve NLRP3 including, in particular, multiple sclerosis, type-1 diabetes (T1D), psoriasis, rheumatoid arthritis (RA), Behcet's disease, Schnitzler syndrome, macrophage activation syndrome (Braddock et al., Nat. Rev. Drug Disc. 2004, 3, 1-10; Inoue et al., Immunology, 2013, 139, 11-18; Coll et a/., Nat. Med. 2015, 21(3), 248-55; Scott et al., Clin. Exp. Rheumatol. 2016, 34(1), 88-93), systemic lupus erythematosus and its complications such as lupus nephritis (Lu et al., J. Immunol., 2017, 198(3), 1119-29), and systemic sclerosis (Artlett et al., Arthritis Rheum. 2011, 63(11), 3563-74). NLRP3 has also been shown to play a role in a number of lung diseases including chronic obstructive pulmonary disorder (COPD), asthma (including steroid-resistant asthma), asbestosis, and silicosis (De Nardo et al., Am. J. Pathol., 2014, 184: 42-54; Kim et al., Am. J. Respir. Crit. Care Med, 2017, 196(3), 283-97). NLRP3 has also been suggested to have a role in a number of central nervous system conditions, including Multiple Sclerosis (MS), Parkinson's disease (PD), Alzheimer's disease (AD), dementia, Huntington's disease, cerebral malaria, brain injury from pneumococcal meningitis (Walsh et al., Nature Reviews, 2014, 15, 84-97; and Dempsey et al., Brain. Behav. Immun. 2017, 61, 306-16), intracranial aneurysms (Zhang et al., J. Stroke and Cerebrovascular Dis., 2015, 24, 5, 972-9), and traumatic brain injury (Ismael et al., J. Neurotrauma., 2018, 35(11), 1294-1303). NLRP3 activity has also been shown to be involved in various metabolic diseases including type 2 diabetes (T2D) and its organ-specific complications, atherosclerosis, obesity, gout, pseudo-gout, metabolic syndrome (Wen et al., Nature Immunology, 2012, 13, 352-357; Duewell et al., Nature, 2010, 464, 1357-1361; Strowig et al., Nature, 2014, 481, 278- 286), and non-alcoholic steatohepatitis (Mridha et al., J. Hepatol. 2017, 66(5), 1037-46). A role for NLRP3 via IL-1 beta has also been suggested in atherosclerosis, myocardial infarction (van Hout et al., Eur. Heart J. 2017, 38(11), 828-36), heart failure (Sano et al., J. Am. Coll. Cardiol. 2018, 71(8), 875-66), aortic aneurysm and dissection (Wu et al., Arteriosc/er. Thromb. Vase. Biol., 2017,37(4), 694-706), and other cardiovascular events (Ridker et al., N. Engl. J. Med., 2017, 377(12), 1119-31).

Other diseases in which NLRP3 has been shown to be involved include: ocular diseases such as both wet and dry age-related macular degeneration (Doyle et al., Nature Medicine, 2012, 18, 791-798; Tarallo et al., Cell 2012, 149(4), 847-59), diabetic retinopathy (Loukovaara et al., Acta Ophthalmol., 2017, 95(8), 803-8), non-infectious uveitis and optic nerve damage (Puyang et al., Sci. Rep. 2016, 6, 20998); liver diseases including non-alcoholic steatohepatitis (NASH) and acute alcoholic hepatitis (Henao-Meija et al., Nature, 2012, 482, 179-185); inflammatory reactions in the lung and skin (Primiano et al., J. Immunol. 2016, 197(6), 2421-33) including contact hypersensitivity (such as bullous pemphigoid (Fang et al., J Dermatol Sci. 2016, 83(2), 116-23)), atopic dermatitis (Niebuhr et al., Allergy, 2014, 69(8), 1058-67), Hidradenitis suppurativa (Alikhan et al., J. Am. Acad. Dermatol., 2009 ,60(4), 539-61), and sarcoidosis (Jager et al., Am. J. Respir. Crit. Care Med., 2015, 191, A5816); inflammatory reactions in the joints (Braddock et al., Nat. Rev. Drug Disc, 2004, 3, 1-10); amyotrophic lateral sclerosis (Gugliandolo et al., Int. J. Mo/. Sci., 2018, 19(7), E1992); cystic fibrosis (lannitti et al., Nat. Commun., 2016, 7, 10791); stroke (Walsh et al., Nature Reviews, 2014, 15, 84-97); chronic kidney disease (Granata et al., PLoS One 2015, 10(3), eoi22272); and inflammatory bowel diseases including ulcerative colitis and Crohn's disease (Braddock et al., Nat. Rev. Drug Disc, 2004, 3, 1-10; Neudecker et a/., J. Exp. Med. 2017, 214(6), 1737-52; Lazaridis et al., Dig. Dis. Sci. 2017, 62(9), 2348-56). The NLRP3 inflammasome has been found to be activated in response to oxidative stress. NLRP3 has also been shown to be involved in inflammatory hyperalgesia (Dolunay et al., Inflammation, 2017, 40, 366-86).

Activation of the NLRP3 inflammasome has been shown to potentiate some pathogenic infections such as influenza and Leishmaniasis (Tate et al., Sci Rep., 2016, 10(6), 27912-20; Novias et al., PLOS Pathogens 2017, 13(2), e1006196).

NLRP3 has also been implicated in the pathogenesis of many cancers (Menu et al., Clinical and Experimental Immunology, 2011, 166, 1-15). For example, several previous studies have suggested a role for IL-1 beta in cancer invasiveness, growth and metastasis, and inhibition of IL-1 beta with canakinumab has been shown to reduce the

incidence of lung cancer and total cancer mortality in a randomised, double-blind, placebo-controlled trial (Ridker et al., Lancet., 2017, 390(10105), 1833-42). Inhibition of the NLRP3 inflammasome or IL-1 beta has also been shown to inhibit the proliferation and migration of lung cancer cells in vitro (Wang et al., Onco/ Rep., 2016, 35(4), 2053-64). A role for the NLRP3 inflammasome has been suggested in myelodysplastic syndromes, myelofibrosis and other myeloproliferative neoplasms, and acute myeloid leukemia (AML) (Basiorka et al., Blood, 2016, 128(25), 2960-75.) and also in the carcinogenesis of various other cancers including glioma (Li et al., Am. J. Cancer Res. 2015, 5(1), 442-9), inflammation-induced tumors (Allen et al., J. Exp. Med. 2010, 207(5), 1045-56; Hu et al., PNAS., 2010, 107(50), 21635-40), multiple myeloma (Li et al., Hematology, 2016 21(3), 144-51), and squamous cell carcinoma of the head and neck (Huang et al., J. Exp. Clin. Cancer Res., 2017, 36(1), 116). Activation of the NLRP3 inflammasome has also been shown to mediate chemoresistance of tumor cells to 5-Fluorouracil (Feng et al., J. Exp. Clin. Cancer Res., 2017, 36(1), 81), and activation of NLRP3 inflammasome in peripheral nerve contributes to chemotherapy-induced neuropathic pain (Jia et al., Mol. Pain., 2017, 13, 1-11). NLRP3 has also been shown to be required for the efficient control of viruses, bacteria, and fungi.

The activation of NLRP3 leads to cell pyroptosis and this feature plays an important part in the manifestation of clinical disease (Yan-gang et al., Cell Death and Disease, 2017, 8(2), 2579; Alexander et al., Hepatology, 2014, 59(3), 898-910; Baldwin et al., J. Med. Chem., 2016, 59(5), 1691- 1710; Ozaki et a/., J. Inflammation Research, 2015, 8, 15-27; Zhen et a/., Neuroimmunology Neuroinflammation, 2014, 1(2), 60-65; Mattia et a/., J Med. Chem., 2014, 57(24), 10366-82; Satoh et al., Cell Death and Disease, 2013, 4, 644). Therefore, it is anticipated that inhibitors of NLRP3 will block pyroptosis, as well as the release of pro-inflammatory cytokines (e.g. IL-1 beta) from the cell.

Hence, the compounds of formula (I), as described herein (e.g. in any of the embodiments described herein, including by the examples, and/or in any of the forms described herein, e.g. in a salt form or free form, etc) exhibit valuable pharmacological properties, e.g. NLRP3 inhibiting properties on the NLRP3 inflammasome pathway e.g. as indicated *in vitro* tests as provided herein, and are therefore indicated for therapy or for use as research chemicals, e.g. as tool compounds. Compounds of formula (I) may be useful in the treatment of an indication selected from: inflammasome-related diseases/disorders, immune diseases, inflammatory diseases, auto-immune diseases, or auto-inflammatory diseases, for example, of diseases, disorders or conditions in which NLRP3 signaling contributes to the pathology, and/or symptoms, and/or progression, and which may be responsive to NLRP3 inhibition and which may be treated or prevented, according to any of the methods/uses described herein, e.g. by use or administration of a compound of formula (I), and, hence, in an embodiment, such indications may include:

I. Inflammation, including inflammation occurring as a result of an inflammatory disorder, e.g. an autoinflammatory disease, inflammation occurring as a symptom of a non- inflammatory disorder, inflammation occurring as a result of infection, or inflammation secondary to trauma, injury or autoimmunity. Examples of inflammation that may be treated or prevented include inflammatory responses occurring in connection with, or as a result of:

a. a skin condition such as contact hypersensitivity, bullous pemphigoid, sunburn, psoriasis, atopical dermatitis, contact dermatitis, allergic contact dermatitis, seborrhoetic dermatitis, lichen planus, scleroderma, pemphigus, epidermolysis bullosa, urticaria, erythemas, or alopecia;

b. a joint condition such as osteoarthritis, systemic juvenile idiopathic arthritis, adult-onset Still's disease, relapsing polychondritis, rheumatoid arthritis, juvenile chronic arthritis, crystal induced arthropathy (e.g. pseudo-gout, gout), or a seronegative spondyloarthropathy (e.g. ankylosing spondylitis, psoriatic arthritis or Reiter's disease);

c. a muscular condition such as polymyositis or myasthenia gravis;

d. a gastrointestinal tract condition such as inflammatory bowel disease (including Crohn's disease and ulcerative colitis), gastric ulcer, coeliac disease, proctitis, pancreatitis, eosinopilic gastro- enteritis, mastocytosis, antiphospholipid syndrome, or a food-related allergy which may have effects remote from the gut (e.g., migraine, rhinitis or eczema);

e. a respiratory system condition such as chronic obstructive pulmonary disease (COPD), asthma (including bronchial, allergic, intrinsic, extrinsic or dust asthma, and particularly chronic or inveterate asthma, such as late asthma and airways hyper- responsiveness), bronchitis, rhinitis (including acute rhinitis, allergic rhinitis, atrophic rhinitis, chronic rhinitis, rhinitis caseosa, hypertrophic rhinitis, rhinitis pumlenta, rhinitis sicca, rhinitis medicamentosa, membranous rhinitis, seasonal rhinitis e.g. hay fever, and vasomotor rhinitis), sinusitis, idiopathic pulmonary fibrosis (IPF), sarcoidosis, farmer's lung, silicosis, asbestosis, adult respiratory distress syndrome, hypersensitivity pneumonitis, or idiopathic interstitial pneumonia;

f. a vascular condition such as atherosclerosis, Behcet's disease, vasculitides, or Wegener's granulomatosis;

g. an immune condition, e.g. autoimmune condition, such as systemic lupus erythematosus (SLE), Sjogren's syndrome, systemic sclerosis, Hashimoto's thyroiditis, type I diabetes, idiopathic thrombocytopenia purpura, or Graves' disease;

h. an ocular condition such as uveitis, allergic conjunctivitis, or vernal conjunctivitis;

i. a nervous condition such as multiple sclerosis or encephalomyelitis;

j. an infection or infection-related condition, such as Acquired Immunodeficiency Syndrome (AIDS), acute or chronic bacterial infection, acute or chronic parasitic infection, acute or chronic viral infection, acute or chronic fungal infection, meningitis, hepatitis (A, B or C, or other viral hepatitis), peritonitis, pneumonia, epiglottitis, malaria, dengue hemorrhagic fever, leishmaniasis, streptococcal myositis, mycobacterium tuberculosis, mycobacterium avium intracellulare, Pneumocystis carinii pneumonia, orchitis/epidydimitis, legionella, Lyme disease, influenza A, Epstein Barr virus, viral encephalitis/aseptic meningitis, or pelvic inflammatory disease;

k. a renal condition such as mesangial proliferative glomerulonephritis, nephrotic syndrome, nephritis, glomerular nephritis, acute renal failure, uremia, or nephritic syndrome;

l. a lymphatic condition such as Castleman's disease;

m. a condition of, or involving, the immune system, such as hyper IgE syndrome, lepromatous leprosy, familial hemophagocytic lymphohistiocytosis, or graft versus host disease;

n. a hepatic condition such as chronic active hepatitis, non-alcoholic steatohepatitis (NASH), alcohol-induced hepatitis, non-alcoholic fatty liver disease (NAFLD), alcoholic fatty liver disease (AFLD), alcoholic steatohepatitis (ASH) or primary biliary cirrhosis;

o. a cancer, including those cancers listed herein below;

p. a burn, wound, trauma, haemorrhage or stroke;

q. radiation exposure;

r. obesity; and/or

s. pain such as inflammatory hyperalgesia;

II. Inflammatory disease, including inflammation occurring as a result of an inflammatory disorder, e.g. an autoinflammatory disease, such as cryopyrin-associated periodic syndromes (CAPS), Muckle-Wells syndrome (MWS), familial cold autoinflammatory syndrome (FCAS), familial Mediterranean fever (FMF), neonatal onset multisystem inflammatory disease (NOMID), Majeed syndrome, pyogenic arthritis, pyoderma gangrenosum and acne syndrome (PAPA), adult-onset Still's disease (AOSD), haploinsufficiency of A20 (HA20), pediatric granulomatous arthritis (PGA), PLCG2-associated antibody deficiency and immune dysregulation (PLAID), PLCG2- associated autoinflammatory, antibody deficiency and immune dysregulation (APLAID), or sideroblastic anaemia with B-cell immunodeficiency, periodic fevers and developmental delay (SIFD);

III. Immune diseases, e.g. auto-immune diseases, such as acute disseminated encephalitis, Addison's disease, ankylosing spondylitis, antiphospholipid antibody syndrome (APS), anti-synthetase syndrome, aplastic anemia, autoimmune adrenalitis, autoimmune hepatitis, autoimmune oophoritis, autoimmune polyglandular failure, autoimmune thyroiditis, Coeliac disease, Crohn's disease, type 1 diabetes (T1D), Goodpasture's syndrome, Graves' disease, Guillain-Barre syndrome (GBS), Hashimoto's disease, idiopathic thrombocytopenic purpura, Kawasaki's disease, lupus erythematosus including systemic lupus erythematosus (SLE), multiple sclerosis (MS) including primary progressive multiple sclerosis (PPMS), secondary progressive multiple sclerosis (SPMS) and relapsing remitting multiple sclerosis (RRMS), myasthenia gravis, opsoclonus myoclonus syndrome (OMS), optic neuritis, Ord's thyroiditis, pemphigus, pernicious anaemia, polyarthritis, primary biliary cirrhosis, rheumatoid arthritis (RA), psoriatic arthritis, juvenile idiopathic arthritis or Still's disease, refractory gouty arthritis, Reiter's syndrome, Sjogren's

syndrome, systemic sclerosis a systemic connective tissue disorder, Takayasu's arteritis, temporal arteritis, warm autoimmune hemolytic anemia, Wegener's granulomatosis, alopecia universalis, Beliefs disease, Chagas' disease, dysautonomia, endometriosis, hidradenitis suppurativa (HS), interstitial cystitis, neuromyotonia, psoriasis, sarcoidosis, scleroderma, ulcerative colitis, Schnitzler syndrome, macrophage activation syndrome, Blau syndrome, giant cell arteritis, vitiligo or vulvodynia;

IV. Cancer including lung cancer, renal cell carcinoma, non-small cell lung carcinoma (NSCLC), Langerhans cell histiocytosis (LCH), myeloproliferative neoplasm (MPN), pancreatic cancer, gastric cancer, myelodysplastic syndrome (MOS), leukaemia including acute lymphocytic leukaemia (ALL) and acute myeloid leukaemia (AML), promyelocytic leukemia (APML, or APL), adrenal cancer, anal cancer, basal and squamous cell skin cancer, bile duct cancer, bladder cancer, bone cancer, brain and spinal cord tumours, breast cancer, cervical cancer, chronic lymphocytic leukaemia (CLL), chronic myeloid leukaemia (CML), chronic myelomonocytic leukaemia (CMML), colorectal cancer, endometrial cancer, oesophagus cancer, Ewing family of tumours, eye cancer, gallbladder cancer, gastrointestinal carcinoid tumours, gastrointestinal stromal tumour (GIST), gestational trophoblastic disease, glioma, Hodgkin lymphoma, Kaposi sarcoma, kidney cancer, laryngeal and hypopharyngeal cancer, liver cancer, lung carcinoid tumour, lymphoma including cutaneous T cell lymphoma, malignant mesothelioma, melanoma skin cancer, Merkel cell skin cancer, multiple myeloma, nasal cavity and paranasal sinuses cancer, nasopharyngeal cancer, neuroblastoma, non-Hodgkin lymphoma, non-small cell lung cancer, oral cavity and oropharyngeal cancer, osteosarcoma, ovarian cancer, penile cancer, pituitary tumours, prostate cancer, retinoblastoma, rhabdomyosarcoma, salivary gland cancer, skin cancer, small cell lung cancer, small intestine cancer, soft tissue sarcoma, stomach cancer, testicular cancer, thymus cancer, thyroid cancer including anaplastic thyroid cancer, uterine sarcoma, vaginal cancer, vulvar cancer, Waldenstrom macroglobulinemia, and Wilms tumour;

V. Infections including viral infections (e.g. from influenza virus, human immunodeficiency virus (HIV), alphavirus (such as Chikungunya and Ross River virus), flaviviruses (such as Dengue virus and Zika virus), herpes viruses (such as Epstein Barr Virus, cytomegalovirus, Varicella-zoster virus, and KSHV), poxviruses (such as vaccinia virus (Modified vaccinia virus Ankara) and Myxoma virus), adenoviruses (such as Adenovirus 5), papillomavirus, or SARS-CoV-2) bacterial infections (e.g. from Staphylococcus aureus, Helicobacter pylori, Bacillus anthracis, Bordetella pertussis, Burkholderia pseudomallei, Corynebacterium diphtheriae, Clostridium tetani, Clostridium botulinum, Streptococcus pneumoniae, Streptococcus pyogenes, Listeria monocytogenes, Hemophilus influenzae, Pasteurella multicida, Shigella dysenteriae, Mycobacterium tuberculosis, Mycobacterium leprae, Mycoplasma pneumoniae, Mycoplasma hominis, Neisseria meningitidis, Neisseria gonorrhoeae, Rickettsia rickettsii, Legionella pneumophila, Klebsiella pneumoniae, Pseudomonas aeruginosa, Propionibacterium acnes, Treponema pallidum, Chlamydia trachomatis, Vibrio cholerae, Salmonella typhimurium, Salmonella typhi, Borrelia burgdorferi or Yersinia pestis), fungal infections (e.g. from Candida or Aspergillus species), protozoan infections (e.g. from Plasmodium, Babesia, Giardia, Entamoeba, Leishmania or Trypanosomes), helminth infections (e.g. from schistosoma, roundworms, tapeworms or flukes), and prion infections;

VI. Central nervous system diseases such as Parkinson's disease, Alzheimer's disease, dementia, motor neuron disease, Huntington's disease, cerebral malaria, brain injury from pneumococcal meningitis, intracranial aneurysms, traumatic brain injury, multiple sclerosis, and amyotrophic lateral sclerosis;

VII. Metabolic diseases such as type 2 diabetes (T2D), atherosclerosis, obesity, gout, and pseudo-gout;

VIII. Cardiovascular diseases such as hypertension, ischaemia, reperfusion injury including post-MI ischemic reperfusion injury, stroke including ischemic stroke, transient ischemic attack, myocardial infarction including recurrent myocardial infarction, heart failure including congestive heart failure and heart failure with preserved ejection fraction, embolism, aneurysms including abdominal aortic aneurysm, cardiovascular risk reduction (CvRR), and pericarditis including Dressler's syndrome;

IX. Respiratory diseases including chronic obstructive pulmonary disorder (COPD), asthma such as allergic asthma and steroid-resistant asthma, asbestosis, silicosis, nanoparticle induced inflammation, cystic fibrosis, and idiopathic pulmonary fibrosis;

X. Liver diseases including non-alcoholic fatty liver disease (NAFLD) and nonalcoholic steatohepatitis (NASH) including advanced fibrosis stages F3 and F4, alcoholic fatty liver disease (AFLD), and alcoholic steatohepatitis (ASH);

XI. Renal diseases including acute kidney disease, hyperoxaluria, chronic kidney disease, oxalate nephropathy, nephrocalcinosis, glomerulonephritis, and diabetic nephropathy;

XII. Ocular diseases including those of the ocular epithelium, age-related macular degeneration (AMO) (dry and wet), uveitis, corneal infection, diabetic retinopathy, optic nerve damage, dry eye, and glaucoma;

XIII. Skin diseases including dermatitis such as contact dermatitis and atopic dermatitis, contact hypersensitivity, sunburn, skin lesions, hidradenitis suppurativa (HS), other cyst-causing skin diseases, and acne conglobata;

XIV.Lymphatic conditions such as lymphangitis, and Castleman's disease;

XV. Psychological disorders such as depression, and psychological stress;

XVI. Graft versus host disease;

XVII. Bone diseases including osteoporosis, osteopetrosis;

XVIII. Blood disease including sickle cell disease;

XIX. Allodynia including mechanical allodynia; and

XX. Any disease where an individual has been determined to carry a germline or somatic non-silent mutation in NLRP3.

More specifically the compounds of formula (I) may be useful in the treatment of an indication selected from: inflammasome-related diseases/disorders, immune diseases, inflammatory diseases, auto-immune diseases, or auto-inflammatory diseases, for example, autoinflammatory fever syndromes (e.g., cryopyrin-associated periodic syndrome), sickle cell disease, systemic lupus erythematosus (SLE), liver related diseases/disorders (e.g. chronic liver disease, viral hepatitis, non-alcoholic steatohepatitis (NASH), alcoholic steatohepatitis, and alcoholic liver disease), inflammatory arthritis related disorders (e.g. gout, pseudogout (chondrocalcinosis), osteoarthritis, rheumatoid arthritis, arthropathy e.g. acute, chronic), kidney related diseases (e.g. hyperoxaluria, lupus nephritis, Type I/Type II diabetes and related complications (e.g. nephropathy, retinopathy), hypertensive nephropathy, hemodialysis related inflammation), neuroinflammation-related diseases (e.g. multiple sclerosis, brain infection, acute injury, neurodegenerative diseases, Alzheimer's disease), cardiovascular/metabolic diseases/disorders (e.g. cardiovascular risk reduction (CvRR), hypertension, atherosclerosis, Type I and Type II diabetes and related complications, peripheral artery disease (PAD), acute heart failure), inflammatory skin diseases (e.g. hidradenitis suppurativa, acne), wound healing and scar formation, asthma, sarcoidosis, age-related macular degeneration, and cancer related diseases/disorders (e.g. colon cancer, lung cancer, myeloproliferative neoplasms, leukemias, myelodysplastic syndromes (MOS), myelofibrosis). In particular, autoinflammatory fever syndromes (e.g. CAPS), sickle cell disease, Type I/Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MOS), myelofibrosis).

In particular, compounds of formula (I), may be useful in the treatment of a disease or disorder selected from autoinflammatory fever syndromes (e.g. CAPS), sickle cell disease, Type I/ Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MOS), myelofibrosis). Thus, as a further aspect, the present invention provides the use of a compound of formula (I) (hence, including a compound as defined by any of the embodiments/forms/examples herein) in therapy. In a further embodiment, the therapy is selected from a disease, which may be treated by inhibition of NLRP3 inflammasome. In another embodiment, the disease is as defined in any of the lists herein. Hence, there is provided any one of the compounds of formula (I) described herein (including any of the embodiments/forms/examples) for use in the treatment of any of the diseases or disorders described herein (e.g. as described in the aforementioned lists).

## PHARMACEUTICAL COMPOSITIONS AND COMBINATIONS

In an embodiment, the invention also relates to a composition comprising a pharmaceutically acceptable carrier and, as active ingredient, a therapeutically effective amount of a compound of formula (I). The compounds of formula (I) may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

In an embodiment, and depending on the mode of administration, the pharmaceutical composition will preferably comprise from 0.05 to 99 % by weight, more preferably from 0.1 to 70 % by weight, even more preferably from 0.1 to 50 % by weight of the active ingredient(s), and, from 1 to 99.95 % by weight, more preferably from 30 to 99.9 % by weight, even more preferably from 50 to 99.9 % by weight of a pharmaceutically acceptable carrier, all percentages being based on the total weight of the composition.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof. The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 gram, e.g. in the range from 10 to 50 mg/kg body weight.

In an embodiment, there is provided a combination comprising a therapeutically effective amount of a compound of formula (I), according to any one of the embodiments described herein, and another therapeutic agent (including one or more therapeutic agents). In a further embodiment, there is provided such a combination wherein the other therapeutic agent is selected from (and where there is more than one therapeutic agent, each is independently selected from): farnesoid X receptor (FXR) agonists; anti-steatotics; anti-fibrotics; JAK inhibitors; checkpoint inhibitors including anti-PD1 inhibitors, anti-LAG-3 inhibitors, anti-TIM-3 inhibitors, or anti-POL 1 inhibitors; chemotherapy, radiation therapy and surgical procedures; urate-lowering therapies; anabolics and cartilage regenerative therapy; blockade of IL-17; complement inhibitors; Bruton's tyrosine Kinase inhibitors (BTK inhibitors); Toll Like receptor inhibitors (TLR7/8 inhibitors); CAR-T therapy; anti-hypertensive agents; cholesterol lowering agents; leukotriene A4 hydrolase (LTAH4) inhibitors; SGLT2 inhibitors; 132-agonists; anti-inflammatory agents; nonsteroidal anti-inflammatory drugs ("NSAIDs"); acetylsalicylic acid drugs (ASA) including aspirin; paracetamol; regenerative therapy treatments; cystic fibrosis treatments; or atherosclerotic treatment. In a further embodiment, there is also provided such (a) combination(s) for use as described herein in respect of compounds of formula (I), e.g. for use in the treatment of a disease or disorder in which the NLRP3 signaling contributes to the pathology, and/or symptoms, and/or progression, of said disease/disorder, or, a disease or disorder associated with NLRP3 activity (including NLRP3 inflammasome activity), including inhibiting NLRP3 inflammasome activity, and in this respect the specific disease/disorder mentioned herein apply equally here. There may also be provided methods as described herein in respect of compounds of formula (I), but wherein the method comprises administering a therapeutically effective amount of such combination (and, in an embodiment, such method may be to treat a disease or disorder mentioned herein in the context of inhibiting NLRP3 inflammasome activity). The combinations mentioned herein may be in a single preparation or they may be formulated in separate preparations so that they can be administered simultaneously, separately or sequentially. Thus, in an embodiment, the present invention also relates to a combination product containing (a) a compound according to the invention, according to any one of the embodiments described herein, and (b) one or more other therapeutic agents (where such therapeutic agents are as described herein), as a combined preparation for simultaneous, separate or sequential use in the treatment of a disease or disorder associated with inhibiting NLRP3 inflammasome activity (and where the disease or disorder may be any one of those described herein), for instance, in an embodiment, the combination may be a kit of parts. Such combinations may be referred to as "pharmaceutical combinations". The route of administration for a compound of formula (I) as a component of a combination may be the same or different to the one or more other therapeutic agent(s) with which it is combined. The other therapeutic agent is, for example, a chemical compound, peptide, antibody, antibody fragment or nucleic acid, which is therapeutically active or enhances the therapeutic activity when administered to a patient in combination with a compound of formula (I).

The weight ratio of (a) the compound according to the invention and (b) the other therapeutic agent(s) when given as a combination may be determined by the person skilled in the art. Said ratio and the exact dosage and frequency of administration depends on the particular compound according to the invention and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of formula (I) and another antibacterial agent may range from 1/10 to 10/1, more in particular from 1/5 to 5/1, even more in particular from 1/3 to 3/1.

The pharmaceutical composition or combination of the present invention can be in unit dosage of about 1-1000 mg of active ingredient(s) for a subject of about 50 - 70 kg, or about 1 - 500 mg, or about 1 - 250 mg, or about 1 - 150 mg, or about 1 - 100 mg, or about 1 - 50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, e.g., mice, rats, dogs, monkeys or isolated organs, tissues and preparations thereof. The compounds of the present invention can be applied *in vitro* in the form of solutions, *e.g.,* aqueous solutions, and *in vivo* either enterally, parenterally, advantageously intravenously, e.g., as a suspension or in aqueous solution. The dosage *in vitro* may range between about $10^{-3}$ molar and $10^{-9}$ molar concentrations. A therapeutically effective amount *in vivo* may range depending on the route of administration, between about 0.1 - 500 mg/kg, or between about 1 - 100 mg/kg.

As used herein, term "pharmaceutical composition" refers to a compound of the invention, or a pharmaceutically acceptable salt thereof, together with at least one pharmaceutically acceptable carrier, in a form suitable for oral or parenteral administration.

As used herein, the term "pharmaceutically acceptable carrier" refers to a substance useful in the preparation or use of a pharmaceutical composition and includes, for example, suitable diluents, solvents, dispersion media, surfactants, antioxidants, preservatives, isotonic agents, buffering agents, emulsifiers, absorption delaying agents, salts, drug stabilizers, binders, excipients, disintegration agents, lubricants, wetting agents, sweetening agents, flavoring agents, dyes, and combinations thereof, as would be known to those skilled in the art (see, for example, Remington The Science and Practice of Pharmacy, 22nd Ed. Pharmaceutical Press, 2013, pp. 1049-1070).

The term "subject" as used herein, refers to an animal, preferably a mammal, most preferably a human, for example who is or has been the object of treatment, observation or experiment.

The term "therapeutically effective amount" as used herein, means that amount of compound of formula (I) (including, where applicable, form, composition, combination comprising such compound of formula (I)) elicits the biological or medicinal response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviate, inhibit, prevent and/or ameliorate a condition, or a disorder or a disease (i) mediated by NLRP3, or (ii) associated with NLRP3 activity, or (iii) characterised by activity (normal or abnormal) of NLRP3; or (2) reduce or inhibit the activity of NLRP3; or (3) reduce or inhibit the expression of NLRP3. In another non-limiting embodiment, the term "a therapeutically effective amount" refers to the amount of the compound of the present invention that, when administered to a cell, or a tissue, or a non-cellular biological material, or a medium, is effective to at least partially reduce or inhibit the activity of NLRP3; or at least partially reduce or inhibit the expression of NLRP3.

As used herein, the term "inhibit", "inhibition" or "inhibiting" refers to the reduction or suppression of a given condition, symptom, or disorder, or disease, or a significant decrease in the baseline activity of a biological activity or

process. Specifically, inhibiting NLRP3 or inhibiting NLRP3 inflammasome pathway comprises reducing the ability of NLRP3 or NLRP3 inflammasome pathway to induce the production of IL-1 and/or IL-18. This can be achieved by mechanisms including, but not limited to, inactivating, destabilizing, and/or altering distribution of NLRP3.

As used herein, the term "NLRP3" is meant to include, without limitation, nucleic acids, polynucleotides, oligonucleotides, sense and anti-sense polynucleotide strands, complementary sequences, peptides, polypeptides, proteins, homologous and/or orthologous NLRP molecules, isoforms, precursors, mutants, variants, derivatives, splice variants, alleles, different species, and active fragments thereof.

As used herein, the term "treat", "treating" or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing" or "prevention" of any disease or disorder refers to the prophylactic treatment of the disease or disorder; or delaying the onset or progression of the disease or disorder.

As used herein, a subject is "in need of" a treatment if such subject would benefit biologically, medically or in quality of life from such treatment.

"Combination" refers to either a fixed combination in one dosage unit form, or a combined administration where a compound of the present invention and a combination partner (e.g. another drug as explained below, also referred to as "therapeutic agent" or "co-agent") may be administered independently at the same time or separately within time intervals. The single components may be packaged in a kit or separately. One or both of the components (e.g. powders or liquids) may be reconstituted or diluted to a desired dose prior to administration. The terms "co-administration" or "combined administration" or the like as utilized herein are meant to encompass administration of the selected combination partner to a single subject in need thereof (e.g. a patient), and are intended to include treatment regimens in which the agents are not necessarily administered by the same route of administration or at the same time.

The term "pharmaceutical combination" as used herein means a product that results from the mixing or combining of more than one therapeutic agent and includes both fixed and non-fixed combinations of the therapeutic agents. The term "pharmaceutical combination" as used herein refers to either a fixed combination in one dosage unit form, or non-fixed combination or a kit of parts for the combined administration where two or more therapeutic agents may be administered independently at the same time or separately within time intervals. The term "fixed combination" means that the therapeutic agents, *e.g.* a compound of the present invention and a combination partner, are both administered to a patient simultaneously in the form of a single entity or dosage. The term "non-fixed combination" means that the therapeutic agents, *e.g.* a compound of the present invention and a combination partner, are both administered to a patient as separate entities either simultaneously, concurrently or sequentially with no specific time limits, wherein such administration provides therapeutically effective levels of the two compounds in the body of the patient. The latter also applies to cocktail therapy, *e.g.* the administration of three or more therapeutic agents.

The term "combination therapy" refers to the administration of two or more therapeutic agents to treat a therapeutic condition or disorder described in the present disclosure. Such administration encompasses co-administration of these therapeutic agents in a substantially simultaneous manner, such as in a single capsule having a fixed ratio of active ingredients. Alternatively, such administration encompasses co-administration in multiple, or in separate containers (e.g. tablets, capsules, powders, and liquids) for each active ingredient. Powders and/or liquids may be reconstituted or diluted to a desired dose prior to administration. In addition, such administration also encompasses use of each type of therapeutic agent in a sequential manner, either at approximately the same time or at different times. In either case, the treatment regimen will provide beneficial effects of the drug combination in treating the conditions or disorders described herein.

Summary of pharmacology, uses, compositions and combinations

In an embodiment, there is provided a pharmaceutical composition comprising a therapeutically effective amount of a compound of formula (I), according to any one of the embodiments described herein, and a pharmaceutically acceptable carrier (including one or more pharmaceutically acceptable carriers).

In an embodiment, there is provided a compound of formula (I), according to any one of the embodiments described herein, for use as a medicament.

In an embodiment, there is provided a compound of formula (I), according to any one of the embodiments described herein (and/or pharmaceutical compositions comprising such compound of formula (I), according to any one of the embodiment described herein) for use: in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor.

In an embodiment, there is provided a use of compounds of formula (I), according to any one of the embodiments

described herein (and/or pharmaceutical compositions comprising such compound of formula (I), according to any one of the embodiment described herein): in the treatment of a disease or disorder associated with NLRP3 activity (including inflammasome activity); in the treatment of a disease or disorder in which the NLRP3 signalling contributes to the pathology, and/or symptoms, and/or progression, of said disease/disorder; in inhibiting NLRP3 inflammasome activity (including in a subject in need thereof); and/or as an NLRP3 inhibitor.

In an embodiment, there is provided use of compounds of formula (I), according to any one of the embodiments described herein (and/or pharmaceutical compositions comprising such compound of formula (I), according to any one of the embodiment described herein), in the manufacture of a medicament for: the treatment of a disease or disorder associated with NLRP3 activity (including inflammasome activity); the treatment of a disease or disorder in which the NLRP3 signalling contributes to the pathology, and/or symptoms, and/or progression, of said disease/disorder; and/or inhibiting NLRP3 inflammasome activity (including in a subject in need thereof).

In an embodiment, there is provided a method of treating a disease or disorder in which the NLRP3 signalling contributes to the pathology, and/or symptoms, and/or progression, of said disease/disorder, comprising administering a therapeutically effective amount of a compound of formula (I), according to any one of the embodiments described herein (and/or pharmaceutical compositions comprising such compound of formula (I), according to any one of the embodiment described herein), for instance to a subject (in need thereof). In a further embodiment, there is provided a method of inhibiting the NLRP3 inflammasome activity in a subject (in need thereof), the method comprising administering to the subject in need thereof a therapeutically effective amount of a compound of formula (I), according to any one of the embodiments described herein (and/or pharmaceutical compositions comprising such compound of formula (I), according to any one of the embodiment described herein).

In all relevant embodiment of the invention, where a disease or disorder is mentioned (e.g. hereinabove), for instance a disease or disorder in which the NLRP3 signalling contributes to the pathology, and/or symptoms, and/or progression, of said disease/disorder, or, a disease or disorder associated with NLRP3 activity (including NLRP3 inflammasome activity), including inhibiting NLRP3 inflammasome activity, then such disease may include inflammasome-related diseases or disorders, immune diseases, inflammatory diseases, auto-immune diseases, or auto-inflammatory diseases. In a further embodiment, such disease or disorder may include autoinflammatory fever syndromes (e.g. cryopyrin-associated periodic syndrome), liver related diseases/disorders (e.g. chronic liver disease, viral hepatitis, non-alcoholic steatohepatitis (NASH), alcoholic steatohepatitis, and alcoholic liver disease), inflammatory arthritis related disorders (e.g. gout, pseudogout (chondrocalcinosis), osteoarthritis, rheumatoid arthritis, arthropathy *e.g.* acute, chronic), kidney related diseases (e.g. hyperoxaluria, lupus nephritis, Type I/Type II diabetes and related complications (e.g. nephropathy, retinopathy), hypertensive nephropathy, hemodialysis related inflammation), neuroinflammation-related diseases (e.g. multiple sclerosis, brain infection, acute injury, neurodegenerative diseases, Alzheimer's disease), cardiovascular/metabolic diseases/ disorders (e.g. cardiovascular risk reduction (CvRR), hypertension, atherosclerosis, Type I and Type II diabetes and related complications, peripheral artery disease (PAD), acute heart failure), inflammatory skin diseases (e.g. hidradenitis suppurativa, acne), wound healing and scar formation, asthma, sarcoidosis, age-related macular degeneration, and cancer related diseases/disorders (e.g. colon cancer, lung cancer, myeloproliferative neoplasms, leukaemia, myelodysplastic syndromes (MOS), myelofibrosis). In a particular aspect, such disease or disorder is selected from autoinflammatory fever syndromes (e.g. CAPS), sickle cell disease, Type I/Type II diabetes and related complications (e.g. nephropathy, retinopathy), hyperoxaluria, gout, pseudogout (chondrocalcinosis), chronic liver disease, NASH, neuroinflammation-related disorders (e.g. multiple sclerosis, brain infection, acute injury, neurodegenerative diseases, Alzheimer's disease), atherosclerosis and cardiovascular risk (e.g. cardiovascular risk reduction (CvRR), hypertension), hidradenitis suppurativa, wound healing and scar formation, and cancer (e.g. colon cancer, lung cancer, myeloproliferative neoplasms, leukemias, myelodysplastic syndromes (MOS), myelofibrosis). In a particular embodiment, the disease or disorder associated with inhibition of NLRP3 inflammasome activity is selected from inflammasome related diseases and disorders, immune diseases, inflammatory diseases, auto-immune diseases, auto-inflammatory fever syndromes, cryopyrin-associated periodic syndrome, chronic liver disease, viral hepatitis, non-alcoholic steatohepatitis, alcoholic steatohepatitis, alcoholic liver disease, inflammatory arthritis related disorders, gout, chondrocalcinosis, osteoarthritis, rheumatoid arthritis, chronic arthropathy, acute arthropathy, kidney related disease, hyperoxaluria, lupus nephritis, Type I and Type II diabetes, nephropathy, retinopathy, hypertensive nephropathy, hemodialysis related inflammation, neuroinflammation-related diseases, multiple sclerosis, brain infection, acute injury, neurodegenerative diseases, Alzheimer's disease, cardiovascular diseases, metabolic diseases, cardiovascular risk reduction, hypertension, atherosclerosis, peripheral artery disease, acute heart failure, inflammatory skin diseases, acne, wound healing and scar formation, asthma, sarcoidosis, age-related macular degeneration, colon cancer, lung cancer, myeloproliferative neoplasms, leukemias, myelodysplastic syndromes and myelofibrosis.

In an embodiment, there is provided a combination comprising a therapeutically effective amount of a compound of formula (I), according to any one of the embodiments described herein, and another therapeutic agent (including one or more therapeutic agents). In a further embodiment, there is provided such a combination wherein the other therapeutic agent is selected from (and where there is more than one therapeutic agent, each is independently selected from):

farnesoid X receptor (FXR) agonists; anti-steatotics; anti-fibrotics; JAK inhibitors; checkpoint inhibitors including anti-PD1 inhibitors, anti-LAG-3 inhibitors, anti-TIM-3 inhibitors, or anti-POL 1 inhibitors; chemotherapy, radiation therapy and surgical procedures; urate-lowering therapies; anabolics and cartilage regenerative therapy; blockade of IL-17; complement inhibitors; Bruton's tyrosine Kinase inhibitors (BTK inhibitors); Toll Like receptor inhibitors (TLR7/8 inhibitors); CAR-T therapy; anti-hypertensive agents; cholesterol lowering agents; leukotriene A4 hydrolase (LTAH4) inhibitors; SGLT2 inhibitors; 132-agonists; anti-inflammatory agents; nonsteroidal anti-inflammatory drugs ("NSAIDs"); acetylsalicylic acid drugs (ASA) including aspirin; paracetamol; regenerative therapy treatments; cystic fibrosis treatments; or atherosclerotic treatment. In a further embodiment, there is also provided such (a) combination(s) for use as described herein in respect of compounds of formula (I), e.g. for use in the treatment of a disease or disorder in which the NLRP3 signaling contributes to the pathology, and/or symptoms, and/or progression, of said disease/disorder, or, a disease or disorder associated with NLRP3 activity (including NLRP3 inflammasome activity), including inhibiting NLRP3 inflammasome activity, and in this respect the specific disease/disorder mentioned herein apply equally here. There may also be provided methods as described herein in respect of compounds of formula (I), but wherein the method comprises administering a therapeutically effective amount of such combination (and, in an embodiment, such method may be to treat a disease or disorder mentioned herein in the context of inhibiting NLRP3 inflammasome activity). The combinations mentioned herein may be in a single preparation or they may be formulated in separate preparations so that they can be administered simultaneously, separately or sequentially. Thus, in an embodiment, the present invention also relates to a combination product containing (a) a compound according to the invention, according to any one of the embodiments described herein, and (b) one or more other therapeutic agents (where such therapeutic agents are as described herein), as a combined preparation for simultaneous, separate or sequential use in the treatment of a disease or disorder associated with inhibiting NLRP3 inflammasome activity (and where the disease or disorder may be any one of those described herein).

Compounds of formula (I) (including forms and compositions/combinations comprising compounds of formula (I)) may have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise.

For instance, compounds of formula (I) may have the advantage that they have a good or an improved thermodynamic solubility (e.g. compared to compounds known in the prior art; and for instance as determined by a known method and/or a method described herein). Compounds of formula (I) may have the advantage that they will block pyroptosis, as well as the release of pro-inflammatory cytokines (e.g. IL-1$\beta$) from the cell. Compounds of formula (I) may also have the advantage that they avoid side-effects, for instance as compared to compounds of the prior art, which may be due to selectivity of NLRP3 inhibition. Compounds of formula (I) may also have the advantage that they have good or improved *in vivo* pharmacokinetics and oral bioavailability. They may also have the advantage that they have good or improved *in vivo* efficacy. Specifically, compounds of formula (I) may also have advantages over prior art compounds when compared in the tests outlined hereinafter (e.g. in Examples C and D).

## GENERAL PREPARATION AND ANALYTICAL PROCESSES

The compounds according to the invention can generally be prepared by a succession of steps, each of which may be known to the skilled person or described herein.

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC, chiral chromatography. Individual diastereoisomers or individual enantiomers can also be obtained by Supercritical Fluid Chromatography (SFC).

The starting materials and the intermediates are compounds that are either commercially available or may be prepared according to conventional reaction procedures generally known in the art.

<u>Analytical Part</u>

LC-MS (LIQUID CHROMATOGRAPHY/MASS SPECTROMETRY)

*General procedure*

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software. Compounds are described by their experimental retention times (Rt) and ions. If not specified differently in the table of data, the reported molecular ion corresponds to the $[M+H]^+$ (protonated molecule) and/or $[M-H]^-$ (deprotonated molecule). In case the compound was not directly ionizable the type of adduct is specified (i.e. $[M+NH_4]^+$, $[M+HCOO]^-$, etc...). For molecules with multiple isotopic patterns (Br, Cl..), the reported value is the one obtained for the lowest isotope mass. All results were obtained with experimental uncertainties that are commonly associated with the method used.

Hereinafter, "SQD" means Single Quadrupole Detector, "MSD" Mass Selective Detector, "RT" room temperature, "BEH" bridged ethylsiloxane/silica hybrid, "DAD" Diode Array Detector, "HSS" High Strength silica.

Table: LCMS Method codes (Flow expressed in mL/min; column temperature (T) in °C; Run time in minutes).

| Method code | Instrument | column | mobile phase | gradient | Flow ------ Col T | Run time |
|---|---|---|---|---|---|---|
| Method 1 | Agilent 1100 HPLC DAD LC/MS G1956A | YMC-pack ODS-AQ C18 (50 x 4.6 mm, 3 μm) | A: 0.1% HCOOH in $H_2O$<br>B: $CH_3CN$ | From 95% A to 5% A in 4.8 min, held for 1.0 min, to 95% A in 0.2 min. | 2.6 ---- 35 | 6.2 |
| Method 2 | Waters: Acquity® UPLC®-DAD and SQD2 | Waters :BEH (1.8μm, 2.1*100mm) | A: 10mM $NH_4HCO_3$ in 95% $H_2O$ + 5% $CH_3CN$<br>B: MeOH | From 100% A to 5% A in 2.10min, to 0% A in 1.4 min | 0.6 ---- 55 | 3.5 |
| Method 3 | Waters: Acquity® UPLC®-DAD and SQD | Waters :BEH (1.8μm, 2.1*100mm) | A: 10mM $CH_3COONH_4$ in 95% $H_2O$ + 5% $CH_3CN$<br>B: $CH_3CN$ | From 100% A to 5% A in 2.10min, to 0% A in0.9min, to 5% A in 0.5min | 0.6 ---- 55 | 3.5 |
| Method 4 | Waters: Acquity® UPLC®-DAD and SQD | Waters :BEH (1.8μm, 2.1*100mm) | A: 10mM $NH_4HCO_3$ in 95% $H_2O$ + 5% $CH_3CN$<br>B: $CH_3CN$ | From 100% A to 5% A in 2.10min, to 0% A in 0.9 min, to 5% A in 0.4 min | 0.6 ---- 55 | 3.5 |
| Method 5 | Waters: Acquity® IClass UPLC®-DAD and Xevo G2-S QTOF | Waters: BEH C18 (1.7μm, 2.1x50mm) | A: 95% $CH_3COONH_4$ 6.5mM + 5% $CH_3CN$, B: $CH_3CN$ | From 95% A to 5% A in 4.6min, held for 0.4min | 1 ---- 50 | 5 |
| Method 6 | Waters: Acquity® UPLC® - DAD and SQD | Waters :BEH (1.8μm, 2.1*100mm) | A: 0.1% $NH_4HCO_3$ in 95% $H_2O$ + 5% $CH_3CN$<br>B: $CH_3CN$ | From 100% A to 5% A in 2.10min, to 0% A in 0.9min, to 5% A in 0.5min | 0.6 ------- 55 | 3.5 |
| Method 7 (HRMS) | Agilent 1260 Infinity DAD TOF-LC/MS G6224A | YMC-pack ODS-AQ C18 (50 x 4.6 mm, 3 μm) | A: 0.1% HCOOH in $H_2O$<br>B: $CH_3CN$ | From 95% A to 5% A in 4.8 min, held for 1.0 min, to 95% A in 0.2 min. | 2.6 ---- 35 | 6.8 |

NMR

For a number of compounds, $^1H$ NMR spectra were recorded on a Bruker Avance III spectrometer operating at

300 or 400 MHz, on a Bruker Avance III-HD operating at 400 MHz, on a Bruker Avance NEO spectrometer operating at 400 MHz, on a Bruker Avance Neo spectrometer operating at 500 MHz, or on a Bruker Avance 600 spectrometer operating at 600 MHz, using CHLOROFORM-*d* (deuterated chloroform, $CDCl_3$), DMSO-$d_6$ (deuterated DMSO, dimethyl-d6 sulfoxide), METHANOL-$d_4$ (deuterated methanol), BENZENE-$d_6$ (deuterated benzene, $C_6D_6$) or ACETONE-$d_6$ (deuterated acetone, $(CD_3)_2CO$) as solvents. Chemical shifts (δ) are reported in parts per million (ppm) relative to tetramethylsilane (TMS), which was used as internal standard.

Melting Points

Values are either peak values or melt ranges, and are obtained with experimental uncertainties that are commonly associated with this analytical method.

Method A: For a number of compounds, melting points were determined in open capillary tubes on a Mettler Toledo MP50. Melting points were measured with a temperature gradient of 10 °C/minute. Maximum temperature was 300 °C. The melting point data was read from a digital display and checked from a video recording system

Method B: For a number of compounds, melting points were determined with a DSC823e (Mettler Toledo) apparatus. Melting points were measured with a temperature gradient of 10 °C/minute. Standard maximum temperature was 300 °C.

**EXPERIMENTAL PART**

Hereinafter, the term "m.p." means melting point, "aq." means aqueous, "r.m." means reaction mixture, "rt" means room temperature, 'DIPEA' means *N*,*N*-diisopropylethylamine, "DIPE" means diisopropylether, 'THF' means tetrahydrofuran, 'DMF' means dimethylformamide, 'DCM' means dichloromethane, "EtOH" means ethanol 'EtOAc' means ethyl acetate, "AcOH" means acetic acid, "iPrOH" means isopropanol, "$iPrNH_2$" means isopropylamine, "MeCN" or "ACN" means acetonitrile, "MeOH" means methanol, "$Pd(OAc)_2$" means palladium(II)diacetate, "NBS" means N-bromosuccinimide, "AIBN" means azobisisobutyronitrile, "rac" means racemic, 'sat.' means saturated, 'FCC' means flash column chromatography, 'SFC' means supercritical fluid chromatography, 'SFC-MS' means supercritical fluid chromatography/mass spectrometry, "LC-MS" means liquid chromatography/mass spectrometry, "GCMS" means gas chromatography/mass spectrometry, "HPLC" means high-performance liquid chromatography, "RP" means reversed phase, "UPLC" means ultra-performance liquid chromatography, "$R_t$" (or "RT") means retention time (in minutes), "$[M+H]^+$" means the protonated mass of the free base of the compound, "DAST" means diethylaminosulfur trifluoride, "DMTMM" means 4-(4,6-dimethoxy-1,3,5-triazin-2-yl)-4-methylmorpholinium chloride, "HATU" means *O*-(7-azabenzotriazol-1-yl)-*N*,*N*,*N'*,*N'*-tetramethyluronium hexafluorophosphate (1-[bis(dimethylamino)methylene]-1H-1,2,3-triazolo [4,5-b]pyridinium 3-oxide hexafluorophosphate), "Xantphos" means (9,9-dimethyl-9H-xanthene-4,5-diyl)bis-[diphenylphosphine], "TBAT" means tetrabutyl ammonium triphenyldifluorosilicate, "TFA" means trifluoroacetic acid, "$Et_2O$" means diethylether, "DMSO" means dimethylsulfoxide, "$SiO_2$" means silica, "XPhos Pd G3" means (2-dicyclohexyl-phosphino-2',4',6'-triisopropyl-1,1'-biphenyl)[2-(2'-amino-1,1'-biphenyl)]palladium(II) ethanesulfonate, "$CDCl_3$" means deuterated chloroform, "MW" means microwave or molecular weight, "min" means minutes, "h" means hours, "rt" means room temperature, "quant" means quantitative, "n.t." means not tested, "Cpd" means compound, "$POCl_3$" means phosphorus(V) oxychloride.

For key intermediates, as well as some final compounds, the absolute configuration of chiral centers (indicated as *R* and/or *S*) were established via comparison with samples of known configuration, or the use of analytical techniques suitable for the determination of absolute configuration, such as VCD (vibrational cicular dichroism) or X-ray crystallography. When the absolute configuration at a chiral center is unknown, it is arbitrarily designated R*.

**Examples - Example A**

Synthesis of 5-(trifluoromethyl)isobenzofuran-1(3H)-one **1A**

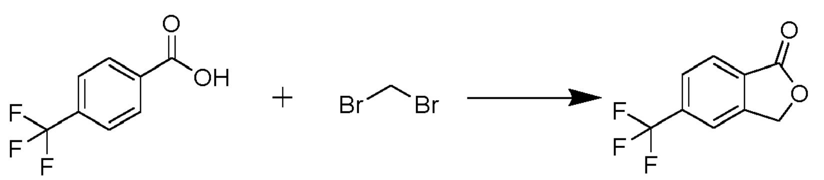

(3 identical reactions run in 3 separate 150-mL thick-walled EasyMax pressure tubes.) 4-(Trifluoromethyl) benzoic acid (7 g, 36.8 mmol), $Pd(OAc)_2$ (868 mg, 3.87 mmol) and $K_2HPO_4$ (19.24 g, 110.5 mmol) were placed in a 150-mL EasyMax thick-walled pressure tube and dibromomethane (110 mL) was added. The vial was sealed and heated at 140 °C for 50 h.

The black suspension was allowed to cool to R.T. The mixtures from all 3 tubes were filtered together and the filter cake washed with DCM (3 x 40 mL). The filtrate was concentrated and purified by FCC (Biotage, Sfär 100 g, 100 mL/min, Heptane/EtOAc 93:7 to 7:3 over 25 CV) to give the title product as colorless crystalline solid (5.91 g, 26%).

**LCMS** (RT: 1.67, Area %: 96.85, MW: 202, BPM1: (Method: 6)
**$^1$H NMR** (400 MHz, $CDCl_3$) δ 5.44 (s, 2 H), 7.83 (br d, J=7.9 Hz, 1 H), 7.86 (s, 1 H), 8.04 (d, J=7.9 Hz, 1 H).
**$^{19}$F NMR** (377 MHz, $CDCl_3$) δ -62.88 (s, 3 F).

Synthesis of 3-hydroxy-5-(trifluoromethyl)isobenzofuran-1(3H)-one **1B**

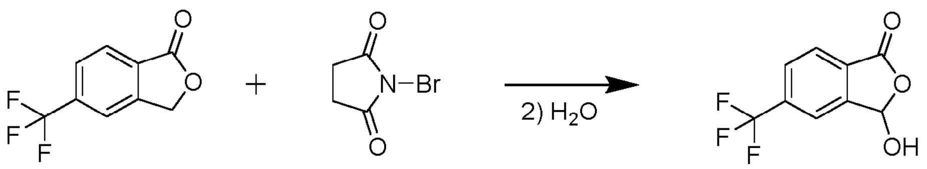


5-(trifluoromethyl)isobenzofuran-1(3H)-one **1A** (10.4 g, 51.5 mmol), AIBN (422.4 mg, 2.57 mmol, 5 mol%) and NBS (9.52 g, 53.5 mmol) were placed in a 250-mL RB flask. 1,2-dichloroethane (143 mL) was added and the mixture stirred vigorously and heated at reflux for 5h. The mixture was allowed to cool to R.T., the solvent was concentrated in vacuo to about 50 mL. It was then diluted with DCM/pentane 1:1 (30 mL) and the solid that had crystallised was filtered off washing the flask and filtered solid with 3 x 10 mL of a 1:1 mixture of DCM/pentane. The filtrate was concentrated in vacuo and the resulting yellow oil was retaken in DI water (170 mL) and the mixture heated at 100 °C for 1 h. It was allowed to cool to rt slowly and stirring was continued for 14h. The white suspension was then cooled in an ice-bath for 30 min. and the solids filtered off, dried on the filter for 30 min then under vacuum at 50 °C for 1.5 hours to obtain an off-white solid (11.33 g, 91 %).

**LCMS** (RT: 1.08, Area %: 95.66, MW: 218, BPM1: ND, BPM2: 217.0 (Method: 6)
**$^1$H NMR** (300 MHz, DMSO-$d_6$) δ 6.77 (br s, 1 H), 8.03 - 8.10 (m, 3 H), 8.13 - 8.15 (m, 1 H), 8.40 (br s, 1 H).
**$^{19}$F NMR** (377 MHz, DMSO-$d_6$) δ -61.23 (s, 3 F).

Synthesis of 5-chloroisobenzofuran-1(3H)-one **8A1** and 6-chloroisobenzofuran-1(3H)-one **8A2**

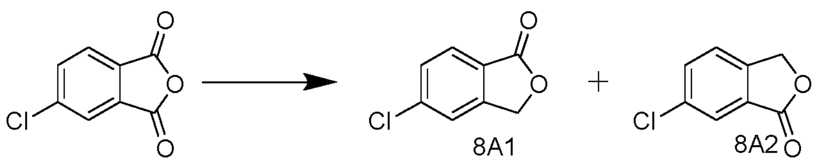
8A1
8A2

Zinc dust (<10 μm) [7440-66-6] (6.70 g, 100.42 mmol) was added to a stirred solution of 4-Chlorophthalic anhydride [118-45-6 ] (6.3 g, 33.47 mmol) in acetic acid glacial (39 mL) at rt. The mixture was stirred at 130ºC for 16 h. The reaction mixture was filtered hot (100ºC) through a pad of diatomaceous earth, washed with hot acetic acid (100ºC) and then the solvent from the filtrate was evaporated in vacuo. The crude product was purified by FCC (silica 80 g; dry load in silica; EtOAc in heptane 0/100 to 18/82). The desired fractions were collected and concentrated in vacuo to yield to a mixture of 5-chloroisobenzofuran-1(3H)-one **8A1** and 6-chloroisobenzofuran-1(3H)-one **8A2** as a yellow solid (8.2 g, 73%).

**LCMS** (RT: 1.068, Area %: 50, MH+: 169.1)
**$^1$H NMR** (300 MHz, $CDCl_3$) δ ppm 5.30 (s, 2H), 7.50 (s, 1H), 7.52 (d, J = 8.0 Hz, 1H), 7.86 (d, J = 8.0 Hz, 1H).

The following compounds were prepared in accordance with the same or similar procedures.

| Number | Starting material | Final compound | yield |
|---|---|---|---|
| 4A | 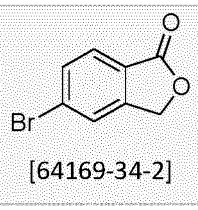 [64169-34-2] | 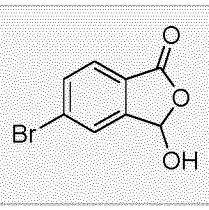 | 94% |
| 8B1+8B2 | 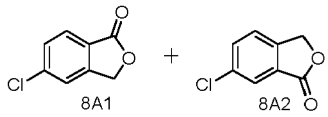 8A1 + 8A2 | 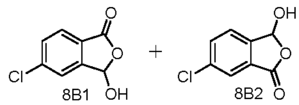 8B1 + 8B2 | 81% |

| Number | LCMS |
|---|---|
| 4A | RT: 1.04, Area %: 98.78, MW: 228, BPM1: ND, BPM2: 226.9 / 229.0, method: 6 |
| 8B1 + 8B2 | RT: 0.944, Area %: 100, MH+: 185.0, |

| Number | NMR |
|---|---|
| 4A | 1H NMR (400 MHz, DMSO-$d_6$) δ ppm 6.66 (br s, 1 H), 7.70 - 7.82 (m, 1 H), 7.87 (br d, J=7.7 Hz, 1 H), 7.93 (s, 1 H), 8.28 (br d, J=3.3 Hz, 1 H). |

| Number | Melting Point |
|---|---|
| 4A | 200.88 °C (DSC: From 30 to 400 °C at 10°C/min 50ml N2) |

Synthesis of 6-(trifluoromethyl)phthalazin-1(2H)-one **1C**

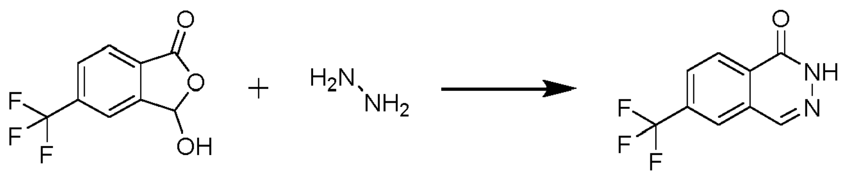

3-hydroxy-5-(trifluoromethyl)isobenzofuran-1(3H)-one **1B** (12.3 g, 50.75 mmol) was placed in a 500-mL RB flask and suspended in EtOH (175 mL). Hydrazine hydrate (12.3 mL, 253.7 mmol) was added and the mixture heated at reflux for 3.5 hours. The mixture was allowed to cool to rt and diluted with DI water (250 mL) and the milky suspension kept at 0 °C for 14 h. The suspension was then filtered off, dried on the filter under vacuum for 30 min then at 50 °C under vacuum for 3h to give a colorless solid (10.24 g, 90%).

**LCMS** (RT: 1.47, Area %: 94.18, MW: 214.00, BPM1: 215.1, BPM2: 213.0 (Method: 6)
**$^1$H NMR** (300 MHz, DMSO-$d_6$) δ ppm 8.15 (dd, J=8.4, 1.5 Hz, 1 H), 8.39 - 8.43 (m, 1 H), 8.44 - 8.46 (m, 1 H), 8.51 (s, 1 H), 11.38 (br s, 1 H).
**$^{19}$F NMR** (377 MHz, DMSO-$d_6$) δ -61.41 (s, 3 F).

The following compounds were prepared in accordance with the same or similar procedures.

| Number | Starting material | Final compound | yield |
|---|---|---|---|
| **4B** | 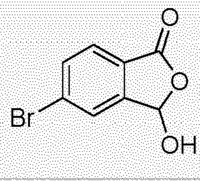 | 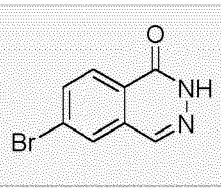 | 95% |
| **8C1+8C2** | 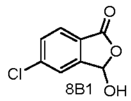 8B1 + 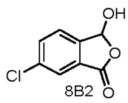 8B2 | 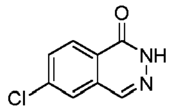 8C1 + 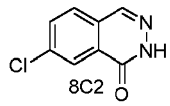 8C2 | 86% |

| Number | LCMS |
|---|---|
| **4B** | RT: 1.37, Area %: 98.12, MW: 224.00, BPM1: 224.9 / 226.9, BPM2: 223.0 / 224.9, Method: 6 |
| **8C1+ 8C2** | RT: 0.915, Area %: 100, MH+: 181.0 |

| Number | NMR |
|---|---|
| **4B** | 1H NMR (400 MHz, DMSO-$d_6$, 27°C) δ ppm 8.00 (dd, J=8.5, 1.9 Hz, 1 H), 8.14 (d, J=8.6 Hz, 1 H), 8.22 (d, J=1.8 Hz, 1 H), 8.34 (s, 1 H), 12.77 (br s, 1 H) |

| Number | Melting Point |
|---|---|
| **4B** | 283.87 °C (DSC: From 30 to 400 °C at 10°C/min 50ml N2) |

Synthesis of 4-bromo-6-(trifluoromethyl)phthalazin-1(2H)-one **1D**

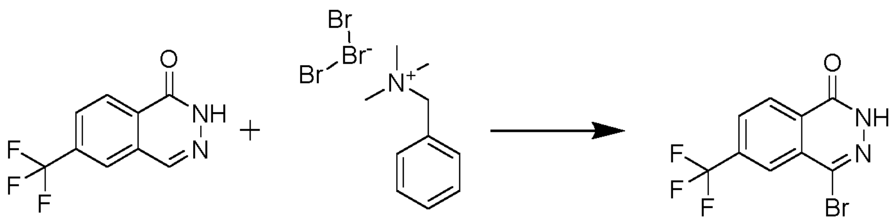

6-(trifluoromethyl)phthalazin-1(2H)-one **1C** (10.24 g, 43.992 mmol) and potassium carbonate (12.22 g, 88.419 mmol) were placed in a 200-mL thick-walled screw-cap EasyMax tube and dry DMF (110 mL) was added. The tube was sealed and stirred at rt for 10 minutes. Then, benzyltrimethylammonium tribromide (34.44 g, 88.318 mmol) was added to the suspension and the resulting mixture stirred 6 h at 40 °C. The mixture was quenched slowly by addition of a saturated aqueous solution of $Na_2S_2O_3$ (overall 500 mL). The mixture was then extracted with DCM (3 x 200 mL). The combined organic extracts were dried over $MgSO_4$, filtered, concentrated under reduced pressure at 60°C. The crude was purified on a 350 g Sfär Silica HC in a Biotage system using a gradient from 0 till 55 % EtOAc in heptane (35 CV). The different product fractions were combined and concentrated under reduced pressure at 40 °C to afford the compound **1D** (12.2 g, yield 95%) as colorless solid.

**LCMS** (RT: 1.79, Area %: 100.00, MW: 291.90, BPM1: 293, BPM2: 291 (Method: 3))
**$^1$H NMR** (400 MHz, DMSO-$d_6$, 27°C) δ ppm 8.08 - 8.12 (m, 1 H) 8.25 (dd, J=8.31, 1.31 Hz, 1 H) 8.44 (d, J=8.26 Hz, 1 H) 13.20 (br s, 1 H). **M.P.** 172.21 °C (DSC: From 30 to 400 °C at 10°C/min 50ml $N_2$)

The following compounds were prepared in accordance with the same or similar procedures.

| Number | Starting material | Final compound | yield |
|---|---|---|---|
| **4C** | 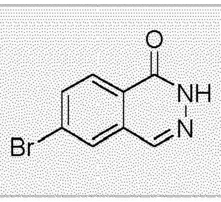 | 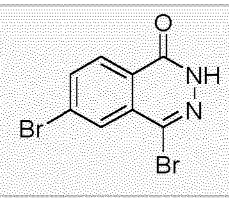 | 77% |
| **8D1+8D2** | 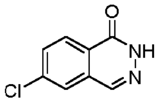 + 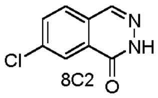 8C1 8C2 | 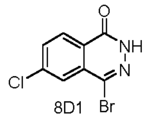 + 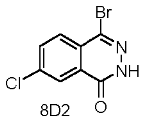 8D1 8D2 | 66% |

| Number | LCMS |
|---|---|
| **4C** | RT: 0.767, Area %: 95, MH+: 302.8 |
| **8D1 + 8D2** | RT: 1.171, Area %: 100, MH+: 260.8 |

| Number | NMR |
|---|---|
| **4C** | 1H NMR (300 MHz, DMSO-$d_6$, 27°C) δ ppm 13.03 (s, 1H), 8.13 (dt, J = 8.0, 4.8 Hz, 2H), 8.02 (d, J = 1.4 Hz, 1H). |

Synthesis of ethyl 2-(4-bromo-1-oxo-6-(trifluoromethyl)phthalazin-2(1H)-yl)acetate **1E**

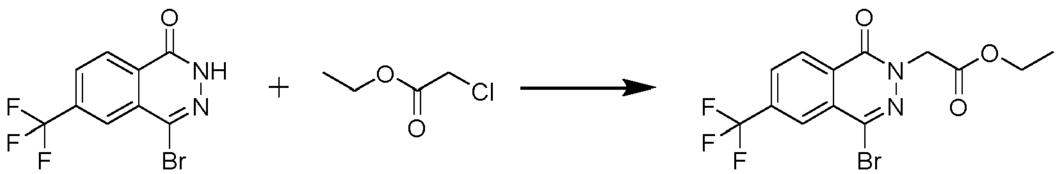

In a 20-mL MW-vial equipped with a magnetic stir bar, was placed: 4-bromo-6-(trifluoromethyl)-2H-phthalazin-1-one (1.5 g, 5.12 mmol), 18-crown-6 (68 mg, 0.26 mmol), KI (102 mg, 0.61 mmol) and $K_2CO_3$ (0.85 g, 6.14 mmol). The vial was sealed and ethyl 2-chloroacetate (0.73 mL, 6.81 mmol) was added as a solution in acetonitrile (6.7 mL). The suspension was heated at 90 °C for 3 h. The mixture was poured onto brine (70 mL) and extracted with EtOAc (2 x 50 mL). The combined organics were concentrated in vacuo and the obtained residue purified by FCC (Biotage, Sfar 100 g, 120 mL/min, Hept/EtOAc 93:7 to 7:3 over 25 CV) to give a colorless fluffy solid (1.46 g, 75%).

**LCMS** (RT: 2.13, Area %: 100.00, MW: 378.00, BPM1: 379.0 / 381.0, BPM2: ND (Method: 6))
**$^1$H NMR** (400 MHz, $CDCl_3$) δ ppm 1.30 (t, J=7.2 Hz, 3 H), 4.27 (q, J=7.1 Hz, 2 H), 4.95 (s, 2 H), 8.05 (dd, J=8.3, 1.2 Hz, 1 H), 8.24 (d, J=0.7 Hz, 1 H), 8.55 (d, J=8.4 Hz, 1 H).
**$^{19}$F NMR** (377 MHz, $CDCl_3$) δ ppm -63.09 (s, 3 F). **M.P.** 121.01 °C (DSC: From 30 to 400 °C at 10°C/min 50ml N2)

The following compounds were prepared in accordance with the same or similar procedures.

| Number | Starting material | Final compound | yield |
|---|---|---|---|
| **4D** | 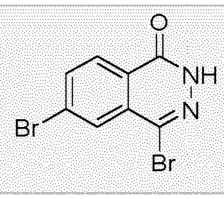 | 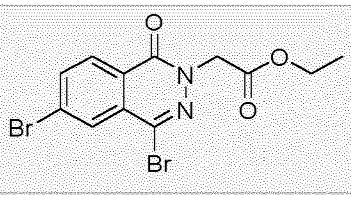 | 61% |
| **8E1** | 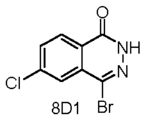 + 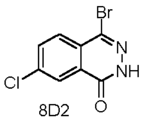 8D1 8D2 | 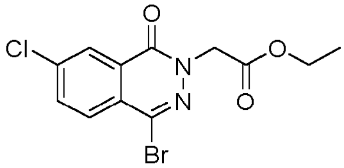 | 31% |
| **8E2** | | 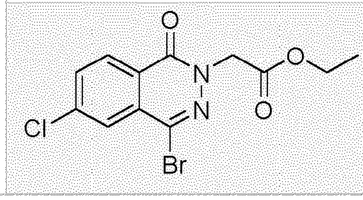 | 41% |

| Number | LCMS |
|---|---|
| **4D** | RT: 3.794, Area %: 99, MH+: 390.8, Method: 1 |
| **8E1** | RT: 3.636, Area %: 99, MH+: 346.9, Method: 1 |
| **8E2** | RT: 6.668, Area %: 98, MH+: 346.9, Method: 1 |

| Number | NMR |
|---|---|
| **4D** | 1H NMR (400 MHz, DMSO-$d_6$) δ ppm 1.21 (t, J = 7.1 Hz, 3H), 4.17 (q, J = 7.1 Hz, 2H), 4.94 (s, 2H), 8.09 (d, J = 1.6 Hz, 1H), 8.19 (dt, J = 8.5, 5.1 Hz, 2H). |
| **8E1** | 1H NMR (300 MHz, $CDCl_3$) δ ppm 1.30 (t, J = 7.1 Hz, 3H), 4.26 (q, J = 7.1 Hz, 2H), 4.92 (s, 2H), 7.83 (dd, J = 8.6, 2.1 Hz, 1H), 7.92 (d, J = 8.6 Hz, 1H), 8.40 (d, J = 2.0 Hz, 1H). |
| **8E2** | 1H NMR (300 MHz, $CDCl_3$) δ ppm 1.30 (t, J = 7.1 Hz, 3H), 4.26 (q, J = 7.1 Hz, 2H), 4.92 (s, 2H), 7.77 (dd, J = 8.5, 1.8 Hz, 1H), 7.95 (d, J = 1.8 Hz, 1H), 8.37 (d, J = 8.5 Hz, 1H). |

| Number | HRMS |
|---|---|
| **4B** | RT: 5.266 (MH+: 390.8920, Method: 7) |

| Number | Melting Point |
|---|---|
| **4B** | 146.3°C (Mettler Toledo (MP50), 10ºC/min ) |

Synthesis of ethyl 2-(4-(dimethylamino)-1-oxo-6-(trifluoromethyl)phthalazin-2(1H)-yl)acetate **1F**

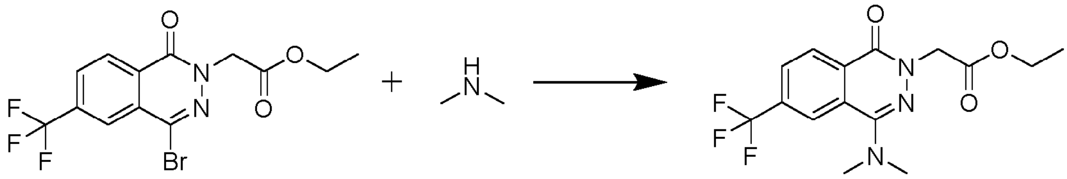

Ethyl 2-(4-bromo-1-oxo-6-(trifluoromethyl)phthalazin-2(1H)-yl)acetate **1E** (300 mg, 0.79 mmol) was placed in an 8-mL MW vial. It was dissolved in anhydrous 1,4-dioxane (2 mL) and N,N-diisopropylethylamine (0.84 mL, 4.75 mmol) and a solution of dimethylamine (2.4 mL, 2 M in THF, 4.8 mmol) were added sequentially. The MW vial was then sealed, stirred and heated at 110 °C for 24 hours. It was then allowed to cool to R.T. and stirred for additional 68 h.

Partial conversion, dimethylamine (1.5 mL, 2 M in THF, 3 mmol) was added and the MW vial was stirred and heated at 110 °C for additional 8 h. A further portion of dimethylamine (1 mL, 2 M in THF, 2 mmol) was added and the MW vial was stirred and heated at 110 °C for additional 16 h.

The crude mixture was concentrated in vacuo and the obtained residue redissolved in the minimum amount of DCM and purified by FCC on Biotage (Sfar 50 g, 105 mL/min, Hept/EtOAc 93:7 to 3:2 over 35 CV) to give a pale yellow oil that solidified on standing to give ethyl 2-(4-(dimethylamino)-1-oxo-6-(trifluoromethyl)phthalazin-2(1H)-yl)acetate **1F** as a pale yellow solid (198 mg, 73%).

**LCMS** (RT: 2.12, Area %: 100.00, MW: 343.00, BPM1: 344.2, BPM2: ND (Method: 6))
**$^1$H NMR** (400 MHz, $CDCl_3$) δ ppm 1.30 (t, J=7.2 Hz, 3 H), 2.88 (s, 6 H), 4.26 (q, J=7.1 Hz, 2 H), 4.87 (s, 2 H), 7.94 (dd, J=8.4, 1.4 Hz, 1 H), 8.23 - 8.18 (m, 1 H), 8.56 (d, J=8.4 Hz, 1 H).
**$^{19}$F NMR** (377 MHz, $CDCl_3$) δ ppm -62.96 (s, 3 F).

The following compounds were prepared in accordance with the same or similar procedures.

| Number | Reagent | Starting material | Final compound | yield |
|---|---|---|---|---|
| **3A** | 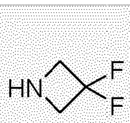 .HCl [288315-03-7] | 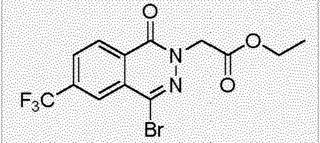 | 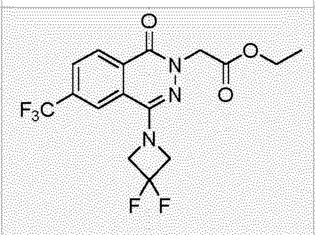 | 96% |
| **4E** | 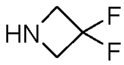 .HCl [288315-03-7] | 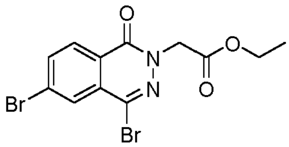 | 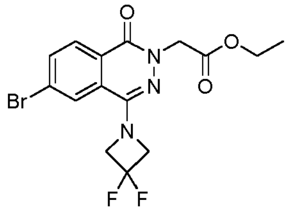 | 32% |
| **5A** | 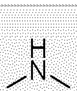 [124-40-3] | 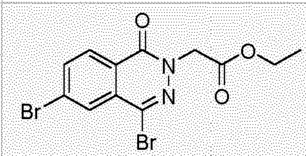 | 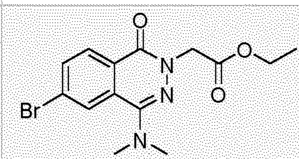 | 30% |
| **6A** | 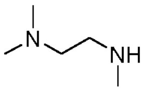 [142-25-6] | 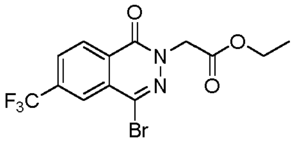 | 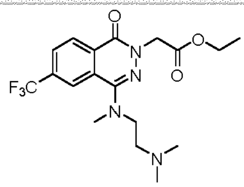 | 65% |
| **7A** | 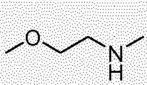 [38256-93-8] | 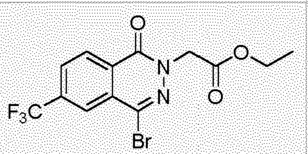 | 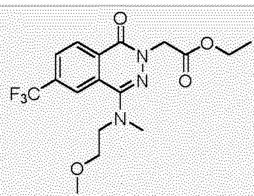 | 61% |
| **8F** | 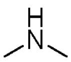 [124-40-3] | 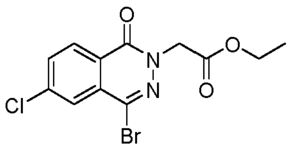 | 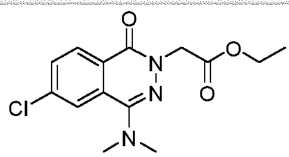 | 37% |
| **9F** | 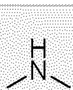 [124-40-3] | 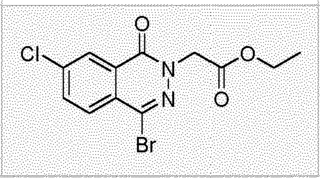 | 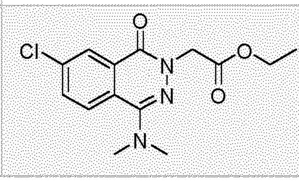 | 39% |

| Number | LCMS |
|---|---|
| **3A** | RT: 0.999, Area %: 99, MH+: 392.0 |
| **4E** | RT: 0.963, Area %: 99, MH+: 402.0 |
| **5A** | RT: 0.965, Area %: 97, MH+: 354.0 |

| Number | LCMS |
|---|---|
| **6A** | RT: 2.00, Area %: 92.50, MW: 400.00, BPM1: 401, Method: 6 |
| **7A** | RT: 2.04, Area %: 1.37, MW: 387.00, BPM1: 388, BPM2: 388, Method: 6 |
| **8F** | RT: 3.545, Area %: 99, MH+: 310.0, Method: 1 |
| **9F** | RT: 3.501, Area %: 99, MH+: 310.1, Method: 1 |

| Number | NMR |
|---|---|
| **3A** | 1H NMR (300 MHz, DMSO-$d_6$) δ ppm 1.21 (dd, J = 9.6, 4.6 Hz, 3H), 4.17 (q, J = 7.1 Hz, 2H), 4.67 (t, J = 12.8 Hz, 4H), 4.85 (s, 2H), 8.10 (s, 1H), 8.24 (d, J = 8.4 Hz, 1H), 8.49 (d, J = 8.3 Hz, 1H). |
| **4E** | 1H NMR (300 MHz, DMSO-$d_6$) δ ppm 1.21 (t, J = 7.1 Hz, 3H), 4.16 (q, J = 7.1 Hz, 2H), 4.63 (t, J = 12.9 Hz, 4H), 4.80 (s, 2H), 8.02 (d, J = 1.5 Hz, 1H), 8.08 (dd, J = 8.5, 1.7 Hz, 1H), 8.19 (d, J = 8.5 Hz, 1H). |
| **5A** | 1H NMR (300 MHz, DMSO-$d_6$) δ ppm 1.21 (t, J = 7.1 Hz, 3H), 2.80 (s, 6H), 4.16 (q, J = 7.1 Hz, 2H), 4.79 (s, 2H), 8.05 (dd, J = 8.5, 1.9 Hz, 1H), 8.10 (d, J = 1.6 Hz, 1H), 8.17 (d, J = 8.5 Hz, 1H). |
| **8F** | 1H NMR (400 MHz, $CDCl_3$) δ ppm 1.29 (t, J = 7.1 Hz, 3H), 2.85 (s, 6H), 4.25 (q, J = 7.1 Hz, 2H), 4.84 (s, 2H), 7.67 (dd, J = 8.5, 1.9 Hz, 1H), 7.90 (d, J = 1.9 Hz, 1H), 8.36 (d, J = 8.5 Hz, 1H). |
| **9F** | 1H NMR (400 MHz, $CDCl_3$) δ ppm 1.29 (t, J = 7.1 Hz, 3H), 2.85 (s, 6H), 4.25 (q, J = 7.1 Hz, 2H), 4.84 (s, 2H), 7.72 (dd, J = 8.6, 2.2 Hz, 1H), 7.87 (d, J = 8.6 Hz, 1H), 8.39 (d, J = 2.2 Hz, 1H). |

| Number | HRMS |
|---|---|
| **8F** | Rt = 4.780 min (MH+: 310.1890, Method: 7) |
| **9F** | Rt = 4.593 min (MH+: 310.1936, Method: 7) |

| Number | Melting Point |
|---|---|
| **9F** | 107.9°C ((Mettler Toledo MP50), 10ºC/min ) |

Synthesis of 2-(4-(dimethylamino)-1-oxo-6-(trifluoromethyl)phthalazin-2(1H)-yl)acetic acid **1G**

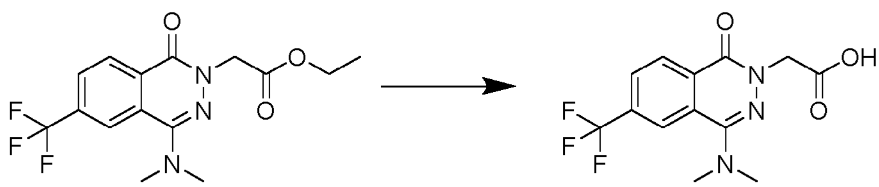

Sodium hydroxide (1.56 mL, 1 M in water, 1.56 mmol) was added to a solution of ethyl 2-(4-(dimethylamino)-1-oxo-6-(trifluoromethyl)phthalazin-2(1H)-yl)acetate **1F** (267 mg, 0.78 mmol) in MeOH (5 mL) and the reaction mixture was stirred at rt for 3 h. The reaction mixture was acidified with HCl 6 M in water to pH 3. Solvent was evaporated in vacuo to yield 2-(4-(dimethylamino)-1-oxo-6-(trifluoromethyl)phthalazin-2(1H)-yl)acetic acid **1G** as a yellow solid (impure with NaCl and NaOH (purity 70 % by LCMS), 344 mg, 98%). The reaction crude was used in the next reaction step without further purification.

30 mg of the crude was purified by reverse phase (Phenomenex Gemini C18 30x100mm 5μm Column; from 95% [25mM NH4HCO3] - 5% MeCN to 63% [25mM NH4HCO3] - 37% MeCN) to characterize the product. The desired fractions were collected and concentrated to yield 2-(4-(dimethylamino)-1-oxo-6-(trifluoromethyl)phthalazin-2(1H)-yl)acetic acid **1G** as a yellowish solid (17 mg).

**LCMS** (RT: 2.650, Area %: 99, MH+: 282.0 (Method: 1))

**$^1$H NMR** 1H NMR (400 MHz, DMSO-$d_6$) δ ppm 2.81 (s, 6H), 4.70 (s, 2H), 7.98 (dd, J = 8.7, 2.0 Hz, 1H), 8.02 (d, J = 8.7

Hz, 1H), 8.20 (d, J = 1.8 Hz, 1H).
**HRMS** (RT: 3.513 min (MH+: 282.1468, Method: 7))
**M.P.** 179.8°C (Mettler Toledo (MP50), 10°C/min )

The following compounds were prepared in accordance with the same or similar procedures.

| Number | Starting material | Final compound | yield |
|---|---|---|---|
| 3B | 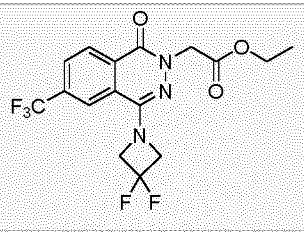 | 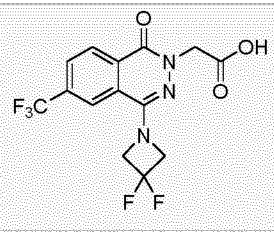 | 98% |
| 4F | 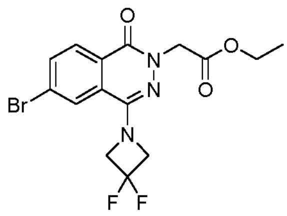 | 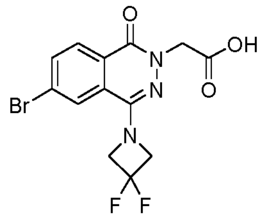 | 99% |

| Number | Starting material | Final compound | yield |
|---|---|---|---|
| 5B * | 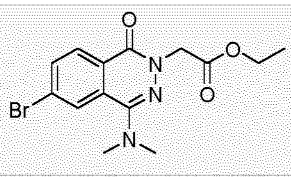 | 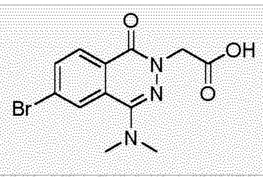 | 92% |
| 6B | 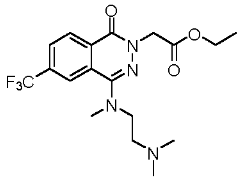 | 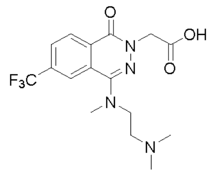 | 88% |
| 7B | 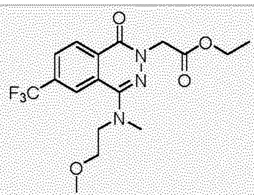 | 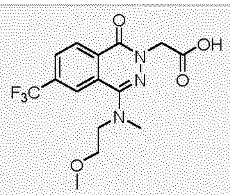 | 94% |
| 8G | 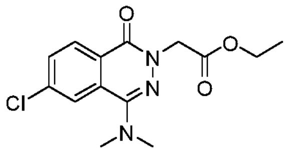 | 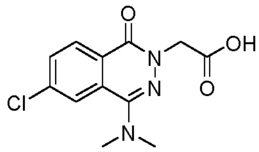 | 75% |
| 9G | 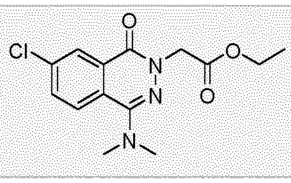 | 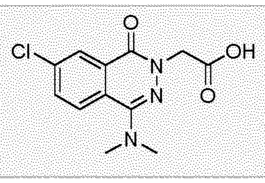 | 98% |

* Lithium hydroxide was used in place of sodium hydroxide (same ratio 2 equiv.)

| Number | LCMS |
|---|---|
| 3B | RT: 3.106, Area %: 99, MH+: 364.0, Method: 1 |
| 4F | RT: 0.734, Area %: 70, MH+: 373.9 |
| 5B | RT: 0.729, Area %: 97, MH+: 326.0 |
| 6B | RT: 1.19, Area %: 93.24, MW: 372.00, BPM1: 373, BPM2: 373, Method: 6 |
| 7B | RT: 1.22, Area %: 1.62, MW: 359.10, BPM1: 360, BPM2: 314, Method: 6 |
| 8G | RT: 2.718, Area %: 99, MH+: 282.0, Method: 1 |
| 9G | RT: 2.650, Area %: 99, MH+: 282.0, Method: 1 |

| Number | NMR |
|---|---|
| 3B | 1H NMR (400 MHz, DMSO-d6) δ ppm 4.67 (t, J = 12.8 Hz, 4H), 4.75 (s, 2H), 8.09 (s, 1H), 8.24 (dd, J = 8.4, 1.1 Hz, 1H), 8.49 (d, J = 8.3 Hz, 1H), OH not observed. |
| 4F | 1H NMR (300 MHz, DMSO-d6) δ ppm 4.58 (t, J = 12.3 Hz, 4H), 4.77 (s, 2H), 6.73 (d, J = 2.2 Hz, 1H), 7.11 (dd, J = 8.7, 2.2 Hz, 1H), 8.12 (d, J = 8.7 Hz, 1H), 13.11 (s, 1H). |

(continued)

| Number | NMR |
|---|---|
| **5B** | 1H NMR (400 MHz, DMSO-d6) δ ppm 2.80 (s, 6H), 4.69 (s, 2H), 8.05 (dd, J = 8.5, 1.8 Hz, 1H), 8.09 (d, J = 1.6 Hz, 1H), 8.17 (d, J = 8.5 Hz, 1H), 13.02 (s, 1H). |
| **8G** | 1H NMR (400 MHz, DMSO-d6) δ ppm 2.81 (s, 6H), 4.69 (s, 2H), 7.91 (dd, J = 8.5, 1.9 Hz, 1H), 7.95 (d, J = 1.9 Hz, 1H), 8.26 (d, J = 8.5 Hz, 1H), 13.04 (br s, 1H). |
| **9G** | 1H NMR (400 MHz, DMSO-d6) δ ppm 2.81 (s, 6H), 4.70 (s, 2H), 7.98 (dd, J = 8.7, 2.0 Hz, 1H), 8.02 (d, J = 8.7 Hz, 1H), 8.20 (d, J = 1.8 Hz, 1H). |

| Number | HRMS |
|---|---|
| **3B** | Rt: 4.095 min (MH+: 364.0651, Method: 7) |
| **8G** | Rt = 4.013 min (MH+: 282.1459, Method: 7) |
| **9G** | Rt = 3.513 min (MH+: 282.1468, Method: 7) |

| Number | Melting Point |
|---|---|
| **3B** | 231.6°C (Mettler Toledo (MP50), 10ºC/min ) |
| **8G** | 263.4°C (Mettler Toledo (MP50), 10ºC/min ) |
| **9G** | 240.0°C (Mettler Toledo (MP50), 10ºC/min ) |

Synthesis of N-([1,2,4]triazolo[4,3-a]pyridin-6-yl)-2-(4-(dimethylamino)-1-oxo-6-(trifluoromethyl)phthalazin-2(1H)-yl) acetamide **1H** (as a Final Compound of formula (I))

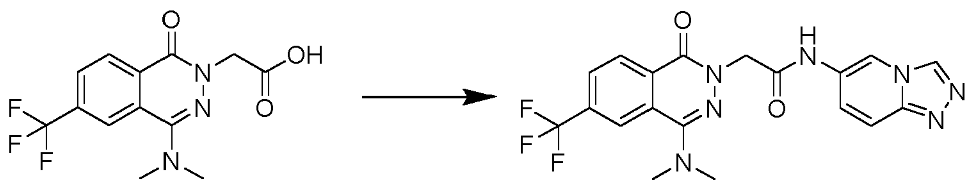

$Et_3N$ (167 μL, 1.21 mmol) was added to a stirred solution of 2-(4-(dimethylamino)-1-oxo-6-(trifluoromethyl) phthalazin-2(1H)-yl)acetic acid **1G** (140 mg, 0.30 mmol) and [1,2,4]triazolo[4,3-A]pyridin-7-amine (40.5 mg, 0.30 mmol) in dry DMF (0.9 mL) at R.T. under nitrogen. The mixture was stirred for 5 min, then 1-propanephosphonic anhydride (0.25 mL, 0.25 mmol) was added and the mixture was stirred 16h at rt. The mixture was diluted with $NaHCO_3$ sat. solution and extracted with EtOAc. The organic layer was separated, dried ($MgSO_4$), filtered and the solvents evaporated in vacuo. The crude product was co-distilled twice with toluene. The crude product was purified by flash column chromatography (silica (12 g); DCM:MeOH (9:1) in DCM 100/0 to 80/20).

The desired fractions were collected and concentrated in vacuo to yieldN-([1,2,4]triazolo[4,3-a]pyridin-6-yl)-2-(4-(dimethylamino)-1-oxo-6-(trifluoromethyl)phthalazin-2(1H)-yl)acetamide **1H** (47 mg, 35%) as a pale yellow solid.

**LCMS** (RT: 2.770, Area %: 96, MH+: 432.0 (Method: 1))
**$^1$H NMR** 1H NMR (400 MHz, DMSO-$d_6$) δ ppm 2.85 (s, 6H), 4.93 (s, 2H), 7.31 (dd, J = 9.8, 1.9 Hz, 1H), 7.79 (d, J = 9.8 Hz, 1H), 8.22 (d, J = 8.3 Hz, 2H), 8.49 (d, J = 8.1 Hz, 1H), 9.20 (s, 1H), 9.23 (s, 1H), 10.53 (s, 1H).
**HRMS** (RT= 3.438 min (MH+: 432.1365 (Method: 7))
**M.P.** 278.6 °C (Mettler Toledo (MP50), 10°C/min )

The following final compounds were prepared in accordance with the same or similar procedures.

| Number | Reagent | Starting material | Final compound | yield |
| --- | --- | --- | --- | --- |
| **1I** | 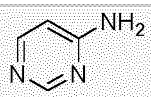 [591-54-8] | 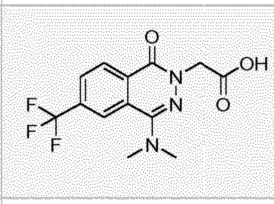 | 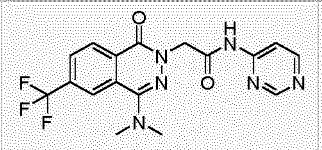 | 46% |
| **1J** | 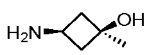 [1363381-58-1] | 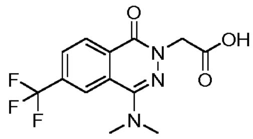 | 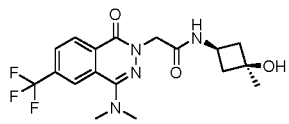 | 59% |
| **1K** | 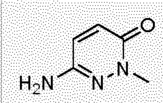 [13506-28-0] | 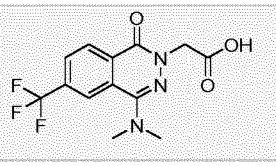 | 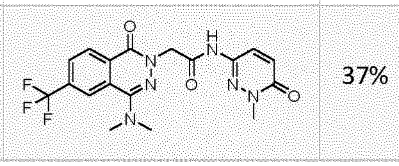 | 37% |

| Number | Reagent | Starting material | Final compound | yield |
|---|---|---|---|---|
| **3C** | 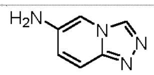 [1082448-58-5] | 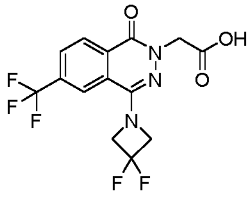 | 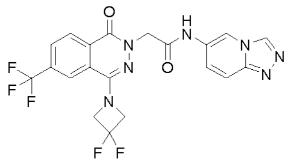 | 38% |
| **3D** | 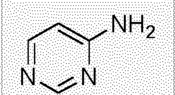 [591-54-8] | 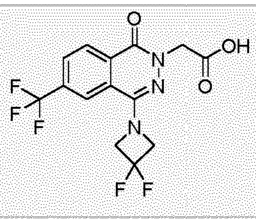 | 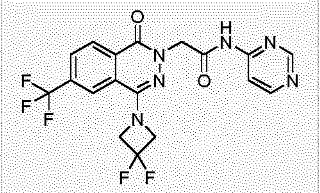 | 11% |
| **3E** | 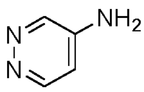 [20744-39-2] | 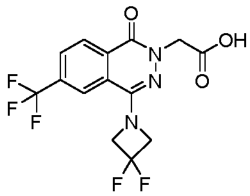 | 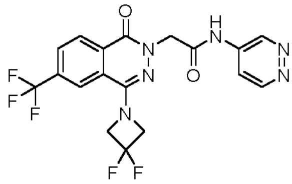 | 38% |
| **3F** | 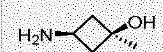 [1363381-58-1] | 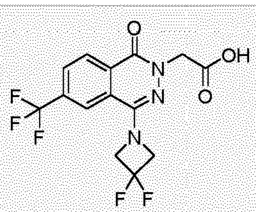 | 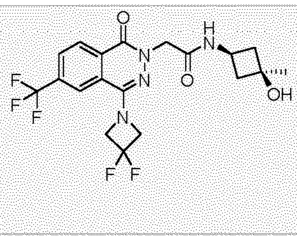 | 19% |
| **3G** | 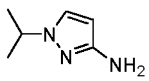 [857267-04-0] | 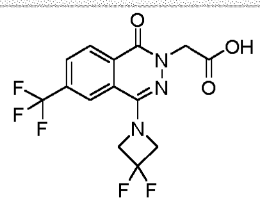 | 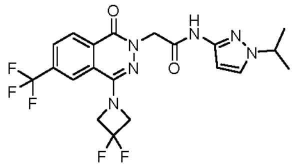 | 12% |
| **4G** | 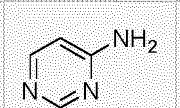 [591-54-8] | 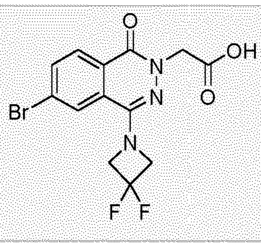 | 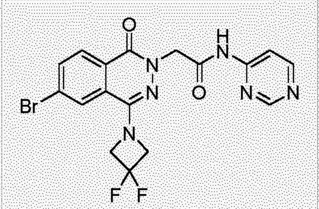 | 37% |
| **5C** | 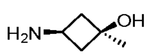 [1363381-58-1] | 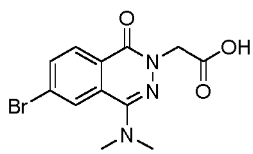 | 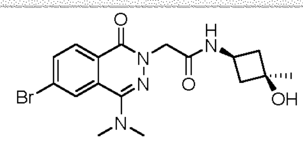 | 56% |

| Number | Reagent | Starting material | Final compound | yield |
|---|---|---|---|---|
| 5D | 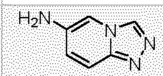 [1082448-58-5] | 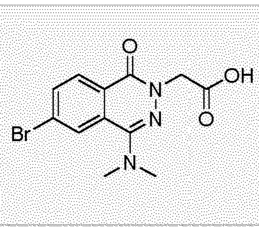 | 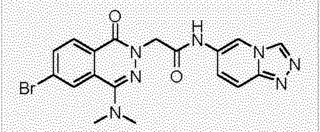 | 43% |
| 5E | 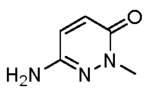 [13506-28-0] | 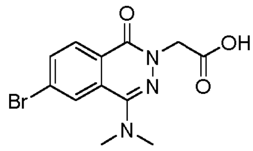 | 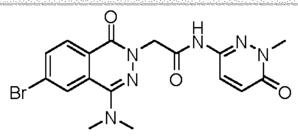 | 37% |
| 6C | 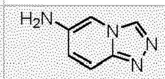 [1082448-58-5] | 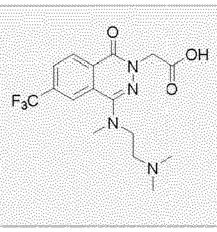 | 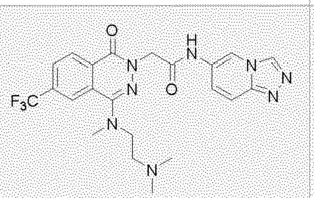 | 90% |
| 7C | 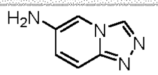 [1082448-58-5] | 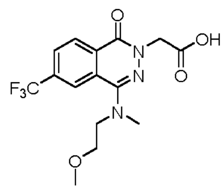 | 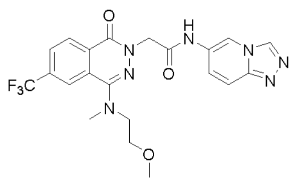 | 57% |
| 8H | 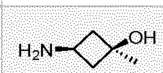 [1363381-58-1] | 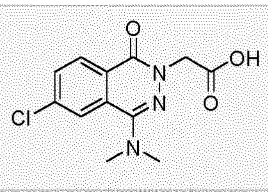 | 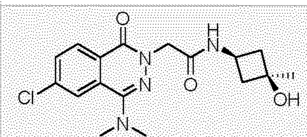 | 58% |
| 8I | 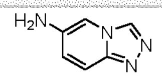 [1082448-58-5] | 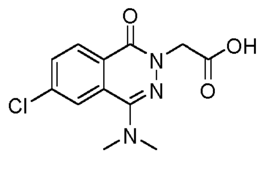 | 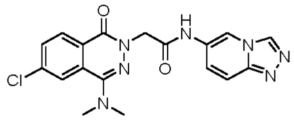 | 35% |
| 8J | 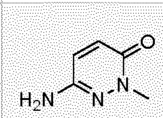 [13506-28-0] | 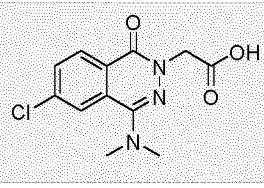 | 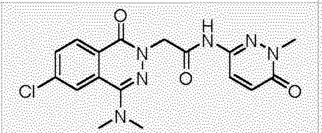 | 19% |
| 9H | 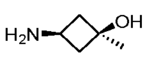 [1363381-58-1] | 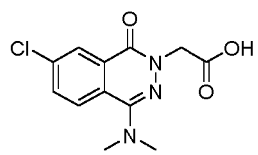 | 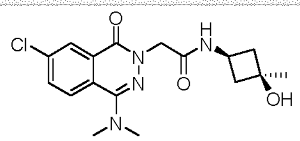 | 37% |

| Number | Reagent | Starting material | Final compound | yield |
|---|---|---|---|---|
| 9I | 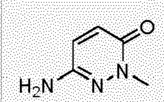 [13506-28-0] | 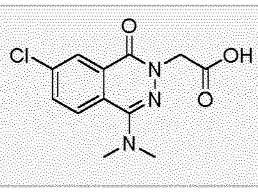 | 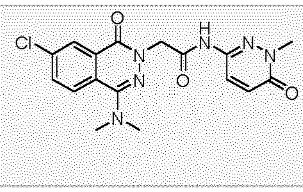 | 38% |
| 9J | 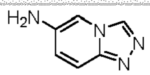 [1082448-58-5] | 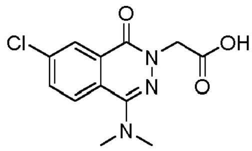 | 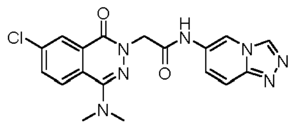 | 20% |

| Number | LCMS |
|---|---|
| 1I | RT: 3.088, Area %: 98, MH+: 393.1, Method: 1 |
| 1J | RT: 2.757, Area %: 98, MH+: 399.1, Method: 1 |
| 1K | RT: 2.924, Area %: 99, MH+: 423.1, Method: 1 |
| 3C | RT: 2.885, Area %: 99, MH+: 480.0, Method: 1 |
| 3D | RT: 3.188, Area %: 99, MH+: 441.0, Method: 1 |
| 3E | RT: 2.899, Area %: 97, MH+: 441.0, Method: 1 |
| 3F | RT: 2.904, Area %: 99, MH+: 447.1, Method: 1 |
| 3G | RT: 3.521, Area %: 98, MH+: 471.1, Method: 1 |
| 4G | RT: 2.975, Area %: 97, MH+: 451.0, Method: 1 |
| 5C | RT: 2.591, Area %: 98, MH+: 409.0, Method: 1 |
| 5D | RT: 2.640, Area %: 97, MH+: 442.0, Method: 1 |
| 5E | RT: 2.771, Area %: 95, MH+: 433.0, Method: 1 |
| 6C | RT: 1.59, Area %: 100.00, MW: 488.00, BPM1: 489, BPM2: 489, Method: 6 |
| 7C | RT: 1.62, Area %: 2.05, MW: 475.00, BPM1: 476, BPM2: 476, Method: 6 |
| 8H | RT: 2.505, Area %: 99, MH+: 365.1, Method: 1 |
| 8I | RT: 2.558, Area %: 99, MH+: 398.1, Method: 1 |
| 8J | RT: 2.684, Area %: 99, MH+: 389.1, Method: 1 |
| 9H | RT: 2.448, Area %: 99, MH+: 365.0, Method: 1 |
| 9I | RT: 2.622, Area %: 97, MH+: 389.1, Method: 1 |
| 9J | RT: 2.496, Area %: 99, MH+: 398.1, Method: 1 |

| Number | NMR |
|---|---|
| 1I | 1H NMR (400 MHz, DMSO-$d_6$) δ ppm 2.84 (s, 6H), 4.98 (s, 2H), 7.99 (dd, J = 5.8, 1.0 Hz, 1H), 8.21 (d, J = 7.8 Hz, 2H), 8.47 (d, J = 8.2 Hz, 1H), 8.66 (d, J = 5.8 Hz, 1H), 8.92 (d, J = 0.9 Hz, 1H), 11.29 (s, 1H). |
| 1J | 1H NMR (400 MHz, DMSO-$d_6$) δ ppm 1.21 (s, 3H), 1.94 (dd, J = 11.2, 8.9 Hz, 2H), 2.19 - 2.25 (m, 2H), 2.82 (s, 6H), 3.71 - 3.83 (m, 1H), 4.60 (s, 2H), 4.96 (s, 1H), 8.19 (d, J = 7.2 Hz, 2H), 8.23 (d, J = 7.2 Hz, 1H), 8.46 (d, J = 8.6 Hz, 1H). |

(continued)

| Number | NMR |
|---|---|
| **1K** | 1H NMR (400 MHz, DMSO-$d_6$) δ ppm 2.84 (s, 6H), 3.59 (s, 3H), 4.88 (s, 2H), 6.97 (d, J = 9.9 Hz, 1H), 7.95 (t, J = 17.6 Hz, 1H), 8.20 (d, J = 7.2 Hz, 2H), 8.49 (dd, J = 18.1, 8.6 Hz, 1H), 10.91 (s, 1H). |
| **3C** | 1H NMR (400 MHz, DMSO-$d_6$) δ ppm 4.68 (t, J = 12.8 Hz, 4H), 4.95 (s, 2H), 7.32 (dd, J = 9.8, 1.7 Hz, 1H), 7.79 (d, J = 9.7 Hz, 1H), 8.12 (s, 1H), 8.25 (d, J = 8.4 Hz, 1H), 8.51 (d, J = 8.3 Hz, 1H), 9.20 (s, 1H), 9.24 (s, 1H), 10.56 (s, 1H). |
| **3D** | 1H NMR (400 MHz, DMSO-$d_6$) δ ppm 4.67 (t, J = 12.8 Hz, 4H), 5.00 (s, 2H), 7.98 (dd, J = 5.8, 0.9 Hz, 1H), 8.11 (s, 1H), 8.24 (d, J = 8.4 Hz, 1H), 8.49 (d, J = 8.3 Hz, 1H), 8.66 (d, J = 5.8 Hz, 1H), 8.92 (d, J = 0.8 Hz, 1H), 11.30 (s, 1H). |
| **3E** | 1H NMR (400 MHz, DMSO-$d_6$) δ ppm 4.60 - 4.75 (m, 4H), 4.97 (s, 2H), 7.88 (dd, J = 5.9, 2.7 Hz, 1H), 8.11 (s, 1H), 8.24 (d, J = 8.5 Hz, 1H), 8.49 (t, J = 6.3 Hz, 1H), 9.04 (d, J = 5.9 Hz, 1H), 9.30 (d, J = 2.1 Hz, 1H), 10.99 (s, 1H). |
| **3F** | 1H NMR (400 MHz, DMSO-$d_6$) δ ppm 1.21 (s, 3H), 1.87 - 2.00 (m, 2H), 2.16 - 2.28 (m, 2H), 3.70 - 3.84 (m, 1H), 8.48 (d, J = 8.3 Hz, 1H), 4.63 (dd, J = 17.1, 8.5 Hz, 6H), 4.97 (s, 1H), 8.08 (s, 1H), 8.23 (t, J = 8.5 Hz, 2H). |
| **3G** | 1H NMR (400 MHz, DMSO-$d_6$) δ ppm 1.37 (s, 3H), 1.39 (s, 3H), 4.38 (dq, J = 13.2, 6.6 Hz, 1H), 4.65 (t, J = 12.8 Hz, 4H), 4.83 (s, 2H), 6.38 (d, J = 2.2 Hz, 1H), 7.61 (d, J = 2.2 Hz, 1H), 8.09 (s, 1H), 8.23 (d, J = 8.5 Hz, 1H), 8.49 (d, J = 8.3 Hz, 1H), 10.74 (s, 1H). |
| **4G** | 1H NMR (400 MHz, DMSO-$d_6$) δ ppm 4.58 (t, J = 12.3 Hz, 4H), 5.02 (s, 2H), 6.74 (d, J = 2.1 Hz, 1H), 7.12 (dd, J = 8.6, 2.1 Hz, 1H), 7.97 (d, J = 5.8 Hz, 1H), 8.11 (d, J = 8.7 Hz, 1H), 8.66 (d, J = 5.8 Hz, 1H), 8.92 (s, 1H), 11.31 (s, 1H). |
| **5C** | 1H NMR (400 MHz, DMSO-$d_6$) δ ppm 1.21 (s, 3H), 1.93 (dd, J = 11.2, 9.0 Hz, 2H), 2.17 - 2.26 (m, 2H), 2.79 (s, 6H), 3.68 - 3.82 (m, 1H), 4.56 (s, 2H), 4.96 (s, 1H), 8.03 (dd, J = 8.5, 1.8 Hz, 1H), 11.31 (s, 1H), |
| **5D** | 1H NMR (400 MHz, DMSO-$d_6$) δ ppm 2.81 (s, 6H), 4.89 (s, 2H), 7.31 (dd, J = 9.8, 1.6 Hz, 1H), 7.79 (d, J = 9.8 Hz, 1H), 8.05 (dd, J= 8.5,1.7 Hz, 1H), 8.11 (d, J = 1.5 Hz, 1H), 8.18 (d, J = 8.5 Hz, 1H), 9.20 (s, 1H), 9.23 (s, 1H), 10.58 (d, J = 34.5 Hz, 1H). |
| **5E** | 1H NMR (400 MHz, DMSO-$d_6$) δ ppm 2.80 (s, 6H), 3.59 (s, 3H), 4.84 (s, 2H), 6.97 (d, J = 9.9 Hz, 1H), 7.92 (d, J = 10.0 Hz, 1H), 8.04 (dd, J = 8.5, 1.8 Hz, 1H), 8.10 (d, J = 1.6 Hz, 1H), 8.17 (d, J = 8.5 Hz, 1H), 10.89 (s, 1H). |
| **6C** | 1H NMR (400 MHz, DMSO-$d_6$) δ ppm 2.17 (s, 6 H) 2.53 - 2.61 (m, 2 H) 2.82 (s, 3 H) 3.15 (t, J=6.2 Hz, 2 H) 4.94 (s, 2 H) 7.32 (dd, J=9.8, 1.8 Hz, 1 H) 7.80 (d, J=9.8 Hz, 1 H) 8.20 (d, J=8.6 Hz, 1 H) 8.48 (d, J=8.3 Hz, 1 H) 8.65 (s, 1 H) 9.21 (s, 1 H) 9.25 (s, 1 H) 10.58 (br s, 1 H), 1 H) |
| **7C** | 1H NMR (400 MHz, DMSO-d6) d ppm 2.83 (s, 3 H) 3.19 - 3.30 (m, 2 H) 3.66 (t, J=4.8 Hz, 2 H) 4.94 (s, 2 H) 7.32 (dd, J=9.8, 1.6 Hz, 1 H) 7.80 (d, J=9.7 Hz, 1 H) 8.19 (br d, J=8.2 Hz, 1 H) 8.47 (d, J=8.3 Hz, 1 H) 8.79 (s, 1 H) 9.19 - 9.27 (m, 2 H) 10.58 (br s, 1 H) |
| **8H** | 1H NMR (400 MHz, DMSO-$d_6$) δ ppm 1.21 (s, 3H), 1.87 - 1.99 (m, 2H), 2.16 - 2.27 (m, 2H), 2.79 (s, 6H), 3.70 - 3.82 (m, 1H), 4.57 (s, 2H), 4.96 (br s, 1H), 7.89 (dd, J = 8.5, 1.7 Hz, 1H), 7.93 (d, J = 1.7 Hz, 1H), 8.24 (d, J = 8.5 Hz, 1H). |
| **8I** | 1H NMR (400 MHz, DMSO-$d_6$) δ ppm 2.81 (s, 6H), 4.90 (s, 2H), 7.35 (dd, J = 9.8, 1.6 Hz, 1H), 7.79 (d, J = 9.8 Hz, 1H), 7.92 (dd, J = 8.5, 1.9 Hz, 1H), 7.97 (d, J = 1.8 Hz, 1H), 8.27 (d, J = 8.5 Hz, 1H), 9.22 (d, J = 10.5 Hz, 2H), 10.69 (br s, 1H). |
| **8J** | 1H NMR (400 MHz, DMSO-$d_6$) δ ppm 1.21 (s, 3H), 1.87 - 1.99 (m, 2H), 2.16 - 2.27 (m, 2H), 2.79 (s, 6H), 3.70 - 3.82 (m, 1H), 4.57 (s, 2H), 4.96 (br s, 1H), 7.89 (dd, J = 8.5, 1.7 Hz, 1H), 7.93 (d, J = 1.7 Hz, 1H), 8.23 (br d, J = 7.0 Hz, 1H), 8.24 (d, J = 8.5 Hz, 1H). |
| **9H** | 1H NMR (400 MHz, DMSO-$d_6$) δ ppm 1.22 (d, J = 9.7 Hz, 3H), 1.84 -2.04 (m, 2H), 2.15 - 2.28 (m, 2H), 2.80 (s, 6H), 3.76 (dt, J = 16.1, 8.0 Hz, 1H), 4.95 (s, 1H), 4.57 (s, 2H), 7.98 (dt, J = 8.7, 5.4 Hz, 2H), 8.20 (dd, J = 12.5, 4.6 Hz, 2H). |
| **9I** | 1H NMR (400 MHz, DMSO-$d_6$) δ ppm 2.81 (s, 6H), 3.59 (s, 3H), 4.85 (s, 2H), 6.97 (d, J = 9.9 Hz, 1H), 7.92 (d, J = 9.6 Hz, 1H), 8.00 (dt, J = 8.3, 5.2 Hz, 2H), 8.20 (d, J = 1.9 Hz, 1H), 10.89 (s, 1H). |

(continued)

| Number | NMR |
|---|---|
| 9J | 1H NMR (400 MHz, DMSO-$d_6$) δ ppm 2.82 (s, 6H), 4.90 (s, 2H), 7.31 (dd, J = 9.8, 1.6 Hz, 1H), 7.79 (d, J = 9.7 Hz, 1H), 8.00 (dd, J = 8.7, 2.1 Hz, 1H), 8.05 (d, J = 8.7 Hz, 1H), 8.22 (d, J = 2.0 Hz, 1H), 9.20 (s, 1H), 9.23 (s, 1H), 10.52 (s, 1H). |

| Number | HRMS |
|---|---|
| 1I | RT= 4.035 min (MH+: 393.1233, Method: 7) |
| 1J | RT= 3.843 min (MH+: 399.1587, Method: 7) |
| 1K | RT= 3.845 min (MH+: 423.2387, Method: 7) |
| 3C | RT= 3.786 min (MH+: 480.1105, Method: 7) |
| 3D | RT= 4.174 min (MH+: 441.1010, Method: 7) |
| 3E | RT= 3.691 min (MH+: 441.1000, Method: 7) |
| 3F | RT= 4.258 min (MH+: 447.0896, Method: 7) |
| 3G | RT= 5.070 min (MH+: 471.0881, Method: 7) |
| 4G | RT= 3.761 min (MH-: 451.0217, Method: 7) |
| 5C | RT= 3.068 min (MH+: 409.1834, Method: 7) |
| 5D | RT= 3.000 min (MH+: 442.1641, Method: 7) |
| 5E | RT=3.904 min (MH+: 433.1614, Method: 7) |
| 8H | RT= 3.051 min (MH+: 365.2417, Method: 7) |
| 8I | RT= 2.558 min (MH+: 398.2295, Method: 7) |
| 8J | RT= 3.051 min (MH+: 389.2080, Method: 7) |
| 9H | RT= 3.136 min (MH+: 365.2311, Method: 7) |
| 9I | RT= 3.250 min (MH+: 389.208, Method: 7) |
| 9J | RT=3.114 min (MH+: 398.2220, Method: 7) |

| Number | Melting Point |
|---|---|
| 1I | 238.3°C (Mettler Toledo (MP50), 10°C/min ) |
| 1J | 184.8°C (Mettler Toledo (MP50), 10°C/min ) |
| 1K | 241.8°C (Mettler Toledo (MP50), 10°C/min ) |
| 3C | >300°C (Mettler Toledo (MP50), 10°C/min ) |
| 3D | 288.6°C (Mettler Toledo (MP50), 10°C/min ) |
| 3E | 221.6°C (Mettler Toledo (MP50), 10°C/min ) |
| 3F | 213.3°C (Mettler Toledo (MP50), 10°C/min ) |
| 3G | 220.0°C (Mettler Toledo (MP50), 10°C/min ) |
| 4G | 286.8°C (Mettler Toledo (MP50), 10°C/min ) |
| 5C | 193.2°C (Mettler Toledo (MP50), 10°C/min ) |
| 5D | >300°C (Mettler Toledo (MP50), 10°C/min ) |
| 5E | 246.8°C (Mettler Toledo (MP50), 10°C/min ) |
| 6C | 252.55 °C (DSC: From 30 to 400 °C at 10°C/min 50ml N2) |
| 7C | 209.01 °C (DSC: From 30 to 400 °C at 10°C/min 50ml N2) |
| 8H | 184.8°C (Mettler Toledo (MP50), 10°C/min ) |

(continued)

| Number | Melting Point |
|---|---|
| **8I** | 295,3°C (Mettler Toledo (MP50), 10°C/min ) |
| **8J** | 253.4°C (Mettler Toledo (MP50), 10°C/min ) |
| **9H** | 173.1°C (Mettler Toledo (MP50), 10°C/min ) |
| **9I** | 213.2°C (Mettler Toledo (MP50), 10°C/min ) |
| **9J** | >300°C (Mettler Toledo (MP50), 10°C/min ) |

Synthesis N-([1,2,4]triazolo[4,3-b]pyridazin-6-yl)-2-(4-(dimethylamino)-1-oxo-6-(trifluoromethyl)phthalazin-2(1H)-yl) acetamide **2A**

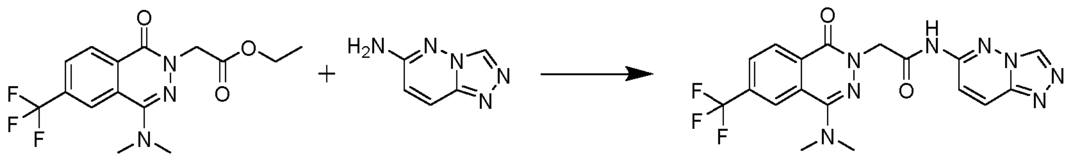

[1,2,4]Triazolo[4,3-B]pyridazin-6-amine (3.94 g, 29.13 mmol) was suspended in DMF, PA dry (90.6 mL) in dry glassware under a nitrogen atmosphere. The reaction mixture was cooled to 0°C and LiHMDS (1.0 M in THF) (55.93 mL, 55.93 mmol) was added over a 25 min period via a syringe pump at 0°C. After the addition was complete, the reaction mixture was stirred at 0°C for 15 min. A solution of ethyl 2-(4-(dimethylamino)-1-oxo-6-(trifluoromethyl)phthalazin-2(1H)-yl)acetate **1F** (8 g, 23.3 mmol) in THF PA dry (75.52 mL) was added over a 25 min period via a syringe pump at 0°C. After the addition was complete, the reaction mixture was stirred at 0°C for 15 min and then slowly warmed up to rt. The reaction mixture was stirred at rt for 2.5 h.

The THF was evaporated at 45°C. The residue was poured out in 500 mL sat. $NaHCO_3$ solution and the mixture was stirred at R.T. for 15 min. The solids were filtered and washed well with water three times. The filter cake was placed in 650 ml ACN and heated until reflux (clouded solution). This solution was filtered hot through a micropore filter and the filtrate was slowly crystallized while cooling. After the solution had reached R.T., the solids were filtered and washed three times with a small portion of ACN. The solids were dried under vacuum at 50°C overnight, yielding N-([1,2,4]triazolo[4,3-b] pyridazin-6-yl)-2-(4-(dimethylamino)-1-oxo-6-(trifluoromethyl)phthalazin-2(1H)-yl)acetamide **2A** (6.13 g, yield 61%) as an off white solid.

**LCMS** (RT: 1.65, Area %: 100.00, MW: 432.00, BPM1: 433, BPM2: 431 (Method: 3))
**$^1$H NMR** (400 MHz, DMSO-$d_6$, 27°C) δ ppm 2.85 (s, 6 H) 5.01 (s, 2 H) 7.93 (br d, J=9.74 Hz, 1 H) 8.20 - 8.23 (m, 1 H) 8.22 (s, 1 H) 8.35 (dd, J=10.07, 0.83 Hz, 1 H) 8.48 (d, J=8.26 Hz, 1 H) 9.52 (d, J=0.81 Hz, 1 H) 11.44 (br s, 1 H)
**$^{13}$C NMR** (151 MHz, DMSO-$d_6$) δ ppm 42.89 (s, 2 C) 54.98 (s, 1 C) 117.51 (s, 1 C) 123.94 (q, J=273.00 Hz, 1 C) 123.02 (q, J=4.42 Hz, 1 C) 126.12 (s, 1 C) 127.34 (s, 1 C) 128.27 (br q, J=3.32 Hz, 1 C) 129.11 (s, 1 C) 131.50 (s, 1 C) 133.32 (q, J=32.80 Hz, 1 C) 139.29 (s, 1 C) 142.95 (s, 1 C) 149.72 (s, 1 C) 150.34 (s, 1 C) 157.56 (s, 1 C) 167.83 (s, 1 C)
**M.P.** 254.28 °C (DSC: From 30 to 400 °C at 10°C/min 50ml $N_2$)

The following final compounds of formula (I) were prepared in accordance with the same or similar procedures.

| Number | Starting material | Final compound | yield |
|---|---|---|---|
| **10A** | 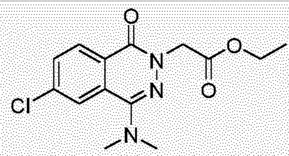 **8F** | 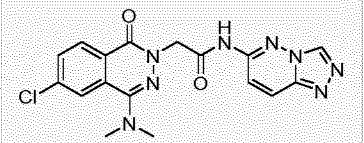 | 14% |
| **11A** | 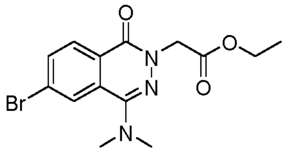 **5A** | 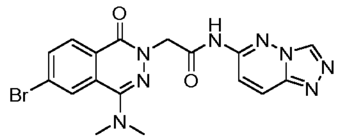 | 4% |

| Number | LCMS |
|---|---|
| **10A** | RT: 2.670, Area %: 99, MH+: 399.1, Method: 1 |
| **11A** | RT: 2.720, Area %: 97, MH+: 443.0, Method: 1 |

| Number | NMR |
|---|---|
| **10A** | 1H NMR (400 MHz, $CDCl_3$) δ ppm 2.93 (s, 6H), 4.99 (s, 2H), 7.75 (d, J = 8.4 Hz, 1H), 7.95 (s, 1H), 8.10 (d, J = 10.1 Hz, 1H), 8.23 (d, J = 10.1 Hz, 1H), 8.44 (d, J = 8.5 Hz, 1H), 8.92 (s, 1H), 9.60 (s, 1H). |
| **11A** | 1H NMR (400 MHz, DMSO-d6) δ ppm 2.81 (s, 6H), 4.96 (s, 2H), 7.92 (d, J = 9.9 Hz, 1H), 8.06 (d, J = 8.4 Hz, 1H), 8.18 (d, J = 8.5 Hz, 1H), 8.11 (s, 1H), 8.35 (d, J = 10.0 Hz, 1H), 9.52 (s, 1H), 11.42 (s, 1H). |

| Number | HRMS |
|---|---|
| **10A** | RT= 3.891 min (MH+: 399.2027, Method: 7) |
| **11A** | RT= 3.353 min (MH+: 443.1582, Method: 7) |

| Number | Melting Point |
|---|---|
| **10A** | 270.3°C (Mettler Toledo (MP50), 10°C/min ) |
| **11A** | 231.6°C (Mettler Toledo (MP50), 10°C/min ) |

Example B - Pharmaceutical Compositions

A compound of formula (I) (for instance, a compound of the examples) is brought into association with a pharmaceutically acceptable carrier, thereby providing a pharmaceutical composition comprising such active compound. A therapeutically effective amount of a compound of formula (I) (e.g. a compound of the examples) is intimately mixed with a pharmaceutically acceptable carrier, in a process for preparing a pharmaceutical composition.

Example C - Biological Examples

The activity of a compound according to the present invention can be assessed by *in vitro* methods. A compound the invention exhibits valuable pharmacological properties, e.g. properties susceptible to inhibit NLRP3 activity, for instance as indicated the following test, and are therefore indicated for therapy related to NLRP3 inflammasome activity.

PBMC assay

Peripheral venous blood was collected from healthy individuals and human peripheral blood mononuclear cells

(PBMCs) were isolated from blood by Ficoll-Histopaque (Sigma-Aldrich, A0561) density gradient centrifugation. After isolation, PBMCs were stored in liquid nitrogen for later use. Upon thawing, PBMC cell viability was determined in growth medium (RPMI media supplemented with 10% fetal bovine serum, 1% Pen-Strep and 1% L-glutamine). Compounds were spotted in a 1:3 serial dilution in DMSO and diluted to the final concentration in 30 μl medium in 96 well plates (Falcon, 353072). PBMCs were added at a density of $7.5 \times 10^4$ cells per well and incubated for 30 min in a 5% $CO_2$ incubator at 37 °C. LPS stimulation was performed by addition of 100 ng/ml LPS (final concentration, Invivogen, tlrl-smlps) for 6 hrs followed by collection of cellular supernatant and the analysis of IL-1β (μM) and TNF cytokines levels (μM) via MSD technology according to manufacturers' guidelines (MSD, K151A0H).

The $IC_{50}$ values (for IL-1β) and $EC_{50}$ values (TNF) were obtained on compounds of formula (I)/examples, and are depicted in the following table:

| Compound | IL1β $IC_{50}$ (μM) | **TNF** $EC_{50}$ (μM) |
|---|---|---|
| **1I** | 0.17 | >10 |
| **5E** | 0.17 | >10 |
| **10A** | 0.13 | >10 |
| **8H** | 0.12 | >10 |
| **11A** | 0.05 | >10 |
| **5D** | 0.05 | >10 |
| **1H** | 0.08 | >10 |
| **1J** | 0.07 | >10 |
| **2A** | 0.07 | >10 |
| **5C** | 0.03 | >10 |
| **3C** | >10 | >10 |
| **3D** | 3.32 | >10 |
| **3E** | 9.61 | >10 |
| **4G** | 4.29 | >10 |
| **3F** | 3.09 | >10 |
| **3G** | >10 | >10 |
| **6C** | >10 | >10 |
| **7C** | 5.63 | >10 |
| **1K** | 0.36 | >10 |
| **9J** | >10 | >10 |
| **9H** | >10 | >10 |
| **9I** | >10 | >10 |
| **8J** | 0.84 | >10 |
| **8I** | 0.31 | >10 |

Example D - Further Testing

One or more compound(s) of the invention (including compounds of the final examples) is/are tested in a number of other methods to evaluate, amongst other properties, permeability, stability (including metabolic stability and blood stability) and solubility.

**Permeability test**

The *in vitro* passive permeability and the ability to be a transported substrate of P-glycoprotein (P-gp) is tested using MDCKcells stably transduced with MDR1 (this may be performed at a commercial organisation offering ADME, PK

services, e.g. Cyprotex). Permeability experiments are conducted in duplicate at a single concentration (5 μM) in a transwell system with an incubation of 120 min. The apical to basolateral (AtoB) transport in the presence and absence of the P-gp inhibitor GF120918 and the basolateral to apical (BtoA) transport in the absence of the P-gp inhibitor is measured and permeation rates (Apparent Permeability) of the test compounds ($P_{app}$ x$10^{-6}$ cm/sec) are calculated.

**Metabolic stability test in liver microsomes**

The metabolic stability of a test compound is tested (this may be performed at a commercial organization offering ADME, PK services, e.g. Cyprotex) by using liver microsomes (0.5 mg/ml protein) from human and preclinical species incubated up to 60 minutes at 37°C with 1 μM test compound.

The *in vitro* metabolic half-life ($t_{1/2}$) is calculated using the slope of the log-linear regression from the percentage parent compound remaining versus time relationship (κ),

$$t_{1/2} = - \ln(2)/ \kappa.$$

The *in vitro* intrinsic clearance ($Cl_{int}$) (ml/min/mg microsomal protein) is calculated using the following formula:

$$Cl_{int} = \frac{0.693}{t_{1/2}} \times \frac{V_{inc}}{W_{mic\ prot,inc}}$$

Where:

$V_{inc}$ = incubation volume,

$W_{mic\ prot,inc}$ = weight of microsomal protein in the incubation.

**Metabolic stability test in liver hepatocytes**

The metabolic stability of a test compound is tested using liver hepatocytes (1 milj cells) from human and preclinical species incubated up to 120 minutes at 37°C with 1 μM test compound.

The in vitro metabolic half-life ($t_{1/2}$) is calculated using the slope of the log-linear regression from the percentage parent compound remaining versus time relationship (κ),

$$t_{1/2} = - \ln(2)/ \kappa.$$

The *in vitro* intrinsic clearance ($Cl_{int}$) (μl/min/million cells) is calculated using the following formula:

$$Cl_{int} = \frac{0.693}{t_{1/2}} \times \frac{V_{inc}}{\#\ cells_{inc}}\ x\ 1000$$

Where:

$V_{inc}$ = incubation volume,
# $cells_{inc}$ = number of cells (x$10^6$) in the incubation

**Solubility test**

The test/assay is run in triplicate and is semi-automated using the Tecan Fluent for all liquid handling with the following general steps:

- 20μl of 10mM stock solution is dispensed in a 500μl 96 well plate
- DMSO is evaporated (Genevac)
- a stir bar and 400μl of buffer/biorelevant media is added
- the solution is stirred for 72h (pH2 and pH7) or 24h (FaSSIF and FeSSIF)
- the solution is filtered
- the filtrate is quantified by UPLC/UV using a three-points calibration curve

The LC conditions are:

- Waters Acquity UPLC
- Mobile phase A: 0.1% formic acid in H2O, B: 0.1% formic acid in CH3CN
- Column: Waters HSS T3 1.8μm 2.1x50mm
- Column temp.: 55°C
- Inj.vol.: 2μl
- Flow: 0.6ml/min
- Wavelength UV: 250_350nm
- Gradient : 0min: 0%B, 0.3min: 5%B, 1.8min: 95%B, 2.6min: 95%B

**Blood Stability assay**

The compound of formula (I)/examples is spiked at a certain concentration in plasma or blood from the agreed preclinical species; then after incubating to predetermined times and conditions (37°C, 0°C (ice) or room temperature) the concentration of the test compound in the blood or plasma matrix with LCMS/MS can then be determined.

**Numbered clauses**

1. A compound of formula (I),

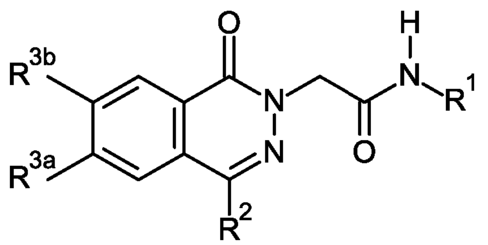

(I)

or a pharmaceutically acceptable salt thereof, wherein:

$R^1$ represents:

(i) $C_{3-6}$ cycloalkyl optionally substituted with one or more substituents independently selected from -OH and $-C_{1-3}$ alkyl;
(ii) aryl or heteroaryl, each of which is optionally substituted with 1 to 3 substituents independently selected from halo, -OH, $-O-C_{1-3}$ alkyl, $-C_{1-3}$ alkyl, halo$C_{1-3}$alkyl, hydroxy$C_{1-3}$ alkyl, $C_{1-3}$ alkoxy, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents $-N(R^{2a})R^{2b}$;
$R^{2a}$ and $R^{2b}$ each represent hydrogen or $C_{1-4}$ alkyl optionally substituted with one or more substituents selected from halo, $-OC_{1-3}$ alkyl and $-N(C_{1-3}\ alkyl)_2$, or $R^{2a}$ and $R^{2b}$ may be linked together to form a 3- to 4-membered ring optionally substituted by one or more fluoro atoms;
one of $R^{3a}$ and $R^{3b}$ represents hydrogen, and the other represents $R^3$;
$R^3$ represents:

(i) hydrogen;
(ii) halo;
(iii) $C_{1-4}$ alkyl optionally substituted with one or more substituents independently selected from halo, -OH and $-OC_{1-3}$ alkyl;
(iv) $C_{2-4}$ alkenyl optionally substituted with $-OC_{1-3}$ alkyl;
(v) $C_{3-6}$ cycloalkyl; or
(vi) $-OC_{1-3}$ alkyl.

2. The compound of clause 1 wherein $R^1$ represents

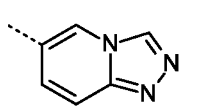
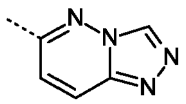
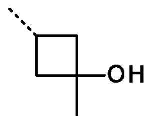
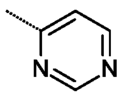
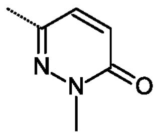
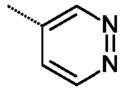
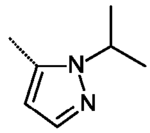

.

3. The compound of clause 2 wherein $R^1$ represents

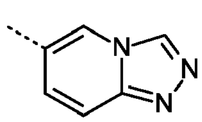

or

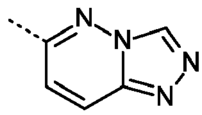

.

4. The compound of clause 1, 2, or 3 wherein one of $R^{2a}$ and $R^{2b}$ represents $C_{1-3}$ alkyl (e.g. unsubstituted methyl) and the other represents $C_{1-3}$ alkyl optionally substituted by one substituent selected from $-N(CH_3)_2$ and $-OCH_3$, or $R^{2a}$ and $R^{2b}$ are linked together to form a 4-membered ring optionally substituted by one or two fluoro atoms.

5. The compound of clause 4 wherein $R^2$ represents 3,3-difluoro-1-azetidine , $-N(CH_3)_2$, $-N(CH_3)-CH_2-CH_2-N(CH_3)_2$ or $-N(CH_3)-CH_2-CH_2-OCH_3$.

6. The compound of any one of clauses 1 to 5 wherein $R^{3a}$ represents bromo, chloro or $-CF_3$ and $R^{3b}$ represents hydrogen.

7. A pharmaceutical composition comprising a therapeutically effective amount of a compound as defined in any one of clauses 1 to 6 and a pharmaceutically acceptable carrier.

8. A process for preparing a pharmaceutical composition as defined in clause 7, characterized in that a pharmaceutically acceptable carrier is intimately mixed with a therapeutically effective amount of a compound as defined in any one of clauses 1 to 6.

9. A compound as defined in any one of clauses 1 to 6, for use as a pharmaceutical or medicament.

10. The compound for use according to clause 9 wherein the disease or disorder associated with inhibition of NLRP3 inflammasome activity is selected from inflammasome related diseases and disorders, immune diseases, inflammatory diseases, auto-immune diseases, auto-inflammatory fever syndromes, cryopyrin-associated periodic syndrome, chronic liver disease, viral hepatitis, non-alcoholic steatohepatitis, alcoholic steatohepatitis, alcoholic liver disease, inflammatory arthritis related disorders, gout, chondrocalcinosis, osteoarthritis, rheumatoid arthritis, chronic arthropathy, acute arthropathy, kidney related disease, hyperoxaluria, lupus nephritis, Type I and Type II diabetes, nephropathy, retinopathy, hypertensive nephropathy, hemodialysis related inflammation, neuroinflammation-related diseases, multiple sclerosis, brain infection, acute injury, neurodegenerative diseases, Alzheimer's disease, cardiovascular diseases, metabolic diseases, cardiovascular risk reduction, hypertension, atherosclerosis, peripheral artery disease, acute heart failure, inflammatory skin diseases, acne, wound healing and scar formation, asthma, sarcoidosis, age-related macular degeneration, colon cancer, lung cancer, myeloproliferative neoplasms, leukemias, myelodysplastic syndromes and myelofibrosis.

11. A process for the preparation of a compound of formula (I) as defined in any of clauses 1 to 6, which comprises:

(i) reaction of a compound of formula (II),

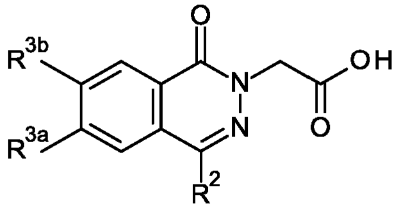

(II)

or a derivative thereof, wherein $R^2$ and $R^{3a}$ and $R^{3b}$ are as defined in clause 1, with a compound of formula (III),

$H_2N\text{-}R^1$ (III)

or a derivative thereof, wherein $R^1$ is as defined in clause 1, under amide-forming reaction conditions;

(ii) reaction of a compound of formula (IV),

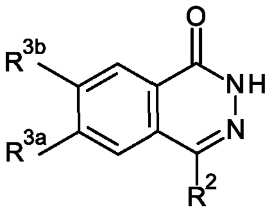

(IV)

wherein $R^2$ and $R^{3a}$ and $R^{3b}$ are as defined in clause 1, with a compound of formula (V),

$LG^a\text{-}CH_2\text{-}C(O)\text{-}N(H)R^1$ (V)

wherein $LG^a$ represents a suitable leaving group and $R^1$ is as defined in clause 1;
(iii) reaction of a compound of formula (IVA),

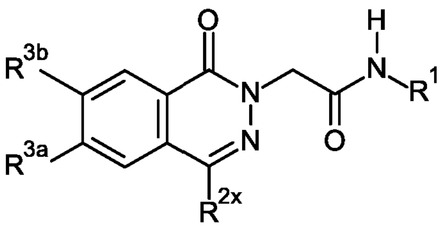

(IVA)

wherein $R^1$ and $R^{3a}$ and $R^{3b}$ are as defined in clause 1, and $R^{2x}$ represents halo, with a compound of formula (IVB),

$HN(R^{2a})R^{2b}$ (IVB)

wherein $R^{2a}$ and $R^{2b}$ are as defined in clause 1;
(iv) by transformation of a certain compound of formula (I) into another.

12. A compound of formula (II) or a compound of formula (IV)

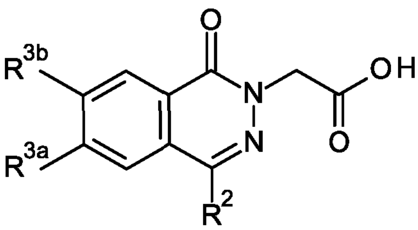

(II)

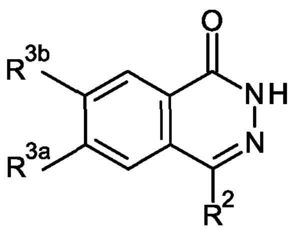

(IV)

wherein $R^2$ and $R^{3a}$ and $R^{3b}$ are as defined in clause 1.

## Claims

1. A compound of formula (I),

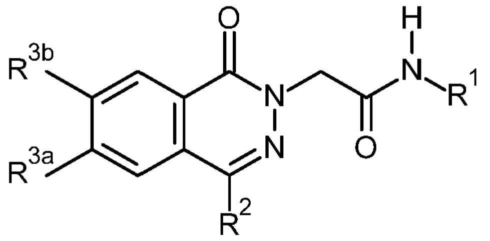

(I)

or a pharmaceutically acceptable salt thereof, wherein:

$R^1$ represents:

(i) $C_{3-6}$ cycloalkyl optionally substituted with one or more substituents independently selected from -OH and -$C_{1-3}$ alkyl;
(ii) aryl or heteroaryl, each of which is optionally substituted with 1 to 3 substituents independently selected from halo, -OH, -O-$C_{1-3}$ alkyl, -$C_{1-3}$ alkyl, halo$C_{1-3}$alkyl, hydroxy$C_{1-3}$ alkyl, $C_{1-3}$ alkoxy, halo$C_{1-3}$alkoxy; or
(iii) heterocyclyl, optionally substituted with 1 to 3 substituents independently selected from $C_{1-3}$ alkyl and $C_{3-6}$ cycloalkyl;

$R^2$ represents -N($R^{2a}$)$R^{2b}$;
$R^{2a}$ and $R^{2b}$ each represent hydrogen or $C_{1-4}$ alkyl optionally substituted with one or more substituents selected from halo, -O$C_{1-3}$ alkyl and -N($C_{1-3}$ alkyl)$_2$, or $R^{2a}$ and $R^{2b}$ may be linked together to form a 3- to 4-membered ring optionally substituted by one or more fluoro atoms;
one of $R^{3a}$ and $R^{3b}$ represents hydrogen, and the other represents $R^3$;
$R^3$ represents:

(i) hydrogen;
(ii) halo;
(iii) $C_{1-4}$ alkyl optionally substituted with one or more substituents independently selected from halo, -OH and -O$C_{1-3}$ alkyl;
(iv) $C_{2-4}$ alkenyl optionally substituted with -O$C_{1-3}$ alkyl;
(v) $C_{3-6}$ cycloalkyl; or
(vi) -O$C_{1-3}$ alkyl.

2. The compound of claim 1 wherein $R^1$ represents

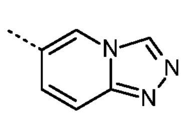
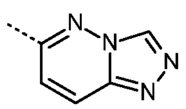
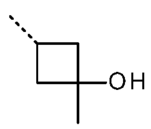
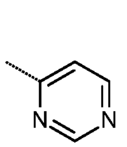
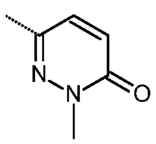
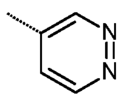
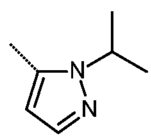

3. The compound of claim 2 wherein $R^1$ represents

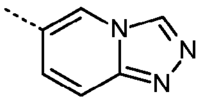 or 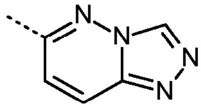.

4. The compound of claim 1, 2, or 3 wherein one of $R^{2a}$ and $R^{2b}$ represents $C_{1-3}$ alkyl (e.g. unsubstituted methyl) and the other represents $C_{1-3}$ alkyl optionally substituted by one substituent selected from $-N(CH_3)_2$ and $-OCH_3$, or $R^{2a}$ and $R^{2b}$ are linked together to form a 4-membered ring optionally substituted by one or two fluoro atoms.

5. The compound of claim 4 wherein $R^2$ represents 3,3-difluoro-1-azetidine , $-N(CH_3)_2$, $-N(CH_3)-CH_2-CH_2-N(CH_3)_2$ or $-N(CH_3)-CH_2-CH_2-OCH_3$.

6. The compound of any one of claims 1 to 5 wherein $R^{3a}$ represents bromo, chloro or - $CF_3$ and $R^{3b}$ represents hydrogen.

7. A pharmaceutical composition comprising a therapeutically effective amount of a compound as defined in any one of claims 1 to 6 and a pharmaceutically acceptable carrier.

8. A process for preparing a pharmaceutical composition as defined in claim 7, **characterized in that** a pharmaceutically acceptable carrier is intimately mixed with a therapeutically effective amount of a compound as defined in any one of claims 1 to 6.

9. A compound as claimed in any one of claims 1 to 6, for use as a pharmaceutical or medicament.

10. The compound for use according to claim 9 wherein the disease or disorder associated with inhibition of NLRP3 inflammasome activity is selected from inflammasome related diseases and disorders, immune diseases, inflammatory diseases, auto-immune diseases, auto-inflammatory fever syndromes, cryopyrin-associated periodic syndrome, chronic liver disease, viral hepatitis, non-alcoholic steatohepatitis, alcoholic steatohepatitis, alcoholic liver disease, inflammatory arthritis related disorders, gout, chondrocalcinosis, osteoarthritis, rheumatoid arthritis, chronic arthropathy, acute arthropathy, kidney related disease, hyperoxaluria, lupus nephritis, Type I and Type II diabetes, nephropathy, retinopathy, hypertensive nephropathy, hemodialysis related inflammation, neuroinflammation-related diseases, multiple sclerosis, brain infection, acute injury, neurodegenerative diseases, Alzheimer's disease, cardiovascular diseases, metabolic diseases, cardiovascular risk reduction, hypertension, atherosclerosis, peripheral artery disease, acute heart failure, inflammatory skin diseases, acne, wound healing and scar formation, asthma, sarcoidosis, age-related macular degeneration, colon cancer, lung cancer, myeloproliferative neoplasms, leukemias, myelodysplastic syndromes and myelofibrosis.

11. A process for the preparation of a compound of formula (I) as claimed in any of claims 1 to 6, which comprises:

(i) reaction of a compound of formula (II),

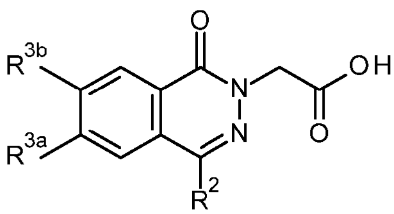

(II)

or a derivative thereof, wherein $R^2$ and $R^{3a}$ and $R^{3b}$ are as defined in claim 1, with a compound of formula (III),

$H_2N-R^1$ (III)

or a derivative thereof, wherein $R^1$ is as defined in claim 1, under amide-forming reaction conditions;

(ii) reaction of a compound of formula (IV),

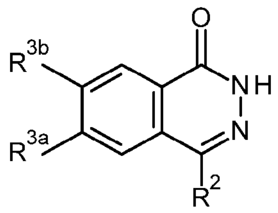

(IV)

wherein $R^2$ and $R^{3a}$ and $R^{3b}$ are as defined in claim 1, with a compound of formula (V),

$LG^a$-$CH_2$-C(O)-N(H)$R^1$ (V)

wherein $LG^a$ represents a suitable leaving group and $R^1$ is as defined in claim 1;
(iii) reaction of a compound of formula (IVA),

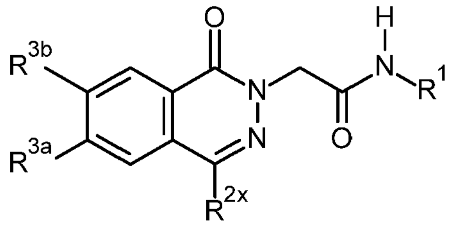

(IVA)

wherein $R^1$ and $R^{3a}$ and $R^{3b}$ are as defined in claim 1, and $R^{2x}$ represents halo, with a compound of formula (IVB),

HN($R^{2a}$)$R^{2b}$ (IVB)

wherein $R^{2a}$ and $R^{2b}$ are as defined in claim 1;
(iv) by transformation of a certain compound of formula (I) into another.

**12.** A compound of formula (II) or a compound of formula (IV)

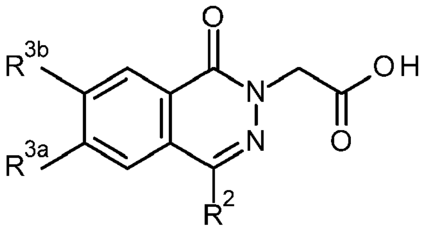

(II)

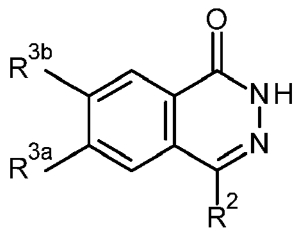

(IV)

wherein $R^2$ and $R^{3a}$ and $R^{3b}$ are as defined in claim 1.

Europäisches Patentamt
European Patent Office
Office européen des brevets

# EUROPEAN SEARCH REPORT

Application Number
EP 25 15 7062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2020/021447 A1 (NOVARTIS AG) 30 January 2020 (2020-01-30) * abstract; claims; examples * | 1-12 | INV. C07D237/34 C07D403/04 C07D403/12 C07D403/14 C07D471/04 C07D487/04 A61P25/28 A61P29/00 A61P35/00 A61P37/00 A61K31/502 |
| A | SETHI, G.S. ET AL.: "PARP inhibition by olaparib alleviates chronic asthma-associated remodeling features via modulating inflammasome signaling in mice", IUBMB LIFE , vol. 71, no. 7 2019, pages 1003-1013, XP055863790, ISSN: 1521-6543, DOI: 10.1002/iub.2048 Retrieved from the Internet: URL:https://onlinelibrary.wiley.com/doi/full-xml/10.1002/iub.2048 * abstract * | 1-12 | |
| X | LIM, C.J. ET AL.: "4-Aminophthalazin-1(2H)-one Derivatives as Melanin Concentrating Hormone Receptor 1 (MCH-R1) Antagonists", BULLETIN OF THE KOREAN CHEMICAL SOCIETY, vol. 34, no. 12, 2013, pages 3851-3854, XP055593746, ISSN: 0253-2964, DOI: 10.5012/bkcs.2013.34.12.3851 * Scheme 1; page 3852; compounds 5a, 5b, 5c * * page 3853; table 1 * | 12 | TECHNICAL FIELDS SEARCHED (IPC): C07D A61P A61K |

-/--

The present search report has been drawn up for all claims

1

| Place of search | Date of completion of the search | Examiner |
|---|---|---|
| Munich | 24 April 2025 | Kiernan, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 15 7062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELAGAWANY, M. ET AL.: "Design, synthesis, and molecular modelling of pyridazinone and phthalazinone derivatives as protein kinases inhibitors", BIOORGANIC & MEDICINAL CHEMISTRY LETTERS, vol. 23, no. 7, 2013, pages 2007-2013, XP055125818, ISSN: 0960-894X, DOI: 10.1016/j.bmcl.2013.02.027 * Scheme 2b.; page 2009; compound 9b * * Scheme 3b.; page 2010; compound 20e * ----- | 12 | |
| X | FLITSCH, W. ET AL.: "Zur Synthese von 4-Amino-phthalazin-1-onen", ANGEWANDTE CHEMIE, vol. 79, no. 3, 1967, pages 149-150, XP055935343, ISSN: 0044-8249, DOI: 10.1002/ange.19670790311 * reaction scheme; page 149, column 2; compound (1) * * page 150; table; compounds (1a), (1b) * ----- | 12 | TECHNICAL FIELDS SEARCHED (IPC) |
| X | KÖRMENDY, K. ET AL.: "AMINOPHTHALAZINONE DERIVATIVES, VIII METHODS FOR THE SYNTHESIS OF IMIDAZO[2,1-a]PHTHALAZINE AND PIRIMIDO[2,1-a]PHTHALAZINE RING SYSTEMS, I CYCLIZATION OF HYDROXYALKYLAMINOPHTHALAZINONES WITH MINERAL ACIDS", ACTA CHIMICA HUNGARICA, vol. 112, no. 1, 1983, pages 65-81, XP009055269, ISSN: 0231-3146 * page 69; figure 3; compounds 12a, 12b, 12c, 12f, 12g * ----- | 12 | |

-/--

1

The present search report has been drawn up for all claims

| Place of search | Date of completion of the search | Examiner |
|---|---|---|
| Munich | 24 April 2025 | Kiernan, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 15 7062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KÖRMENDY, K. ET AL.: "AMINOPHTHALAZINONE DERIVATIVES, VII REACTION OF CHLOROPHTHALAZINONE WITH SECONDARY AMINES STUDY OF THE STERIC EFFECT, II", ACTA CHIMICA ACADEMIAE SCIENTIARUM HUNGARICAE, vol. 106, no. 2, 1981, pages 155-166, XP009055267, ISSN: 0001-5407 * page 156; figure 1; compound 7 * * page 166; compounds (7a), (7c) * ----- | 12 | |
| X | KÖRMENDY, K.: "Aminophthalazinone derivatives, I", ACTA CHIMICA ACADEMIAE SCIENTIARUM HUNGARICAE, vol. 88, no. 2, 1976, pages 129-136, XP009187441, ISSN: 0001-5407 * page 130; compound 2 * * page 132; table I; compound 5k * ----- | 12 | |
| X | LINK, H. ET AL.: "199. Reaktionsprodukte aus 3-Dimethylamino-2,2-dimethyl-2H-azirin und Phthalohydrazid bzw. Maleohydrazid", HELVETICA CHIMICA ACTA, vol. 61, no. 6, 1978, pages 2116-2129, XP055935369, DOI: 10.1002/hlca.19780610619 * page 2119; compound 12 * * Scheme 6; page 2121; compound 14a * ----- | 12 | TECHNICAL FIELDS SEARCHED (IPC) |

-/--

The present search report has been drawn up for all claims

| Place of search | Date of completion of the search | Examiner |
|---|---|---|
| Munich | 24 April 2025 | Kiernan, Andrea |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 15 7062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KRISHNANANTHAN, S. ET AL.: "Regioselective Synthesis of Substituted 4-Alkylamino and 4-Arylaminophthalazin-1(2H)-ones", THE JOURNAL OF ORGANIC CHEMISTRY , vol. 81, no. 4 2016, pages 1520-1526, XP055935372, ISSN: 0022-3263, DOI: 10.1021/acs.joc.5b02652 Retrieved from the Internet: URL:https://pubs.acs.org/doi/pdf/10.1021/acs.joc.5b02652 * abstract * * Scheme 1; page 1521 * * Scheme 3; page 1523; compound 5i * ----- | 12 | |
| X | VLAAR, T. ET AL.: "Multicomponent Synthesis of 4-Aminophthalazin-1(2H)-ones by Palladium-Catalyzed Isocyanide Insertion", THE JOURNAL OF ORGANIC CHEMISTRY , vol. 78, no. 13 2013, pages 6735-6745, XP055935380, ISSN: 0022-3263, DOI: 10.1021/jo401131p Retrieved from the Internet: URL:https://pubs.acs.org/doi/pdf/10.1021/jo401131p * abstract * * page 6737; table 1; compounds 4a, 4b, 4c, 4d, 4f, 4h * * page 6738; table 2; compounds 5a, 5b, 5c, 5e * ----- -/-- | 12 | TECHNICAL FIELDS SEARCHED (IPC) |

1 The present search report has been drawn up for all claims

| Place of search | Date of completion of the search | Examiner |
|---|---|---|
| Munich | 24 April 2025 | Kiernan, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 15 7062

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2021/239885 A1 (JANSSEN PHARMACEUTICA NV) 2 December 2021 (2021-12-02)<br>* abstract; claims; examples *<br>* page 23 - page 26 *<br>* page 110, line 12; compounds I-155 *<br>----- | 1-12 | |
| X | KÖRMENDY, K.: "AMINOPHTHALAZINONE DERIVATIVES, III THE REACTION OF CHLOROPHTHALAZINONE WITH PRIMARY ALKYL-, CYCLOALKYL- AND ARALKYLAMINES, STUDY OF THE STERIC EFFECT, I",<br>ACTA CHIMICA ACADEMIAE SCIENTIARUM HUNGARICA,<br>vol. 98, no. 3, 1978, pages 303-313,<br>XP009536902,<br>ISSN: 0001-5407<br>* page 305; compound 6 *<br>* page 306; table I; compounds 6a, 6b, 6c, 6d, 6e *<br>----- | 12 | |

TECHNICAL FIELDS SEARCHED (IPC)

The present search report has been drawn up for all claims

1

| Place of search | Date of completion of the search | Examiner |
|---|---|---|
| Munich | 24 April 2025 | Kiernan, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 15 7062

This annex lists the patent family members relating to the patent documents cited in the above-mentioned European search report. The members are as contained in the European Patent Office EDP file on
The European Patent Office is in no way liable for these particulars which are merely given for the purpose of information.

24-04-2025

| Patent document cited in search report | Publication date | Patent family member(s) | Publication date |
|---|---|---|---|
| WO 2020021447 A1 | 30-01-2020 | AU 2019309727 A1 | 07-01-2021 |
| | | BR 112021000964 A2 | 20-04-2021 |
| | | CA 3103943 A1 | 30-01-2020 |
| | | CL 2021000191 A1 | 20-08-2021 |
| | | CN 112424207 A | 26-02-2021 |
| | | CO 2021000537 A2 | 29-01-2021 |
| | | CR 20210045 A | 18-06-2021 |
| | | CU 20210009 A7 | 06-08-2021 |
| | | DO P2021000019 A | 15-02-2021 |
| | | EC SP21004787 A | 31-03-2021 |
| | | EP 3827008 A1 | 02-06-2021 |
| | | IL 280336 A | 01-03-2021 |
| | | JP 7555327 B2 | 24-09-2024 |
| | | JP 2021532124 A | 25-11-2021 |
| | | KR 20210038877 A | 08-04-2021 |
| | | MA 53388 A | 02-06-2021 |
| | | PE 20210406 A1 | 02-03-2021 |
| | | PH 12021550166 A1 | 13-09-2021 |
| | | SG 11202012321P A | 25-02-2021 |
| | | US 2021308140 A1 | 07-10-2021 |
| | | WO 2020021447 A1 | 30-01-2020 |
| WO 2021239885 A1 | 02-12-2021 | AU 2021279305 A1 | 09-02-2023 |
| | | BR 112022023271 A2 | 20-12-2022 |
| | | CA 3177672 A1 | 02-12-2021 |
| | | CN 115667225 A | 31-01-2023 |
| | | EP 4157823 A1 | 05-04-2023 |
| | | JP 2023527010 A | 26-06-2023 |
| | | KR 20230016658 A | 02-02-2023 |
| | | US 2023202989 A1 | 29-06-2023 |
| | | WO 2021239885 A1 | 02-12-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020018975 A
- WO 2020037116 A
- WO 2020021447 A
- WO 2020010143 A
- WO 2019079119 A
- WO 20190166621 A
- WO 2019121691 A


### Non-patent literature cited in the description


- **VAN OPDENBOSCH N** ; **LAMKANFI M**. *Immunity*, 18 June 2019, vol. 50 (6), 1352-1364
- **TARTEY S** ; **KANNEGANTI TD**. *Immunology*, April 2019, vol. 156 (4), 329-338
- **KELLEY et al.** *Int J Mol Sci*, 06 July 2019, vol. 20, 13
- **FUNG et al.** *Emerg Microbes Infect*, 14 March 2020, vol. 9 (1), 558-570
- **YANG Y et al.** *Cell Death Dis*, 12 February 2019, vol. 10 (2), 128
- **SHARIF et al.** *Nature*, vol. 570 (7761), 338-343
- **MIYAMAE T**. Cryopyrin-associated Periodic Syndrome. *Paediatr Drugs*, 01 April 2012, vol. 14 (2), 109-17
- **SZABO G** ; **PETRASEK J.** *Nat Rev Gastroenterol Hepatol*, July 2015, vol. 12 (7), 387-400
- **ZHEN Y** ; **ZHANG H**. *Front Immunol*, 28 February 2019, vol. 10, 276
- **VANDE WALLE L et al.** *Nature*, 07 August 2014, vol. 512 (7512), 69-73
- **KNAUF et al.** *Kidney Int*, November 2013, vol. 84 (5), 895-901
- **KRISHNAN et al.** *Br J Pharmacol*, February 2016, vol. 173 (4), 752-65
- **SHAHZAD et al.** *Kidney Int*, January 2015, vol. 87 (1), 74-84
- **SARKAR et al.** *NPJ Parkinson's Dis*, 17 October 2017, vol. 3 (30)
- **RIDKER et al.** CANTOS Trial Group. *N Engl J Med*, 21 September 2017, vol. 377 (12), 1119-1131
- **TOLDO S** ; **ABBATE A**. *Nat Rev Cardiol*, April 2018, vol. 15 (4), 203-214
- **KELLY et al.** *Br J Dermatol*, December 2015, vol. 173 (6)
- **NIETO-TORRES et al.** *Virology*, November 2015, vol. 485, 330-9
- **DOYLE et al.** *Nat Med*, May 2012, vol. 18 (5), 791-8
- **RIDKER et al.** *Lancet*, 21 October 2017, vol. 390 (10105), 1833-1842
- **DERANGERE et al.** *Cell Death Differ.*, December 2014, vol. 21 (12), 1914-24
- **BASIORKA et al.** *Lancet Haematol*, September 2018, vol. 5 (9), e393-e402
- **ZHANG et al.** *Hum Immunol*, January 2018, vol. 79 (1), 57-62
- **T.W. GREENE** ; **P.G.M. WUTS**. Protective Groups in Organic Synthesis. Wiley-Interscience, 1999
- **MENU et al.** *Clinical and Experimental Immunology*, 2011, vol. 166, 1-15
- **STROWIG et al.** *Nature*, 2012, vol. 481, 278-286
- **OZAKI et al.** *J. Inflammation Research*, 2015 (8), 15-27
- **SCHRODER et al.** *Cell*, 2010, vol. 140, 821-832
- **COOK et al.** *Eur. J. Immunol.*, 2010, vol. 40, 595-653
- **BRADDOCK et al.** *Nat. Rev. Drug Disc.*, 2004 (3), 1-10
- **INOUE et al.** *Immunology*, 2013, vol. 139, 11-18
- **COLL**. *Nat. Med.*, 2015, vol. 21 (3), 248-55
- **SCOTT et al.** *Clin. Exp. Rheumatol.*, 2016, vol. 34 (1), 88-93
- **LU et al.** *J. Immunol.*, 2017, vol. 198 (3), 1119-29
- **ARTLETT et al.** *Arthritis Rheum.*, 2011, vol. 63 (11), 3563-74
- **DE NARDO et al.** *Am. J. Pathol.*, 2014, vol. 184, 42-54
- **KIM et al.** *Am. J. Respir. Crit. Care Med*, 2017, vol. 196 (3), 283-97
- **WALSH et al.** *Nature Reviews*, 2014, vol. 15, 84-97
- **DEMPSEY et al.** *Brain. Behav. Immun.*, 2017, vol. 61, 306-16
- **ZHANG et al.** *J. Stroke and Cerebrovascular Dis.*, 2015, vol. 24 (5), 972-9
- **ISMAEL et al.** *J. Neurotrauma.*, 2018, vol. 35 (11), 1294-1303

- **WEN et al.** *Nature Immunology*, 2012, vol. 13, 352-357
- **DUEWELL et al.** *Nature*, 2010, vol. 464, 1357-1361
- **STROWIG et al.** *Nature*, 2014, vol. 481, 278-286
- **MRIDHA et al.** *J. Hepatol.*, 2017, vol. 66 (5), 1037-46
- **VAN HOUT et al.** *Eur. Heart J.*, 2017, vol. 38 (11), 828-36
- **SANO et al.** *J. Am. Coll. Cardiol.*, 2018, vol. 71 (8), 875-66
- **WU et al.** *Arteriosc/er. Thromb. Vase. Biol.*, 2017, vol. 37 (4), 694-706
- **RIDKER et al.** *N. Engl. J. Med.*, 2017, vol. 377 (12), 1119-31
- **DOYLE et al.** *Nature Medicine*, 2012, vol. 18, 791-798
- **TARALLO et al.** *Cell*, 2012, vol. 149 (4), 847-59
- **LOUKOVAARA et al.** *Acta Ophthalmol.*, 2017, vol. 95 (8), 803-8
- **PUYANG et al.** *Sci. Rep.*, 2016, vol. 6, 20998
- **HENAO-MEIJA et al.** *Nature*, 2012, vol. 482, 179-185
- **PRIMIANO et al.** *J. Immunol.*, 2016, vol. 197 (6), 2421-33
- **FANG et al.** *J Dermatol Sci.*, 2016, vol. 83 (2), 116-23
- **NIEBUHR et al.** *Allergy*, 2014, vol. 69 (8), 1058-67
- **ALIKHAN et al.** *J. Am. Acad. Dermatol.*, 2009, vol. 60 (4), 539-61
- **JAGER et al.** *Am. J. Respir. Crit. Care Med.*, 2015, vol. 191, A5816
- **BRADDOCK et al.** *Nat. Rev. Drug Disc*, 2004, vol. 3, 1-10
- **GUGLIANDOLO et al.** *Int. J. Mo/. Sci.*, 2018, vol. 19 (7), E1992
- **LANNITTI et al.** *Nat. Commun.*, 2016, vol. 7, 10791
- **GRANATA et al.** *PLoS One*, 2015, vol. 10 (3), eoi22272
- **NEUDECKER.** *J. Exp. Med.*, 2017, vol. 214 (6), 1737-52
- **LAZARIDIS et al.** *Dig. Dis. Sci.*, 2017, vol. 62 (9), 2348-56
- **DOLUNAY et al.** *Inflammation*, 2017, vol. 40, 366-86
- **TATE et al.** *Sci Rep.*, 2016, vol. 10 (6), 27912-20
- **NOVIAS et al.** *PLOS Pathogens*, 2017, vol. 13 (2), e1006196
- **RIDKER et al.** *Lancet.*, 2017, vol. 390 (10105), 1833-42
- **WANG et al.** *Onco/ Rep.*, 2016, vol. 35 (4), 2053-64
- **BASIORKA et al.** *Blood*, 2016, vol. 128 (25), 2960-75
- **LI et al.** *Am. J. Cancer Res.*, 2015, vol. 5 (1), 442-9
- **ALLEN et al.** *J. Exp. Med.*, 2010, vol. 207 (5), 1045-56
- **HU et al.** *PNAS.*, 2010, vol. 107 (50), 21635-40
- **LI et al.** *Hematology*, 2016, vol. 21 (3), 144-51
- **HUANG et al.** *J. Exp. Clin. Cancer Res.*, 2017, vol. 36 (1), 116
- **FENG et al.** *J. Exp. Clin. Cancer Res.*, 2017, vol. 36 (1), 81
- **JIA et al.** *Mol. Pain.*, 2017, vol. 13, 1-11
- **YAN-GANG et al.** *Cell Death and Disease*, 2017, vol. 8 (2), 2579
- **ALEXANDER et al.** *Hepatology*, 2014, vol. 59 (3), 898-910
- **BALDWIN et al.** *J. Med. Chem.*, 2016, vol. 59 (5), 1691-1710
- **OZAKI.** *J. Inflammation Research*, 2015, vol. 8, 15-27
- **ZHEN.** *Neuroimmunology Neuroinflammation*, 2014, vol. 1 (2), 60-65
- **MATTIA.** *J Med. Chem.*, 2014, vol. 57 (24), 10366-82
- **SATOH et al.** *Cell Death and Disease*, 2013, vol. 4, 644
- Remington The Science and Practice of Pharmacy. Pharmaceutical Press, 2013, 1049-1070